# FASCICULE DE BREVET EUROPEEN

(11) **EP 2 235 003 B1**
(45) Date de publication et mention de la délivrance du brevet: **19.12.2012**
(21) Numéro de dépôt: 09718226.5
(22) Date de dépôt: 20.01.2009
(51) Int. Cl.: C07D 401/12, C07D 401/14, C07F 7/08, C07F 7/10, C07F 7/12, A61K 31/695, A61K 31/4439, A61P 25/00, A61P 3/00, A61P 13/00, A61P 15/00

(54) **DERIVES D'INDOLE 2-CARBOXAMIDES ET D'AZAINDOLE 2-CARBOXAMIDES SUBSTITUES PAR UN GROUPE SILANYLE, LEUR PREPARATION ET LEUR APPLICATION EN THERAPEUTIQUE**
DERIVATE VON DURCH EINE SILANYLGRUPPE SUBSTITUIERTEN INDOL-2-CARBOXAMIDEN UND AZAINDOL-2-CARBOXAMIDEN, IHRE HERSTELLUNG UND IHRE THERAPEUTISCHE VERWENDUNG
DERIVATIVES OF INDOLE 2-CARBOXAMIDES AND AZAINDOLE 2-CARBOXAMIDES SUBSTITUTED BY A SILANYLE GROUP, PREPARATION THEREOF AND THERAPEUTIC USE THEREOF

(30) Priorité: 23.01.2008 FR 0800343
(43) Date de publication de la demande: 06.10.2010
(73) Titulaire: SANOFI, 75008 Paris (FR)
(72) Inventeur: DUBOIS, Laurent, F-75013 Paris (FR); EVANNO, Yannick, F-75013 Paris (FR); MALANDA, André, F-75013 Paris (FR)
(74) Mandataire: Le Coupanec, Pascale A.M.P.
(86) Numéro de dépôt international: PCT/FR2009/000054
(87) Numéro de publication internationale: WO 2009/109710

(56) Documents cités:
- WO-A-03/068749
- WO-A-2005/032493
- WO-A-2005/072681
- WO-A-2007/010144

## Description

L'invention a pour objet des composés dérivés d'indole 2-carboxamides et d'azaindole 2-carboxamides substitués par un groupe silanyle, qui présentent une activité antagoniste ou agoniste *in vitro* et *in vivo* pour les récepteurs de type TRPV1 (ou VR1).

Un premier objet de l'invention concerne les composés répondant à la formule générale (I) ci-après.

Un autre objet de l'invention concerne des procédés de préparation des composés de formule générale (I).

Un autre objet de l'invention concerne l'utilisation des composés de formule générale (I) notamment dans des médicaments ou dans des compositions pharmaceutiques.

Les composés de l'invention répondent à la formule générale (I) : 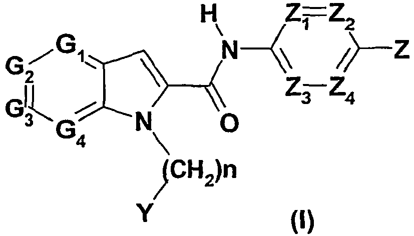 dans laquelle
G₁, G₂, G₃ et G₄ représentent, indépendamment l'un de l'autre, un groupe C-X ou un atome d'azote ;
X est choisi parmi un atome d'hydrogène, d'halogène ou un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxyle, C₃-C₇-cydoalcoxyle, C₃-C₇-cycloalkyl-C₁-C₆-alkylène-O-, C₁-C₆-fluoroalcoxyle, cyano, C₁-C₆-thioalkyle, NR₁R₂, aryl-C₁-C₆-alkylène-O, aryle, hétéroaryle ou -Si(X₁)(X₂)(X₃) ; les groupes C₁-C₆-alkyle, C₃-C₇-cycloalkyle, C₃-C₇-cycloalkyl-C₁-C₃-alkylène, C₁-C₆-fluoroalkyle, C₁-C₆-alcoxyle, C₃-C₇-cycloalcoxyle, C₃-C₇-cycloalkyl-C₁-C₆-alkylène-O- étant éventuellement substitués par un groupe hydroxy, C₁-C₆-alcoxyle ou NR₄R₅ ; l'aryle et l'hétéroaryle étant éventuellement substitué par un ou plusieurs substituants choisis parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
l'un des G₁, G₂, G₃ et G₄ et au maximum l'un des G₁, G₂, G₃ et G₄ représente un groupe C-X où X est un groupe -Si(X₁)(X₂)(X₃);
X₁, X₂, X₃ représentent, indépendamment l'un de l'autre, un groupe C₁-C₆-alkyle, C₃-C₇-cycloalkyle, C₃-C₇-cycloalkyl-C₁-C₆-alkyle, hydroxyle, aryle ou hétéroaryle ;
les groupes aryle ou hétéroaryle de X₁, X₂, X₃ étant éventuellement substitués par un ou plusieurs substituants choisi parmi un halogène, un groupe C₁-C₆-alkyle, C_{3*}-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
les groupes C₁-C₆-alkyle, C₃-C₇-cycloalkyle, C₃-C₇-cycloalkyl-C₁-C₆-alkyle de X₁, X₂, X₃ étant éventuellement substitués par un ou plusieurs substituants choisi parmi un groupe hydroxyle, C₁-C₆-alcoxyle, aryl-O-, NR₁R₂, aryle ou hétéroaryle, l'aryle et l'hétéroaryle étant éventuellement substitué par un ou plusieurs substituants choisis parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
X₁ et X₂ pouvant également former ensemble avec l'atome de silicium qui les porte un cycle de 4 à 7 chaînons contenant éventuellement un hétéroatome choisi parmi O, S et N;
n est égal à 0, 1, 2 ou 3 ;
Y représente un aryle ou un hétéroaryle ; l'aryle ou l'hétéroaryle étant éventuellement substitué par un ou plusieurs groupes choisis parmi un atome d'halogène ou un groupe C₁-C₆-alkyle, C₃-C₇-cycloalkyle, C₃-C₇-cycoalkyle-C₁-C₆-alkylène, C₁-C₆-fluoroalkyle, hydroxyle, C₁-C₆-alcoxyle, C₃-C₇-cycloalcoxyle, C₃-C₇-cycloalkyle-C₁-C₆-alkylène-O-, C₁-C₆-fluoroalcoxyle, cyano, C₁-C₆-thioalkyle, thiol, -S(O)-C₁-C₆-alkyle, -S(O)₂-C₁-C₆-alkyle, C(O)NR₁R₂, SO₂NR₁R₂, SF₅, nitro, OCONR₁R₂, NR₃COR₄, NR₃CONR₁R₂, NR₁R₂, NR₃SO₂NR₁R₂, NR₃COR₄, NR₃SO₂R₅, aryl-C₁-C₆-alkylène ou aryle ; l'aryle et l'aryl-C₁-C₆-alkylène étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
Z₁, Z₂, Z₃, Z₄ représentent, indépendamment l'un de l'autre, un atome d'azote ou un groupe C(R₆), l'un au moins correspondant à un atome d'azote et l'un au moins correspondant à un groupe C(R₆) ; l'atome d'azote ou un des atomes d'azote présent dans le cycle, défini comme azote de position 1, étant éventuellement substitué par R₇ lorsque l'atome de carbone en position 2 ou 4 par rapport à cet azote de référence est substitué par un groupe oxo ou thio ;
Z représente
soit une amine cyclique reliée par l'atome d'azote, de formule : 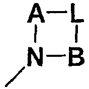 dans laquelle
A représente un groupe C₁-C₇-alkylène éventuellement substitué par un ou deux groupes R₈;
B représente un groupe C₁-C₇-alkylène éventuellement substitué par un ou deux groupes R₉ ;
L représente une liaison, un atome de soufre, d'oxygène ou d'azote ; l'atome d'azote étant éventuellement substitué par un groupe R₁₀ ou R₁₁ ;
les atomes de carbone de l'amine cyclique Z étant éventuellement substitués par un ou plusieurs groupes R₁₂ identiques ou différents l'un de l'autre ;
soit une amine acyclique, reliée par l'atome d'azote, de formule NRaRb dans laquelle Ra et Rb représentent, indépendamment l'un de l'autre, un atome d'hydrogène ou un groupe C₁-C₆-alkyle, C₃-C₇-cycloalkyle, C₃-C₇₋cycloalkyle-C₁-C₆-alkylène, C₁-C₆-fluoroalkyle, hydroxyle, C₁-C₆-alcoxyle, C₃-C₇-cycloalkyle-C₁-C₆-alkylène-O-, C₁-C₆-fluoroalcoxyle, C₁-C₆-alkyle-C(O)-, HO-C(O)-C₁-C₆-alkylène, C₁-C₆-alkyle-OC(O)-C₁-C₆-alkylène, aryle ou hétéroaryle, Ra et Rb pouvant être éventuellement substitués par un ou plusieurs groupes Rc identiques ou différents l'un de l'autre ;
Rc représente un atome d'halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalkyle-C₁-C₆-alkylène-O-, C₁-C₆-fluoroalcoxyle, C₁-C₆-thioalkyle, -S(O)-C₁-C₆-akyle, -S(O)₂-C₁-C₆-alkyle, cyano, C(O)NR₁R₂, NR₁R₂, SO₂NR₁R₂, NR₃COR₄, NR₃SO₂R₅, OC(O)NR₁R₂, NR₃COOR₅, NR₃CONR₁R₂, NR₃SO₂NR₁R₂, hydroxyle, thiol, oxo, thio, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₁ et R₂ représentent, indépendamment l'un de l'autre, un atome d'hydrogène ou un groupe C₁-C₆-alkyle, C₃-C₇-cycloalkyle, C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ; ou R₁ et R₂ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe C₁-C₆-alkyle, C₃-C₇-cycloalkyle, C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₃ et R₄ représentent, indépendamment l'un de l'autre, un atome d'hydrogène ou un groupe C₁-C₆-alkyle, C₃-C₇-cycloalkyle. C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cydoalkyle, C₃-C₇-cyclalkyle-C₁-C₆-alkylène, C₁-C₆-fluoroalkyle, C₁-C₆-alcoxyle, C₁-C₆-fluoroalcoxyle, nitro ou cyano ;
ou R₃ et R₄ forment ensemble un groupe (C₂-C₅)alkylène ;
ou R₁ et R₃ forment ensemble un groupe (C₂-C₅)alkylène ;
R₅ représente un groupe C₁-C₆-alkyle, C₃-C₇-cycloalkyle, C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ; ou R₃ et R₅ forment ensemble un groupe (C₂-C₅)alkylène ;
R₆ représente un atome d'hydrogène ou d'halogène ou un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, C₁-C₆-thioalkyle, -S(O)-C₁-C₆-alkyle, -S(O)₂-C₁-C₆-alkyle, hydroxyle, thiol, oxo, thio, aryle, aryl-C₁-C₆-alkylène, hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₇ représente un atome d'hydrogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, aryle, aryl-C₁-C₆-alkylène ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;

R₈, R₉ et R₁₀ sont définis tels que :
deux groupes R₈ peuvent former ensemble une liaison ou un groupe C₁-C₆-alkylène ;
deux groupes R₉ peuvent former ensemble une liaison ou un groupe C₁-C₆-alkylène ;
R₈ et R₉ peuvent former ensemble une liaison ou un groupe C₁-C₆-alkylène ;
R₈ et R₁₀ peuvent former ensemble une liaison ou un groupe C₁-C₆-alkylène ;
R₉ et R₁₀ peuvent former ensemble une liaison ou un groupe C₁-C₆-alkylène ;
R₁₁ représente un atome d'hydrogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, hydroxyle, C₁-C₆-alkyl-CO-, COOR₅, C(O)NR₁R₂, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₁-C₆-fluoroalcoxyle, nitro ou cyano ;
R₁₂ représente un atome de fluor, un groupe C₁-C₆-alkyle éventuellement substitué par un groupe R₁₃; C₃-C₇-cycloalkyle, C₃-C₇-cycloalkyle-C₁-C₆-alkylène, C₁-C₆-fluoroalkyle, C₁-C₆-cycloalk-1 ,1-diyle, C₃-C₇-hétérocycloalk-1,1-diyle éventuellement substitué sur un atome d'azote par un groupe R₁₁; C₁-C₆-alcoxyle, C₃-C₇-cycloalcoxyle, C₃-C₇-cycloalkyle-C₁-C₆-alkylène-O-, C₁-C₆-fluoroalcoxyle, CO₂H, C(O)O-C₁-C₆-alkyle, C(O)NR₁R₂, NR₁R₂, NR₃COR₄, OC(O)NR₁R₂, NR₃COOR₅, NR₃CONR₁R₂, hydroxyle, thiol, oxo, thio, aryl-C₁-C₆-alkylène, aryle ; l'aryle étant éventuellement substitué par un ou plusieurs substituants choisi parmi un halogène, un groupe C₁-C₆-alkyle, C₃-C₇-cycloalkyle, C₃-C-₇-cycloalkyle-C₁-C₆-alkylène, C₁-C₆-fluoroalkyle, C₁-C₆-alcoxyle, C₃-C₇-cycloalcoxyle, C₁-C₆-fluoroalcoxyle, nitro ou cyano ;
R₁₃ représente un groupe C₁-C₆-alcoxyle, C₃-C₇-cycloalcoxyle, C₃-C₇-cycloalkyle-C₁-C₆-alkylène-O-, C(O)NR₁R₂, NR₁R₂, NR₃COR₄, OC(O)NR₁R₂, NR₃COOR₅, hydroxyle.

Dans les composés de formule générale (I), le ou les atomes d'azote peuvent être sous forme oxydée (N-oxyde).

A titre d'exemples non limitatifs d'amines Z substituées ou non substituées , on peut citer la méthylamine, l'éthylamine, la 2-méthoxyéthylamine, la 2-hydroxyéthylamine, la cyclopropylamine, l'hydroxylamine, la 2-(*N,N*-diméthylamino)éthylamine, la diméthylamine, l'isopropylamine, la N-éthyl-méthylamine, la 2,5-diméthylpyrrolidine, la 3-hydroxypyrrolidine, la 3-éthoxypyrrolidine, la 3,3-difluoropyrrolidine, la 3,3-difluoroazétidine, la 3-hydroxyazétidine, la proline, l'aziridine, l'azétidine, la pyrrolidine, la pipéridine, l'azépine, la morpholine, la thiomorpholine, la pipérazine, l'homopipérazine, les azabicyclo[3.1.0]hexanes, les azabicyclo[3.2.0]heptanes , les azabicyclo[3.3.0]octanes, les octahydrofuropyrroles, les octahydropyrrolopyrroles, l'octahydroindole, l'octahydroisoindole, les octahydropyrrolopyridines, la décahydroquinoléine, la décahydroisoquinoléine, les décahydronaphthyridines, l'octahydropyridopyrazine, les azabicylo[3.1.1]heptanes, les diazabicylo[2.2.1]heptanes, les azabicylo[3.2.1]octanes, les diazabicylo[3.2.1]octanes, les azabicylo[3.3.1]nonanes.

Dans le cadre de la présente invention on entend par :
- Cₜ-C_{z} où t et z peuvent prendre les valeurs de 1 à 7, une chaîne carbonée pouvant avoir de t à z atomes de carbone, par exemple C₁-C₃ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, par exemple un méthylène, éthylène, 1-méthyléthylène, propylène ;
- un cycloalkyle : un groupe carboné cyclique. A titre d'exemples, on peut citer les groupes cyclopropyle, cyclobutyle, cyclopentyle, cyclohexyle, etc ;
- un hétérocycloalkyle : un groupe cyclique de 3 à 7 chaînons contenant de 1 à 2 hétéroatomes choisis parmi O, S ou N ;
- un cycloalk-1,1-diyle ou un hétérocycloalk-1,1-diyle : un groupe du type 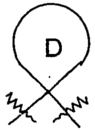 où D est un groupe cycloalkyle ou hétérocycloalkyle ;
- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un cycloalcoxyle : un radical -O-cycloalkyle où le groupe cylcoalkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphthyle ;
- un hétéroaryle : un groupe mono-, bi- ou tricyclique aromatique de 5 à 14 chaînons contenant de 1 à 8 hétéroatomes choisis parmi O, S ou N.
   A titre d'exemples d'hétéroaryle monocyclique, on peut citer les groupes imidazolyle, pyrazolyle thiazolyle, oxazolyle, isothiazolyle, isoxazolyle, furanyle, thiophényle, oxadiazolyle, thiadiazolyle, triazolyle, tétrazolyle, pyridinyle, pyrazinyle, pyrimidinyle, pyridazinyle, triazinyle ;
   à titre d'exemples d'hétéroaryle bicyclique, on peut citer les groupes indolyle, isoindolyle benzofuranyle, benzothiophényle, benzoxazolyle, benzimidazolyle, indazolyle, benzothiazolyle, isobenzofuranyle, isobenzothiazolyle, pyrrolo[2,3-c]pyridinyle, pyrrolo[2,3-*b*]pyridinyle, pyrrolo[3,2-*b*]pyridinyle, pyrrolo[3,2-*c*]pyridinyle, quinoléinyle, isoquinoléinyle, cinnolinyle, quinazolinyle ou quinoxalinyle ;
   à titre d'exemples d'hétéroaryle tricyclique, on peut citer les groupes pyrido[1,2-a]benzimidazolyle, thiazolo[1,2-*a*]benzimidazolyle, imidazo[1,2-a]benzimidazolyle , pyrimido[1,2-*a*]benzimidazolyle ou pyrazino[1,2-a]benzimidazolyle ;
- un silanyle : un groupe -Si(X₁)(X₂)(X₃) où X₁, X₂ et X₃ sont tels que définis dans la formule générale (I) ;
- un atome d'halogène : un fluor, un chlore, un brome ou un iode ;
- « oxo » signifie « =O » ;
- « thio » signifie « =S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.
Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Parmi les composés de formule générale (I) objets de l'invention, un premier sous-groupe de composés est constitué par les composés pour lesquels
l'un de G₁, G₂, G₃ et G₄ représente un groupe C-X où X est un groupe -Si(X₁)(X₂)(X₃) ; les autres de G₁, G₂, G₃ et G₄ représentent un groupe CH ;
X₁, X₂, X₃ représentent, indépendamment l'un de l'autre, un groupe C₁-C₆-alkyle, C₃-C₇-cycloalkyle ou aryle.

Parmi les composés de formule générale (I) objets de l'invention, un second sous-groupe de composés est constitué par les composés pour lesquels
l'un de G₁, G₂, G₃ et G₄ représente un groupe C-X où X est un groupe -Si(X₁)(X₂)(X₃) ; les autres de G₁, G₂, G₃ et G₄ représentent un groupe CH ;
X₁, X₂, X₃ représentent, indépendamment l'un de l'autre, un groupe méthyle, éthyle, isopropyle, ter-butyle, cyclohexyle ou phényle.

Parmi les composés de formule générale (I) objets de l'invention, un troisième sous-groupe de composés est constitué par les composés pour lesquels n est égal à 1.

Parmi les composés de formule générale (I) objets de l'invention, un quatrième sous-groupe de composés est constitué par les composés pour lesquels
Y représente un aryle ou un hétéroaryle ; l'aryle ou l'hétéroaryle étant éventuellement substitué par un ou plusieurs groupes choisis parmi un atome d'halogène ou un groupe C₁-C₆-fluoroalkyle.

Parmi les composés de formule générale (I) objets de l'invention, un cinquième sous-groupe de composés est constitué par les composés pour lesquels
Y représente un phényle ou un pyridinyle ; le phényle étant éventuellement substitué par un ou plusieurs groupes choisis parmi un atome de fluor ou un groupe CF₃.

Parmi les composés de formule générale (I) objets de l'invention, un sixième sous-groupe de composés est constitué par les composés pour lesquels
Z₁, Z₂, Z₃, Z₄ représentent, indépendamment l'un de l'autre, un atome d'azote ou un groupe C(R₆), l'un correspondant à un atome d'azote et les autres correspondant à un groupe C(P₆) ; R₆ représente un atome d'hydrogène.

Parmi les composés de formule générale (I) objets de l'invention, un septième sous-groupe de composés est constitué par les composés pour lesquels
Z₄ représente un atome d'azote et Z₁, Z₂, Z₃ représentent un groupe CH.

Parmi les composés de formule générale (I) objets de l'invention, un huitième sous-groupe de composés est constitué par les composés pour lesquels
Z représente une amine cyclique reliée par l'atome d'azote, de formule : 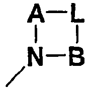 dans laquelle
A représente un groupe C₁-C₇-alkylène éventuellement substitué par un ou deux groupes R₈ ;
B représente un groupe C₁-C₇-alkylène éventuellement substitué par un ou deux groupes R₉ ;
L représente une liaison, un atome de soufre, d'oxygène ou d'azote ; l'atome d'azote étant éventuellement substitué par un groupe R₁₀ ou R₁₁ ;
les atomes de carbone de l'amine cyclique Z étant éventuellement substitués par un ou plusieurs groupes R₁₂ identiques ou différents l'un de l'autre ;
R₈, R₉, R₁₀, R₁₁ et R₁₂ étant tels que définis dans la formule générale (I).

Parmi les composés de formule générale (I) objets de l'invention, un neuvième sous-groupe de composés est constitué par les composés pour lesquels
Z représente une amine cyclique reliée par l'atome d'azote, de formule : 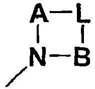 dans laquelle
A représente un groupe C₁-C₇-alkylène ;
B représente un groupe C₁-C₇-alkylène ;
L représente une liaison, un atome de soufre, d'oxygène ou d'azote ; l'atome d'azote étant éventuellement substitué par un groupe R₁₁ ;
les atomes de carbone de l'amine cyclique Z étant éventuellement substitués par un ou plusieurs groupes R₁₂ identiques ou différents l'un de l'autre ;
R₁₁ et R₁₂ étant tels que définis dans la formule générale (I).

Parmi les composés de formule générale (I) objets de l'invention, un dixième sous-groupe de composés est constitué par les composés pour lesquels
Z représente une amine cyclique reliée par l'atome d'azote, de formule : 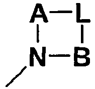 dans laquelle
A représente un groupe C₁-C₇-alkylène ;
B représente un groupe C₁-C₇-alkylène ;
L représente une liaison ;
les atomes de carbone de l'amine cyclique Z étant éventuellement substitués par un ou plusieurs groupes R₁₂ identiques ou différents l'un de l'autre ;
R₁₂ étant tel que défini dans la formule générale (I).

Parmi les composés de formule générale (I) objets de l'invention, un onzième sous-groupe de composés est constitué par les composés pour lesquels
Z représente une amine cyclique reliée par l'atome d'azote, de formule : 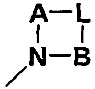 dans laquelle
A représente un groupe C₁-C₃-alkylène
B représente un groupe C₁-C₃-alkylène ;
L représente une liaison ;
les atomes de carbone de l'amine cyclique Z étant éventuellement substitués par un ou plusieurs groupes R₁₂ identiques ou différents l'un de l'autre ;
R₁₂ représente un groupe hydroxyle.

Parmi les composés de formule générale (I) objets de l'invention, un douzième sous-groupe de composés est constitué par les composés pour lesquels
Z représente un groupe azétidinyle ou pyrrolidinyle ;
les atomes de carbone de l'amine cyclique Z étant éventuellement substitués par un ou plusieurs groupes R₁₂ identiques ou différents l'un de l'autre ;
R₁₂ représente un groupe hydroxyle.

Parmi les composés de formule générale (I) objets de l'invention, un treizième sous-groupe de composés est constitué par les composés pour lesquels
Z représente une amine acyclique, reliée par l'atome d'azote, de formule NRaRb dans laquelle Ra et Rb représentent, indépendamment l'un de l'autre, un atome d'hydrogène ou un groupe C₁-C₆-alkyle.

Parmi les composés de formule générale (I) objets de l'invention, un quatorzième sous-groupe de composés est constitué par les composés pour lesquels
Z représente une amine acyclique, reliée par l'atome d'azote, de formule NRaRb dans laquelle Ra et Rb représentent, indépendamment l'un de l'autre, un groupe C₁-C₆-alkyle, plus particulièrement méthyle.

Parmi les composés de formule générale (I) objets de l'invention, un quinzième sous-groupe de composés est constitué par les composés pour lesquels les définitions de G₁, G₂, G₃ et G₄, n, Y, Z₁, Z₂, Z₃, Z₄ et Z données ci-dessus sont combinées.

Parmi les composés de formule générale (I) objets de l'invention, un seizième sous-groupe de composés est constitué par les composés pour lesquels l'un de G₁, G₂, G₃ et G₄ représente un groupe C-X où X est un groupe -Si(X₁)(X₂)(X₃) ; les autres de G₁, G₂, G₃ et G₄ représentent un groupe CH ;
X₁, X₂, X₃ représentent, indépendamment l'un de l'autre, un groupe C₁-C₆-alkyle, C₃-C₇-cycloalkyle ou aryle ;
n est égal à 1 ;
Y représente un aryle ou un hétéroaryle ; l'aryle ou l'hétéroaryle étant éventuellement substitué par un ou plusieurs groupes choisis parmi un atome d'halogène ou un groupe C₁-C₆-fluoroalkyle ;
Z₁, Z₂, Z₃, Z₄ représentent, indépendamment l'un de l'autre, un atome d'azote ou un groupe C(R₆), l'un correspondant à un atome d'azote et les autres correspondant à un groupe C(R₆) ; R₆ représente un atome d'hydrogène ;
Z représente
soit une amine cyclique reliée par l'atome d'azote, de formule : 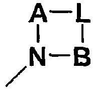 dans laquelle
A représente un groupe C₁-C₃-alkylène ;
B représente un groupe C₁-C₃-alkylène ;
L représente une liaison ;
les atomes de carbone de l'amine cyclique Z étant éventuellement substitués par un ou plusieurs groupes R₁₂ identiques ou différents l'un de l'autre ;
R₁₂ représente un groupe hydroxyle ;
soit une amine acyclique, reliée par l'atome d'azote, de formule NRaRb dans laquelle Ra et Rb représentent, indépendamment l'un de l'autre, un atome d'hydrogène ou un groupe C₁-C₆-alkyle.

Parmi les composés de formule générale (I) objets de l'invention, on peut notamment citer les composés suivants :
1. *N*-[6-(azétidin-1-yl)pyridin-3-yl]-6-triméthylsilyl-1-[(3-fluorophényl)méthyl]-1*H-*indole-2-carboxamide
2. *N*-[6-(3-hydroxyazétidin-1-yl)pyridin-3-yl]-6-triméthylsilyl-1-[(3-fluorophényl)méthyl]-1*H*-indole-2-carboxamide
3. *N*-[6-(azétidin-1-yl)pyridin-3-yl]-5-triméthylsilyl-1-[(3-fluorophényl)méthyl]-1H-indole-2-carboxamide
4. *N*-[6-(3-hydroxyazétidin-1-yl)pyridin-3-yl]-5-triméthylsilyl-1-[(3-fluorophényl)méthyl]-1*H*-indole-2-carboxamide
5. *N*-[6-(azétidin-1-yl)pyridin-3-yl]-7-triméthylsilyl-1-[(3-fluorophényl)méthyl]-1*H-*indole-2-carboxamide
6. *N*-[6-(3-hydroxyazétidin-1-yl)pyridin-3-yl]-7-triméthylsilyl-1-[(3-fluorophényl)méthyl]-1*H*-indole-2-carboxamide
7. *N*-[6-(Diméthylamino)pyridin-3-yl]-5-triméthylsilyl-1-[(3-fluorophényl)méthyl]-1*H-*indole-2-carboxamide
8. *N*-[6-(azétidin-1-yl)pyridin-3-yl]-5-(éthyl)diméthylsilyl-1-[(3-fluorophényl)méthyl]-1*H*-indole-2-carboxamide
9. *N*-[6-(azétidin-1-yl)pyridin-3-yl]-5-(cyclohexyl)diméthylsilyt-1-[(3-fluorophényl)méthyl]-1 *H*-indole-2-carboxamide
10. *N*-[6-(azétidin-1-yl)pyridin-3-yl]-6-triméthylsilyl-1-[[(3-trifluorométhyl)phényl]méthyl]-1 *H*-indole-2-carboxamide
11. *N*-[6-(3-hydroxyazétidin-1-yl)pyridin-3-yl]-6-triméthylsilyl-1-[[(3-trifluorométhyl)phényl]méthyl]-1*H*-indole-2-carboxamide
12. N-[6-(azétidin-1-yl)pyridin-3-yl]-5-(diphényl)méthylsilyl-1-[(3-fluorophényl)méthyl]-1*H*-indole-2-carboxamide
13. *N*-[6-(azétidin-1-yl)pyridin-3-yl]-5-triméthylsilyl-1-[[(3-trifluorométhyl)phényl]méthyl]-1*H*-indole-2-carboxamide
14. *N*-[6-(3-hydroxyazétidin-1-yl)pyridin-3-yl]-5-triméthylsilyl-1-[[(3-trifluorométhyl)phényl]méthyl]-1*H*-indole-2-carboxamide
15. *N*-[6-(3-hydroxyazétidin-1-yl)pyridin-3-yl]-6-triméthylsilyl-1-[(pyridin-4-yl)méthyl]-1 *H*-indole-2-carboxamide
16. *N*-[6-(azétidin-1-yl)pyridin-3-yl)pyridin-3-yl]-6-triméthylsilyl-1-[(pyridin-4-yl)méthyl]-1*H-*indole-2-carboxamide
17. *N*-[6-(azétidin-1-yl)pyridin-3-yl]-5-triéthylsilyl-1-[(3-fuorophényl)méthyl]-1*H-*indole-2-carboxamide
18. *N*-[6-(azétidin-1-yl)pyridin-3-yl]-5-tert-butyl(diméthyl)silyl-1-[(3-fluorophényl)méthyl]-1*H*-indole-2-carboxamide
19. *N*-[6-(Pyrrolidin-1-yl)pyridin-3-yl]-5-(cyclohexyl)diméthylsilyl-1-[(3-fluorophényl)méthyl]-1*H*-indole-2-carboxamide
20. *N*-[6-(azétidin-1-yl)pyridin-3-yl]-5-phényldiméthylsilyl-1-[(3-fluorophényl)méthyl]-1*H*-indole-2-carboxamide
21. *N*-[6-(azétidin-1-yl)pyridin-3-yl)-5-(isopropyl)diméthylsilyl-1-[(3-fluorophényl)méthyl]-1*H*-indole-2-carboxamide
22. *N*-[6-(Azétidin-1-yl)pyridin-3-yl]-5-(méthyl)diéthylsilyl-1-[(3-fluorophényl)méthyl]-1*H*-indole-2-carboxamide

Parmi les composés de formule générale (I) objets de l'invention, un dix-septième sous-groupe de composés est constitué par les composés pour lesquels
G₁, G₂, G₃ et G₄ représentent, indépendamment l'un de l'autre, un groupe C-X ;
X est choisi parmi un atome d'hydrogène ou un groupe -Si(X₁)(X₂)(X₃) ;
l'un des G₁, G₂, G₃ et G₄ et au maximum l'un des G₁, G₂, G₃ et G₄ représente un groupe C-X où X est un groupe -Si(X₁)(X₂)(X₃);
X₁, X₂, X₃ représentent, indépendamment l'un de l'autre, un groupe C₁-C₆-alkyle.

Parmi les composés de formule générale (I) objets de l'invention, un dix-huitième sous-groupe de composés est constitué par les composés pour lesquels n est égal à 1 et Y représente un aryle, plus particulièrement un phényle, éventuellement substitué par un ou plusieurs atomes d'halogène, plus particulièrement de fluor.

Parmi les composés de formule générale (I) objets de l'invention, un dix-neuvième sous-groupe de composés est constitué par les composés pour lesquels Z₁, Z₂, 2₃, Z₄ représentent, indépendamment l'un de l'autre, un atome d'azote ou un groupe C(R₆), l'un correspondant à un atome d'azote et les autres correspondant à un groupe C(R₆) ; où R₆ représente un atome d'hydrogène.

Parmi les composés de formule générale (I) objets de l'invention, un vingtième sous-groupe de composés est constitué par les composés pour lesquels
Z représente une amine cyclique reliée par l'atome d'azote, de formule : 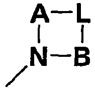 dans laquelle
A représente un groupe C₁-C₇-alkylène éventuellement substitué par un ou deux groupes R₈;
B représente un groupe C₁-C₇-alkylène éventuellement substitué par un ou deux groupes R₉ ;
L représente une liaison, un atome de soufre, d'oxygène ou d'azote ; l'atome d'azote étant éventuellement substitué par un groupe R₁₀ ou R₁₁ ;
les atomes de carbone de l'amine cyclique Z étant éventuellement substitués par un ou plusieurs groupes R₁₂ identiques ou différents l'un de l'autre.

Parmi les composés de formule générale (I) objets de l'invention, un vingt-et-unième sous-groupe de composés est constitué par les composés pour lesquels
Z représente une amine cyclique reliée par l'atome d'azote, de formule : 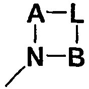 cette amine cyclique étant un groupe azétidinyle ;
les atomes de carbone de l'amine cyclique Z étant éventuellement substitués par un groupe R₁₂; R₁₂ représente un groupe hydroxyle.

Parmi les composés de formule générale (I) objets de l'invention, un vingt-deuxième sous-groupe de composés est constitué par les composés pour lesquels
G₁, G_{2,} G₃ et G₄ représentent, indépendamment l'un de l'autre, un groupe C-X ;
X est choisi parmi un atome d'hydrogène ou un groupe -Si(X₁)(X₂)(X₃) ;
l'un des G₁, G₂, G₃ et G₄ et au maximum l'un des G₁, G₂, G₃ et G₄ représente un groupe C-X où X est un groupe -Si(X₁)(X₂)(X₃) ;
X₁, X₂, X₃ représentent, indépendamment l'un de l'autre, un groupe C₁-C₆-alkyle ;
n est égal à 1 ;
Y représente un aryle, plus particulièrement un phényle, éventuellement substitué par un ou plusieurs atomes d'halogène, plus particulièrement de fluor ;
Z₁, Z₂, Z₃, Z₄ représentent, indépendamment l'un de l'autre, un atome d'azote ou un groupe C(R₆), l'un correspondant à un atome d'azote et les autres correspondant à un groupe C(R₆) ; où R₆ représente un atome d'hydrogène ;
Z représente une amine cyclique reliée par l'atome d'azote, de formule : 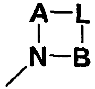 cette amine cyclique étant un groupe azétidinyle ;
les atomes de carbone de l'amine cyclique Z étant éventuellement substitués par un groupe R₁₂; où R₁₂ représente un groupe hydroxyle.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.
Selon le schéma 1, les composés de formule générale (IV) peuvent être obtenus par réaction d'un composé de formule générale (II) dans laquelle G₁, G₂, G₃ et G₄ sont tels que définis dans la formule générale (I) et B représente un groupe C₁-C₆-alcoxyle, avec un composé de formule générale (III), dans laquelle Y et n sont tels que définis dans la formule générale (I) et GP représente un groupe partant ou GP représente un groupe hydroxyle.
Dans le schéma 1, lorsque le composé de formule générale (III), est défini tel que n est égal à 1, 2 ou 3 et GP représente un groupe partant tel qu'un atome de chlore, de brome ou d'iode, la réaction de formation des composés de formule générale (IV) peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1), 1).
Lorsque le composé de formule générale (III) est défini tel que n est égal à 1, 2 ou 3 et GP représente un groupe hydroxyle, les composés de formule générale (IV), peuvent être obtenus par réaction du composé de formule générale (II) avec un composé de formule générale (III) en présence d'une phosphine telle que, par exemple, la triphénylphosphine et d'un réactif tel que, par exemple, l'azodicarboxylate de diéthyle en solution dans un solvant tel que le dichlorométhane ou le tétrahydrofuranne (O. Mitsonobu, Synthesis, 1981, 1-28).

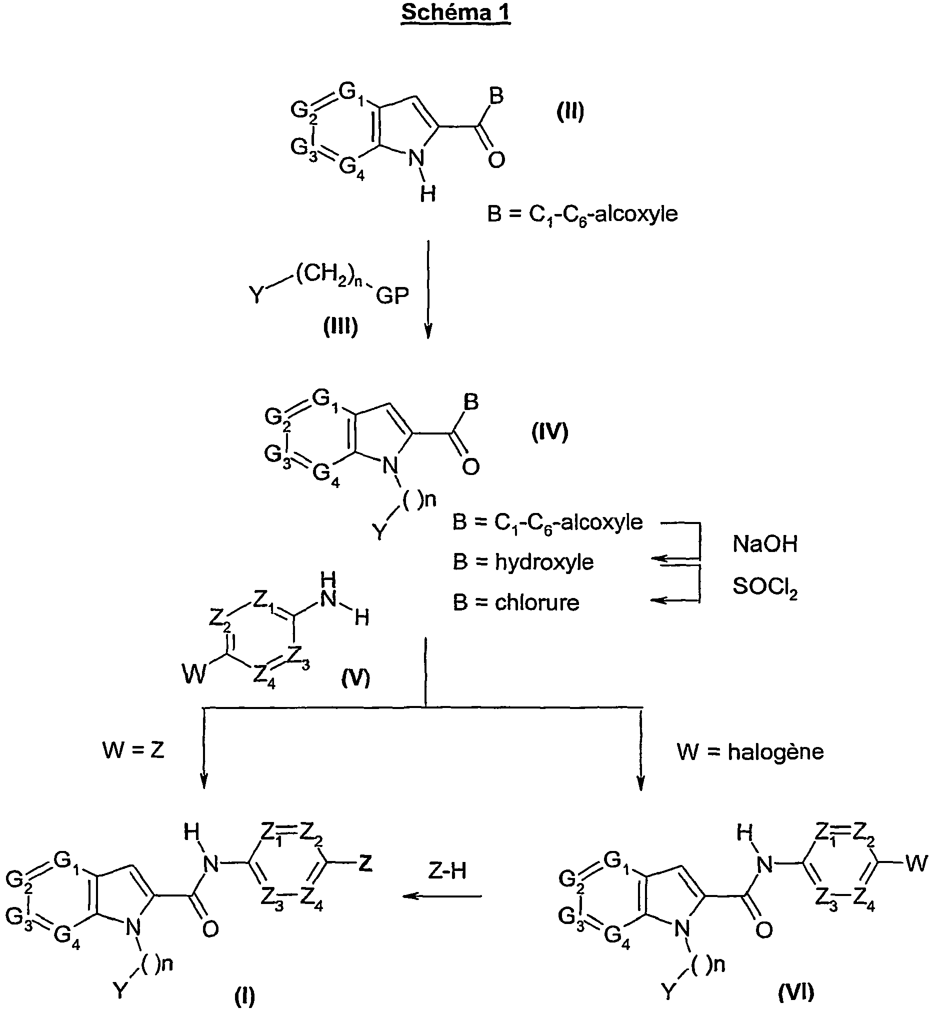

De façon analogue, les composés de formule générale (IV), peuvent être obtenus par réaction du composé de formule générale (II) avec un composé de formule générale (III) en présence d'une phosphine supportée sur une résine et d'un réactif tel que, par exemple, l'azodicarboxylate de diisopropyle en solution dans un solvant tel que le dichlorométhane ou le tétrahydrofuranne.

Lorsque le composé de formule générale (III) est défini tel que n est égal à 0 et GP représente un groupe partant tel qu'un atome de chlore, de brome ou d'iode, la réaction de formation des composés de formule générale (IV) peut être réalisée par application et adaptation des méthodes décrites par S. L. Buchwald et al. (J. Am. Chem. Soc., 2001, 123, 7727 et 2002, 124, 11684), de préférence sous atmosphère inerte en milieu basique, par exemple en présence de triphosphate de potassium, en présence d'un sel de cuivre tel que l'iodure de cuivre, éventuellement en présence d'un additif tel que la *N,N*'-diméthylcyclohexane-1,2-diamine, le tout dans un solvant organique tel que le toluène.

Le composé de formule générale (IV), pour lequel B représente un groupe C₁-C₆-alcoxyle, peut être transformé en composé de formule générale (IV), où B représente un groupe hydroxyle, par l'action d'une base telle que la soude ou la potasse en solution dans un solvant tel que l'éthanol. Le composé de formule générale (IV), où B représente un groupe hydroxyle peut, par la suite, être transformé en composé de formule générale (IV), où B représente un atome de chlore, par l'action d'un agent chlorant tel que le chlorure de thionyle dans un solvant tel que le dichlorométhane.

Le composé de formule générale (VI) peut ensuite être obtenu, par exemple, par réaction d'un composé de formule générale (IV) où B est un atome de chlore, tel qu'obtenu ci-dessus, avec une amine de formule générale (V), dans laquelle Z₁, Z₂, Z₃, Z₄ sont tels que définis dans la formule générale (I) et W correspond à un halogène tel qu'un atome de chlore, dans un solvant tel que le dichloroéthane, le toluène ou le tétrahydrofuranne.

Le composé de formule générale (VI) peut également être obtenu par réaction d'un composé de formule générale (IV) où B est un groupe hydroxyle, tel qu'obtenu ci-dessus, avec une amine de formule générale (V), dans laquelle Z₁, Z₂, Z₃, Z₄ sont tels que définis dans la formule générale (I) et W correspond à un atome d'halogène tel qu'un atome de chlore, en présence d'un agent de couplage tel que le cyanophosphonate de diéthyle ou le *N*-(3-diméthylaminopropyl)-*N*-éthylcarbodiimide, éventuellement en présence d'une base telle que la triéthylamine, dans un solvant tel que le diméthylformamide.

Le composé de formule générale (I) peut être obtenu, par exemple, par réaction d'un composé de formule générale (IV) où B est un atome de chlore, tel qu'obtenu ci-dessus, avec une amine de formule générale (V), dans laquelle W = Z et Z, Z₁, Z₂, Z₃, Z₄ sont tels que définis dans la formule générale (I), dans un solvant tel que le dichloroéthane, le toluène ou le tétrahydrofuranne.

Le composé de formule générale (I) peut également être obtenu par réaction d'un composé de formule générale (IV) où B est un groupe hydroxyle, tel qu'obtenu ci-dessus, avec une amine de formule générale (V), dans laquelle W = Z et Z, Z₁, Z₂, Z₃, Z₄ sont tels que définis dans la formule générale (I), en présence d'un agent de couplage tel que le diéthylcyanophosphonate ou le *N*-(3-diméthylaminopropyl)-*N*'-éthylcarbodiimide, et éventuellement en présence d'une base telle que la triéthylamine, dans un solvant tel que le diméthylformamide.

Le composé de formule générale (I) peut également être obtenu par réaction d'un composé de formule générale (VI) en présence d'une amine de formule Z-H, dans laquelle Z est tel que défini dans la formule générale (I), sans solvant ou dans un solvant tel que la N-méthylpyrrolidone, ou bien par application et adaptation des méthodes décrites par S. L. Buchwald et al. (J. Am. Chem. Soc., 2001, 123, 7727 et 2002, 124, 11684), de préférence sous atmosphère inerte en milieu basique, par exemple en présence de triphosphate de potassium, en présence d'un sel de cuivre tel que l'iodure de cuivre, éventuellement en présence d'un additif tel que la *N,N*'-diméthylcyclohexane-1,2-diamine, le tout dans un solvant organique tel que le toluène, ou bien par application et adaptation des méthodes décrites par Hartwig et al. (Angewandte Chemie, 2005, 44, 1371-1375), par exemple en présence d'une base et de quantités catalytiques d'un catalyseur à base de palladium, tel que le palladium diacétate, et d'une phosphine.

Les composés de formule générale (II) du schéma 1, dans laquelle G₁, G₂, G₃ et G₄ sont tels que définis dans la formule générale (I) et B représente un groupe C₁-C₆-alcoxyle, quand leur mode de préparation n'est pas décrit, peuvent être obtenus, par exemple, selon la méthode illustrée dans le schéma 2.

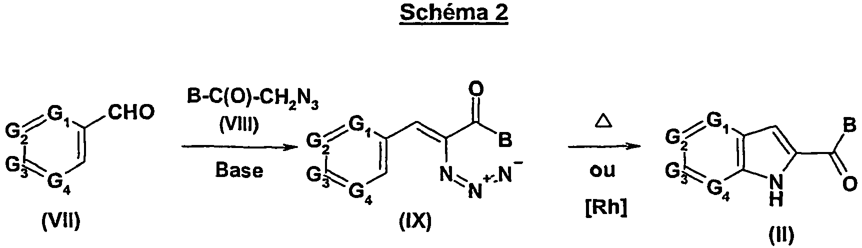

Les composés de formule générale (II) sont préparés à partir d'aldéhydes aromatiques ou hétéroaromatiques substitués par un groupe silanyle de formule générale (VII) dans laquelle G₁, G₂, G₃ et G₄ sont tels que définis dans la formule générale (I), par réaction avec un azidoacétate d'alkyle de formule générale (VIII) dans laquelle B représente un groupe C₁-C₆-alcoxyle, tel que l'azidoacétate d'éthyle par exemple, en présence d'une base telle que l'éthoxide de sodium, dans un solvant comme l'éthanol ou le méthanol, pour conduire aux azido-2-cinnamates d'alkyles de formule générale (IX). Ces derniers sont ensuite transformés en ester indoliques ou azaindoliques au reflux d'un solvant, par exemple dans le xylène ou le toluène, par adaptation de protocoles décrits dans la littérature (Hemetsberger et al Monatsh. Chem., 1969, 100, 1599 et 1970, 101, 161 ; P. Roy et al Synthesis., 2005, 16, 2751-2757; R. Guilard et al J. Heterocyclic. Chem., 1981, 18, 1365-1377 ; W.Rees et al J. Chem. Soc, perkin trans 1 1984,2189-2196; P. Molina et al J. org. Chem., 2003, 68(2), 489-499; C. Moody et al J. Chem. Soc., Perkin Trans. 1 1984, 2189-2196 ; J. Sawyer et al J. Med. Chem , 2005, 48, 893-896 ; D. Tanner Synlett 2006, 18, 3140-3144).

Alternativement, selon le schéma 2 la formation des composés de formule générale (II) peut être obtenue par décomposition de l'azido-2-cinnamate d'alkyle de formule générale (IX), en présence d'un complexe dimère de rhodium, dans un solvant tel que le toluène à une température comprise entre 25°C et 60°C, selon une adaptation de protocoles décrits dans la littérature (Tom G. Drivers et al J. Am. Chem. Soc., 2007, 129, 7500-7501 ; J. Sawyer et al J. Med. Chem, 2005, 48, 893-896).

Le groupe silanyle des composés de formule générale (II) peut également être introduit par réaction de l'ester indolique ou azaindolique correspondant substitué par un halogène, tel qu'un brome ou un iode, dans la position où le groupe silanyle doit être introduit, en présence d'un disilane, tel que l'hexaméthyldisilane, d'une quantité catalytique d'un complexe métallique, de préférence un complexe de palladium comme par exemple le tetrakis(triphénylphosphine)palladium, et d'une base, dans un solvant, tel que le toluène, à une température comprise entre 20°C et la température d'ébullition du solvant.

Le groupe silanyle des composés de formule générale (II) peut également être introduit par réaction de l'ester indolique ou azaindolique correspondant substitué par un halogène, tel qu'un iode, dans la position où le groupe silanyle doit être introduit, en présence d'un silane, tel que le triéthylsilane, d'une quantité catalytique d'un complexe métallique, de préférence un complexe de palladium comme par exemple le bis(tri-tert butylphosphine)palladium, et d'une base comme le triphosphate de potassium, dans un solvant, tel que le N-méthyl-2-pyrrolidinone à une température proche de 20°C (adaptation des protocoles décrits dans la littérature : Yoshinori Yamanoi et al J. org. Chem., 2005, 70 (23), 9607-9609 et références cités).

Les aldéhydes aromatiques ou hétéroaromatiques substitués par un groupe silanyle de formule générale (VII), quand ils ne sont pas disponibles dans le commerce, peuvent être obtenus à partir des aldéhydes aromatiques ou hétéroaromatiques correspondants, de préférence masqués sous la forme d'un acétal par exemple, substitués par un atome d'halogène tel qu'un brome ou un iode, dans la position où le groupe silanyle doit être introduit :
- par exemple par réaction avec un disilane tel que l'hexaméthyldisilane, en présence d'une quantité catalytique d'un complexe métallique, de préférence un complexe de palladium, comme par exemple le tétrakis(triphénylphosphine)palladium, sans solvant ou dans un solvant, de préférence polaire comme par exemple l'hexaméthylphosphoramide, en présence d'une base comme le carbonate de potassium, à une température comprise entre 20°C et la température d'ébullition du solvant (adaptation des protocoles décrits dans la littérature : J. Babin et al J. organométal. Chem., 1993, 446 (1-2), 135-138 ; E. Shirakawa et al Chem. Commun., 2000, 1895-1896 ; L. Goossen et al Synlett, 2000, 1801-1803; H. Matsumoto et al J. organométal. Chem., 1975, 85, C1 ; FR2677358).
- par exemple par réaction avec un disilane tel que l'hexaméthyldisilane, en présence d'une base forte, comme par exemple le méthoxide de potassium ou de sodium, dans un solvant polaire comme par exemple l'hexaméthylphosphoric triamide (HMPT), à une température proche de 20°C (adaptation des protocoles décrits dans la littérature : A.I Meyers et al J. org. Chem., 1977, 42 (15), 2654-2655 ; K. Ishimaru et al Heterocycles., 2001, 55 (8), 1591-1597).
- par exemple par réaction avec un silane tel que le triéthylsilane, en présence d'une quantité catalytique d'un complexe métallique, de préférence un complexe de palladium, comme par exemple le bis(tri-tert butylphosphine)palladium, et d'une base comme le triphosphate de potassium, dans un solvant, tel que le N-méthyl-2-pyrrolidinone à une température proche de 20°C (adaptation des protocoles décrits dans la littérature : Yoshinori Yamanoi et al J. org. Chem., 2005, 70 (23), 9607-9609 et références cités).

Les aldéhydes aromatiques ou hétéroaromatiques substitués par un groupe silanyle de formule générale (VII) selon le schéma 2 quand ils ne sont pas disponibles dans le commerce, peuvent également être obtenus à partir des aromatiques ou hétéroaromatiques dihalogénés correspondants, tel qu'un dérivé dibromé, dans la position où le groupe silanyle doit être introduit, par échange avec un organométallique, comme par exemple le n-butyllithium. Les aromatiques ou hétéroaromatiques métalliques ainsi formés peuvent ensuite réagir avec des organohalogénosilanes ou être transformés en dérivés formylés par adaptation des méthodes décrits dans la littérature. La réaction est menée de préférence à des températures basses comprises entre - 110°C et la température ambiante, dans un solvant comme l'éther ou le THF (adaptation des protocoles décrits dans la littérature : Bao-Hui Ye et al Tetrahedron., 2003, 59, 3593-3601 ; P. Pierrat et al Synlett 2004, 13, 2319-2322 ; K.T.Warner et al Heterocycles 2002, 58, 383 ; D. Deffieux et al J. organométal. Chem., 1994, 13 (6), 2415-2422 ; WO2005080328 ; S.G.Davies et al J. Chem. Soc, perkin trans 1 1991, 501 ; G. Queguiner et al J. Org. Chem. , 1981, 46, 4494-4497 ; G. Breton et al. Tetrahedron 2000, 56 (10), 1349-1360 ; S. De Montis et al. Tetrahedron 2004, 60 (17), 3915-3920 ; L. Buchwald et al J. Am. Chem. Soc., 1998, 120, 4960-4976).

Selon le schéma 3, le groupe silanyle de G₁, G₂, G₃ ou G₄ des composés de formule générale (I) peut également être introduit en partant des composés de formule générale (X), dans laquelle A₁, A₂, A₃ et A₄ représentent, indépendamment l'un de l'autre, un groupe C-X ou un atome d'azote ; X étant tel que défini dans la formule générale (I) ; l'un des A₁, A₂, A₃, A₄ et au maximum l'un des A₁, A₂, A₃, A₄ représente un groupe C-q où q est un atome d'halogène, tel qu'un iode ou un brome, dans la position où le groupe silanyle doit être introduit. Par exemple, la réaction peut être réalisée en présence d'un silane, tel que le triéthylsilane, d'une quantité catalytique d'un complexe métallique, de préférence un complexe de palladium comme par exemple le bis(tri-tert butylphosphine)palladium, et d'une base comme le triphosphate de potassium, de préférence sous atmosphère inerte, dans un solvant, tel que le N-méthyl-2-pyrrolidinone à une température proche de 20°C ou bien par application et adaptation des méthodes décrites par Yoshinori Yamanoi et al. J. org. Chem., 2005, 70 (23), 9607-9609 et références cités.

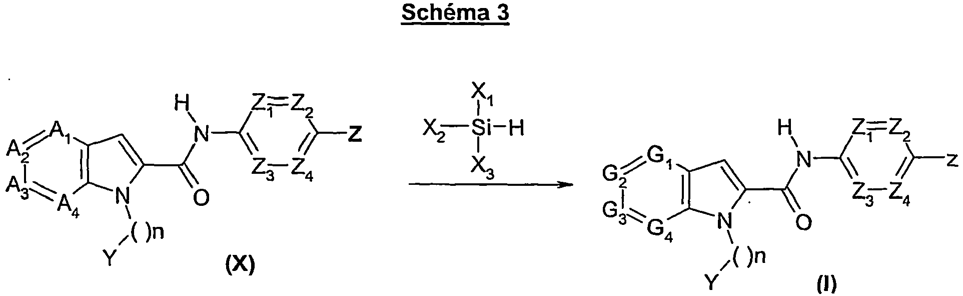

Les composés de formule générale (X) tels que définis ci-dessus peuvent être obtenus par exemple par analogie à la préparation des composés de formule générale (I) précédemment décrites ou par adaptation des protocoles décrits dans la littérature (WO2007010138) et connus de l'homme de l'art.

Les composés de formule générale (I) dans lesquelles Z représente une amine acyclique NRaRb correspondant à un groupe NH₂ peuvent être obtenus, selon des conditions connues de l'homme de l'art et décrites dans la littérature (Greene, Wuts, Protective groups in organic synthesis, Wiley-Interscience) à partir de précurseurs de formule générale (I) où NRaRb = NH-GP, GP correspondant à un groupe protecteur tel qu'un groupe acétyle ou terbutoxycarbonyle.

Les composés de formule générale (I) comportant un groupe CO₂H peuvent être obtenus, selon des conditions connues de l'homme de l'art et décrites dans la littérature (Greene, Wuts, Protective groups in organic synthesis, Wiley-Interscience) à partir de précurseurs de formule générale (I) comportant un groupe CO₂GP, GP correspondant à un groupe protecteur d'acide carboxylique tel qu'un groupe méthyle ou terbutyle.
Les composés de formule générale (I) comportant un groupe CH₂OH peuvent être obtenus, selon des conditions connues de l'homme de l'art à partir de composés de formule générale (I) comportant un groupe CO₂alkyle, par exemple, par réaction en présence d'un agent réducteur tel que le borohydrure de sodium dans un solvant tel que le tétrahydrofuranne.

Les composés de formule générale (III) sont disponibles dans le commerce, décrits dans la littérature (Carling R.W. et al J.Med.Chem. 2004 (47), 1807 - 1822 ou Russel M.G.N. et al. J.Med.Chem. 2005 (48), 1367 - 1383) ou accessibles en utilisant des méthodes connues de l'homme du métier.

Les composés de formule générale (V) et les autres réactifs, quand leur mode de préparation n'est pas décrit, sont disponibles dans le commerce ou décrits dans la littérature (WO2005028452, WO2002048152, WO2006040522, WO2004052869, WO2004062665, WO2005035526, WO2004110986, Heterocycles 1977, 6(12), 1999 - 2004, par exemple).

L'invention, selon un autre de ses aspects, a également pour objet les composés de formules générales (IVa), (IVb), (IVc), (IVd), (IVe), (IVf). Ces composés sont utiles comme intermédiaires de synthèse des composés de formule (I).

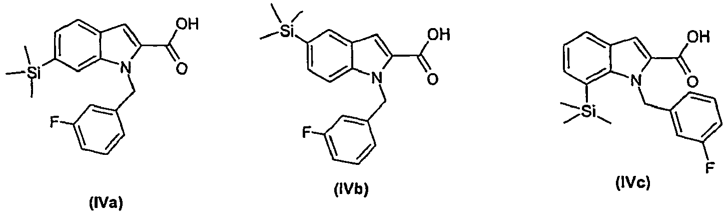

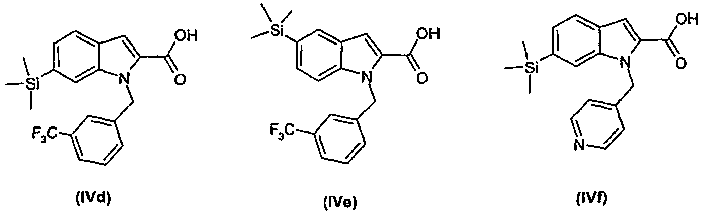

Les acides (IVa), (IVb), (IVc), (IVd), (IVe), (IVf) sont préparés selon les procédés décrits dans les exemples n°1, 3, 5, 10, 13, 15.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 1. Les microanalyses élémentaires, les analyses LC-MS (chromatographie liquide couplée à la spectrométrie de masse) les spectres I.R. ou les spectres R.M.N. confirment les structures des composés obtenus.

### Exemple 1 (Composé N°1)

### N-[6-(azétidin-1-yl)pyridin-3-yl]-6-triméthylsilyl-1-[(3-fluorophényl)méthyl]-1H-indole-2-carboxamide

### 1.1. 2-Azido-3-(4-triméthylsilylphényl)propénoate d'éthyle

Dans un ballon de 100 mL, muni d'un agitateur magnétique et maintenu sous atmosphère d'azote sont introduits 1,26 g (54,96 mmoles) de sodium et 30 mL d'éthanol anhydre. Le mélange réactionnel est agité à température ambiante jusqu'à obtention d'une solution homogène. A cette solution refroidie à -10°C, on ajoute, goutte à goutte, une solution contenant 16,83 mL (54,96 mmoles) d'azidoacétate d'éthyle (34% dans le CH₂Cl₂) et 5 g (27,48 mmoles) de 4-triméthylsilylbenzaldehyde dans 5 mL d'éthanol. On agite ensuite le mélange réactionnel à 0°C pendant 4 heures. On hydrolyse le milieu réactionnel par ajout sous agitation vigoureuse de 100 mL d'une solution de chlorure d'ammonium (30% aqueuse). On extrait le produit avec trois fois 50 mL d'acétate d'éthyle. On lave les phases organiques rassemblées avec deux fois 20 mL d'eau, on sèche sur sulfate de sodium et on concentre sous pression réduite. On purifie l'huile résultante par chromatographie sur colonne de gel de silice en éluant avec un mélange d'heptane et de dichlorométhane. On isole 4,96 g du produit attendu sous forme d'une huile jaune.
R.M.N. ¹H (DMSO D₆), δ (ppm) : 7,6 (d, 2H) ; 7,35 (d, 2H) ; 6,7 (s, 1H) ; 4,1 (q, 2H) ; 1,1 (t, 3H) ; 0 (s, 9H).

### 1.2 6-Triméthylsilyl-1H-indole-2-carboxylate d'éthyle

A une solution de 1,0 g (3,14 mmoles) de 2-azido-3-(4-triméthylsilylphényl)propénoate d'éthyle obtenu à l'étape 1.1, dans 20 mL de toluène sec, maintenue sous atmosphère inerte, est ajouté 0,17 g (0,16 mmole) du complexe dimère d'heptafluorobutyrate de dirhodium. Le mélange réactionnel est alors agité pendant 12 h à 70°C. Après retour à température ambiante, on filtre le mélange réactionnel sur gel de silice en éluant avec de l'acétate d'éthyle. Le filtrat est ensuite concentré sous pression réduite. On purifie le résidu par chromatographie sur colonne de gel de silice en éluant avec un mélange d'heptane et de dichlorométhane. On isole 0,61 g du produit attendu sous la forme d'une poudre beige.
PF = 127-129°C
R.M.N. ¹H (DMSO D₆), δ (ppm) : 11,7 (s, 1 H) ; 7,41 (dd, 1 H) ; 7,39 (d, 1 H) ; 6,97 (dd, 1 H) ; 6,88 (d, 1H) ; 4,1 (q, 2H) ; 1,1 (t, 3H) ; 0,0 (s, 9H).

### 1.3. 6-Triméthylsilyl-1-[(3-fluorophényl)méthyl]-1H-indole-2-carboxylate d'éthyle

A une suspension de 0,15 g (3,79 mmole) d'hydrure de sodium (60%) dans 5 mL de DMF sec, maintenue sous atmosphère inerte, est ajoutée, goutte à goutte à température ambiante, une solution de 0,9 g (3,44 mmoles) de 6-triméthylsilyl-1*H-*indole-2-carboxylate d'éthyle obtenu selon le protocole décrit à l'étape 1.2 dans 5 mL de DMF sec. Le mélange réactionnel est agité à température ambiante pendant pendant 2 heures. On ajoute ensuite, goutte à goutte, à température ambiante, 0,82 g (4,13 mmoles) de bromure de 3-fluorobenzyle. Le mélange réactionnel est alors agité pendant 20 h à température ambiante puis dilué avec 100 mL d'un mélange d'eau et d'éther (1/1). On décante et on extrait la phase aqueuse avec 30 mL d'ether. Les phases organiques rassemblées sont lavées trois fois à l'eau, séchées sur du sulfate de sodium, filtrées et concentrées sous pression réduite. On purifie l'huile résultante par chromatographie sur colonne de gel de silice en éluant avec un mélange d'heptane et d'acétate d'éthyle. On isole 0,89 g du produit attendu.
PF = 87-88°C
R.M.N. ¹H (DMSO D₆), δ (ppm) : 7,65 (m, 1H) ; 7,35 (s, 1H) ; 7,32-7,1 (m, 3H) ; 6,9-6,6 (m, 3H) ; 5,80 (s, 2H) ; 4,25 (q, 2H) ; 1,3 (t, 3H) ; 0,2 (s, 9H).

### 1.4 Acide 6-triméthylsilyl-1-[(3-fluorophényl)méthyl]-1H-indole-2-carboxylique (Composé N° IV a)

Une solution de 0,85 g (2,30 mmoles) de 5-triméthylsilyl-1-[(3-fluorophényl)méthyl]-1*H-*indole-2-carboxylate d'éthyle, obtenu à l'étape 1.3, dans 15 mL d'éthanol et 5 mL de soude 1 N, est agitée quatre heures à reflux. Après ce temps, le mélange réactionnel est concentré sous pression réduite puis repris dans 50 mL d'eau. Le milieu est acidifié à pH 1 par ajouts successifs d'acide chlorhydrique 1 N. Le précipité est recueilli par filtration, lavé à l'eau puis séché sous pression réduite. On isole ainsi 0,65 g du produit attendu, qui est utilisé tel quel dans l'étape suivante.
PF = 189-191°C
R.M.N. ¹H (DMSO D₆), δ (ppm): 13,1 (pic élargi, 1H) ; 7,7 (m, 2H) ; 7,3 (m, 3H) ; 7,1 (m, 1H) ; 6,9 (m, 2H) ; 5,9 (s, 2H) ; 0,35 (s, 9H).

### 1.5 2-(azétidin-1-yl)-5-nitropyridine

On ajoute, goutte à goutte, 4,7 g (80,76 mmoles) d'azétidine sur une suspension agitée à 20°C de 27,9 g (0,201 mole) de carbonate de potassium et de 11 g (67,3 mmoles) de 2-chloro-5-nitropyridine dans 100 mL de diméthylformamide. Le mélange est agité 5 minutes à 20°C puis 6 heures à 70°C. Après ce temps, la suspension est versée sur un mélange de 300 mL d'eau et 300 mL d'acétate d'éthyle. La phase aqueuse est séparée puis extraite avec 200 mL d'acétate d'éthyle. Les phases organiques sont réunies, lavées trois fois avec 250 mL d'eau puis séchées sur sulfate de sodium et concentrées sous pression réduite. On obtient ainsi 11,7 g du produit attendu sous la forme d'un solide.
PF = 132 - 134°C
R.M.N. ¹H (CDCl₃), δ (ppm) : 8,92 (d, 1H) ; 8,09 (dxd, 1H) ; 6,07 (d, 1H) ; 4,12 (m, 4H) ; 2,46 (quint., 2H).

### 1.6 3-amino-6-(azétidin-1-yl)pyridine

Une suspension de 11,5 g (64,18 mmoles) de 2-(azétidin-1-yl)-5-nitropyridine, préparée à l'étape précédente, et de 1 g de Pd/C à 10% dans 400 mL d'éthanol est agitée vigoureusement à 20°C sous 5 atmosphère d'hydrogène durant 4 heures. Après ce temps, le mélange est filtré sur un tampon de célite puis concentré sous pression réduite. On obtient ainsi 9,3 g du produit attendu qui est utilisé tel quel dans la suite de la synthèse.
R.M.N. ¹H (DMSO D₆), δ (ppm) : 7,57 (d, 1H) ; 6,92 (dxd, 1H) ; 6,2 (d, 1H) ; 4,46 (pic élargi, 2H) ; 3,78 (m, 4H) ; 2,25 (quint., 2H).

### 1.7 N-[6-(azétidin-1-yl)pyridin-3-yl]-6-triméthylsilyl-1-[(3-fluorophényl)méthyl]-1H-indole-2-carboxamide (Composé n°1)

A une solution, agitée à 20°C sous atmosphère inerte, de 0,2 g (0,59 mmole) de l'acide 6-triméthylsilyl-1-[(3-fluorophényl)méthyl]-1*H*-indole-2-carboxylique préparé à l'étape 1.4 dans 5 mL de DMF, sont ajoutés, 114 mg (0,59 mmole) de chlorhydrate de *N*-(3-diméthylaminopropyl)-*N'*-éthylcarbodiimide (EDAC) et 81 mg (0,59 mmole) de 1-hydroxy-benzotriazole (HOBT). Le mélange réactionnel est agité à température ambiante pendant 15 minutes. On ajoute ensuite au mélange réactionnel, 131 mg (0,88 mmole) de 3-amino-6-(azétidin-1-yl)pyridine, préparé à l'étape 1.6. Après 16 heures d'agitation à 20°C, On verse le mélange réactionnel dans un mélange de 30 mL d'une solution aqueuse saturée en NaHCO₃ et de 30 mL d'acétate d'éthyle. On décante et on extrait la phase aqueuse par 20 mL d'acétate d'éthyle. Les phases organiques rassemblées sont lavées trois fois avec 30 mL d'eau, séchées sur sulfate de sodium puis concentrées sous pression réduite. Le produit obtenu est purifié par chromatographie sur colonne de silice en éluant avec un mélange de dichlorométhane et de méthanol. Le solide obtenu après évaporation du solvant sous pression réduite est lavé à l'ébullition de l'éther isopropylique, filtré à chaud puis séché sous pression réduite. On isole ainsi 170 mg du produit attendu.
PF = 208 - 210 °C
R.M.N. ¹H (DMSO D₆), δ (ppm) : 10,2 (s, 1H) ; 8,28 (d, 1H) ; 7,75 (dd, 1H) ; 7,6 (d, 1H) ; 7,58 (s, 1H) ; 7,2 (m, 3H) ; 7,0-6,8 (m, 3H) ; 6,3 (d, 1H) ; 5,8 (s, 2H) ; 3,8 (t, 4H) ; 2,2 (m, 2H) ; 0,15 (s, 9H).

### Exemple 2 (Composé N°2)

### N-[6-(3-hydroxyazétidin-1-yl)pyridin-3-yl]-6-triméthylsilyl-1-[(3-fluorophényl)méthyl]-1H-indole-2-carboxamide

### 2.1 2-(3-hydroxyazétidin-1-yl)-5-nitropyridine

On chauffe la nuit à 100°C un mélange de 0,311 g (2,84 mmoles) de chlorhydrate de 3-hydroxyazétidine, de 0,3 g (1,89 mmole) de 2-chloro-5-nitropyridine et de 0,41 mL (2,84 mmoles) de triéthylamine dans 10 mL de diméthylformamide. Après ce temps, la suspension est versée sur un mélange de 30 mL d'eau et 30 mL d'acétate d'éthyle. La phase aqueuse est séparée puis extraite avec 20 mL d'acétate d'éthyle. Les phases organiques sont réunies, lavées trois fois avec 25 mL d'eau puis séchées sur sulfate de sodium et concentrées sous pression réduite. On obtient ainsi 0,34 g du produit attendu sous la forme d'un solide qui sera utilisé tel quel dans la suite de la synthèse. R.M.N. ¹H (DMSO D₆), δ (ppm): 8,95 (s, 1H) ; 8,21 (dxd, 1H) ; 6,41 (d, 1H) ; 5,85 (d, 1H) ; 4,62 (m, 1H) ; 4,37 (m, 2H) ; 3,9 (m, 2H).

### 2.2 3-amino-6-(3-hydroxyazétidin-1-yl)pyridine

Une suspension de 0,34 g (1,74 mmole) de 2-(3-hydroxyazétidin-1-yl)-5-nitropyridine, préparée à l'étape précédente, et de 9 mg de Pd/C à 10% dans 10 mL d'éthanol est agitée vigoureusement à 20°C sous 5 atmosphère d'hydrogène durant 4 heures. Après ce temps, le mélange est filtré sur un tampon de célite puis concentré sous pression réduite. Le produit résultant est purifié par chromatographie sur colonne de silice en éluant avec un mélange de dichlorométhane et de méthanol. On obtient ainsi 0,1 g du produit attendu qui sera utilisé tel quel dans la suite de la synthèse.
R.M.N. ¹H (DMSO D₆), δ (ppm) : 7,58 (s, 1H) ; 6,91 (d, 1H) ; 6,22 (d, 1H) ; 5,48 (pic élargi, 1H) ; 4,48 (pic élargi, 3H) ; 3,98 (m, 2H) ; 3,49 (m, 2H).

### 2.3 N[6-(3-hydroxyazétidin-1-yl)pyridin-3-yl]-6-triméthylsilyl-1-[(3-fluorophényl)méthyl]-1H-indole-2-carboxamide (Composé n°2)

A une solution, agitée à 20°C sous atmosphère inerte, de 0,2 g (0,59 mmole) de l'acide 6-triméthylsilyl-1-[(3-fluorophényl)méthyl]-1*H*-indole-2-carboxylique, préparé à l'étape 1.4, dans 5 mL de DMF, sont ajoutés, 114 mg (0,59 mmole) de chlorhydrate de *N*-(3-diméthylaminopropyl)-*N'*-éthylcarbodiimide (EDAC) et 81 mg (0,59 mmole) de 1-hydroxy-benzotriazole (HOBT). Le mélange réactionnel est agité à température ambiante pendant 15 minutes. On ajoute ensuite au mélange réactionnel, 145 mg (0,88 mmole) de 3-amino-6-(3-hydroxyazétidin-1-yl)pyridine, préparé à l'étape 2.2. Après 20 heures d'agitation à 20°C, On verse le mélange réactionnel dans un mélange de 30 mL d'une solution aqueuse saturée en NaHCO₃ et de 30 mL d'acétate d'éthyle. On décante et on extrait la phase aqueuse par 20 mL d'acétate d'éthyle. Les phases organiques rassemblées sont lavées trois fois avec 30 mL d'eau, séchées sur sulfate de sodium puis concentrées sous pression réduite. Le produit obtenu est purifié par chromatographie sur colonne de silice en éluant avec un mélange de dichlorométhane et de méthanol. Le solide obtenu après évaporation du solvant sous pression réduite est recristallisé dans l'éther isopropylique. On isole ainsi 175 mg du produit attendu.
PF = 188 - 189 °C
R.M.N. ¹H (DMSO D₆), δ (ppm) : 10,15 (s, 1H) ; 8,25 (d, 1H); 7,7 (dd, 1H) ; 7,6 (d, 1H) ; 7,55 (s, 1H) ; 7,3-7,18 (m, 3H) ; 7,0-6,75 (m, 3H) ; 6,3 (d, 1H) ; 5,85 (s, 2H) ; 5,5 (d, 1H) ; 4,45 (m, 1H) ; 4,0 (m, 2H) ; 3,5 (m, 2H) ; 0,15 (s, 9H).

### Exemple 3 (Composé N°3)

### N-[6-(azétidin-1-yl)pyridin-3-yl]-5-triméthylsilyl-1-[(3-fluorophényl)méthyl]-1H-indole-2-carboxamide

### 3.1 1-Bromo-3-triméthylsilylbenzène

A une solution de 10 g (42,39 mmoles) de 1,3-dibromobenzène dans 80 mL d'Et₂O anhydre, refroidie à -78°C et maintenue sous atmosphère d'azote, sont ajoutés goutte à goutte sous agitation, durant 30 min, 26,49 mL (42,39 mmoles) d'une solution de BuLi (1,5M/hexane). Après 30 min d'agitation supplémentaire à -78°C, 5,96 mL (46,63 mmoles) de TMSCI sont introduits goutte à goutte dans le mélange réactionnel. L'agitation est maintenue à cette température pendant 90 min et le mélange réactionnel est ensuite hydrolysé par ajout de 15 mL d'eau. Le produit est extrait avec de l'acétate d'éthyle (3 x 50 mL). Les phases organiques rassemblées sont lavées avec une solution aqueuse saturée en NaCl (2 x 25 mL), séchées sur Na₂SO₄, filtrées et évaporées sous pression réduite. Le brut réactionnel est purifié par chromatographie sur colonne de gel de silice en éluant avec de l'heptane, pour isoler 9,3 g du 1-bromo-3-triméthylsilylbenzène attendu, sous la forme d'une huile incolore.
R.M.N. ¹H (DMSO D₆), δ (ppm) : 5,75 (s, 1H), 7,46 (m, 1H), 7,4 (m, 1H), 7,22 (t, 1H), 0,2 (s, 9H).

### 3.2 3-Triméthylsilylbenzaldehyde

A une solution de 5 g (21,89 mmoles) du 1-bromo-3-triméthylsilylbenzène préparé selon le protocole décrit à l'étape 3.1, dans 40 ml d'Et₂O anhydre, refroidie à 0°C et maintenue sous atmosphère d'azote, sont ajoutés goutte à goutte, sous agitation et durant 30 min, 16,36 mL (26,18 mmoles) de BuLi (1,6M/hexane). L'agitation est poursuivie à 0°C pendant 30 min supplémentaire, puis maintenue à température ambiante pendant 90 min. 2,69 mL (34,91 mmoles) de DMF, dilués dans 17 mL d'Et₂O anhydre sont ensuite introduits dans le mélange réactionnel. Après 3h d'agitation à température ambiante, le mélange réactionnel est hydrolysé à 0°C par ajouts successifs de 10 mL d'une solution d'HCl concentré et de 100 mL d'eau. Le produit est extrait avec 3 x 50 mL de CH₂Cl₂. Les phases organiques rassemblées sont lavées avec 100 mL d'eau, séchées sur Na₂SO₄, filtrées et évaporées sous pression réduite. Le brut réactionnel est purifié par chromatographie flash sur colonne de gel de silice, en éluant avec un gradient de 10 à 20% de CH₂Cl₂ dans l'heptane pour donner 1,82 g de 3-triméthylsilylbenzaldéhyde attendu sous la forme d'une huile jaune.
R.M.N. ¹H (DMSO D₆), δ (ppm) : 10,01 (s, 1H); 8,0 (s, 1H) ; 7,85 (d, 1H) ; 7,8 (d, 1H) ; 7,5 (dd, 1 H) 0,3 (s, 9H)

### 3.3 2-Azido-3-(3-triméthylsilylphényl)propénoate d'éthyle

A une solution de 2 g (87,5 mmoles) de sodium dans 30 mL d'EtOH anhydre, maintenue sous atmosphère d'azote et refroidie à -10°C, est ajouté, goutte à goutte, un mélange de 31,4 mL (87,5 mmoles) d'azidoacétate d'éthyle (à 34% dans CH₂Cl₂) et 3,9 g (21,87 mmoles) du 3-triméthylsilylbenzaldehyde préparé selon la procédure décrite à l'étape 3.2, dilué dans 3 mL d'EtOH. Le mélange réactionnel est agité à 0°C pendant 4 h. Il est ensuite hydrolysé par ajout sous agitation vigoureuse de 100 mL d'une solution aqueuse de NH₄Cl (30%). La phase aqueuse est extraite avec 3 x 50 mL d'AcOEt. Les phases organiques rassemblées sont lavées à l'eau, séchées sur Na₂SO₄ et concentrées sous pression réduite. Le brut réactionnel est purifié par chromatographie sur colonne de gel de silice, en éluant avec un mélange isocratique d'heptane et de CH₂Cl₂ (80/20). On isole ainsi 1,7 g du 2-azido-3-(3-triméthylsilylphényl)propénoate d'éthyle attendu, sous forme d'une huile jaune.
R.M.N. ¹H (DMSO D₆), δ (ppm) : 7,9 (d, 1H) ; 7,8 (s, 1H) ; 7,4 (d, 1H) ; 7,3 (dd, 1H) ; 6,9 (s, 1H) ; 4,2 (q, 2H) ; 1,2 (t, 3H) ; 0,15 (s, 9H)
MS: [MH]⁺ = 289

### 3.4 5-Triméthylsilyl-1H-indole-2-carboxylate d'éthyle

A une solution de 1,7 g (5,90 mmoles) du 2-azido-3-(3-triméthylsilylphényl)propénoate d'éthyle préparé selon la procédure décrite à l'étape 3.3, dans 25 mL de toluène sec, maintenue sous atmosphère inerte, est ajoutée 0,62 g (0,59 mmoles) du complexe dimère d'heptafluorobutyrate de dirhodium (II). Le mélange réactionnel est agité pendant 7 h à 40°C. Une deuxième portion de 0,62 g (0,59 mmoles) du complexe dimère d'heptafluorobutyrate de dirhodium (II) est ajoutée au mélange réactionnel en maintenant l'agitation et le chauffage à 40°C pendant 1 h supplémentaire. Après retour à température ambiante, le mélange réactionnel est filtré sur gel de silice en éluant avec du toluène. Le filtrat est ensuite concentré sous pression réduite. Le solide verdâtre obtenu est trituré plusieurs fois dans un minimum d'heptane, jusqu'à obtenir une poudre blanche. Celle-ci est séchée sous pression réduite pour donner 0,87 g du 5-triméthylsilyl-1*H*-indole-2-carboxyfate d'éthyle attendu, sous la forme d'une poudre blanche.
PF = 114-115°C
R.M.N. ¹H (DMSO D₆), δ (ppm) : 11,7 (s, 1H) ; 7,7 (s, 1H) ; 7,35 (d, 1H) ; 7,25 (d, 1H) ; 7,0 (s, 1H) ; 4,2 (q, 2H) ; 1,2 (t, 3H) ; 0,15 (s, 9H).
LC-MS: 260 ([M-H]-, Rt= 5,05 min.).

### 3.5 5-Triméthylsilyl-1-[(3-tluorophényl)méthyl]-1H-indole-2-carboxylate d'éthyle

A une suspension de 0,4 g (1,53 mmoles) de 5-triméthylsilyl-1*H*-indole-2-carboxylate d'éthyle, obtenu selon le protocole décrit à l'étape 3.4 et de 0,64 g (4,59 mmoles) de carbonate de potassium dans 15 mL d'acétonitrile sec, maintenue sous atmosphère inerte, est ajoutée, lentement à température ambiante, 0,32 g (1,68 mmoles) de bromure de 3-fluorobenzyle et 100 mg (0,31 mmole) de bromure de tétrabutylammonium. Le mélange réactionnel est agité à reflux pendant 2 h. Après ce temps, la suspension refroidie est filtrée sur fritté, rincée à l'acétonitrile. Le brut réactionnel obtenu après évaporation du filtrat sous pression réduite est purifié par chromatographie flash sur colonne de gel de silice, en éluant avec un gradient de 5 à 15 % d'éther dans l'heptane. On isole ainsi 0,5 g du 5-triméthylsilyl-1-[(3-fluorophényl)méthyl]-1*H*-indole-2-carboxylate d'éthyle attendu.
PF = 80-82°C
R.M.N. ¹H (CDCl₃), δ (ppm) : 7,9 (s, 1H) ; 7,5-7,1 (m, 4H) ; 6,9 (m, 2H) ; 6,75 (m, 1H) ; 5,8 (s, 2H) ; 4,3 (q, 2H) ; 1,35 (t, 3H) ; 0,3 (s, 9H).
LC-MS: 370 ([M+H]+, Rt= 6,22 min.).

### 3.6 Acide 5-triméthylsilyl-1-[(3-fluorophényl)méthyl]-1H-indole-2-carboxylique (Composé N° IV b)

Une solution de 0,6 g (1,62 mmole) de 5-triméthylsilyl-1-[(3-fluorophényl)méthyl]-1*H-*indole-2-carboxylate d'éthyle, obtenu à l'étape 3.5, dans 10 mL d'éthanol absolu et 2,44 mL de soude 1N, est agitée quatre heures à reflux. Après ce temps, le mélange réactionnel est concentré sous pression réduite puis repris dans 50 mL d'eau. Le milieu est acidifié à pH 1 par ajouts successifs d'acide chlorhydrique 1 N. Le précipité est recueilli par filtration, lavé à l'eau puis séché sous pression réduite. On isole ainsi 0,53 g du produit attendu, qui est utilisé tel quel dans l'étape suivante.
PF = 177-178°C
R.M.N. ¹H (DMSO D₆), δ (ppm): 13,1 (pic élargi, 1H) ; 7,9 (s, 1H) ; 7,4 (d, 1H) ; 7,4 (m, 3H) ; 7,1 (m, 1H) ; 6,85 (m, 2H) ; 5,9 (s, 2H) ; 0,3 (s, 9H).

### 3.7 N-[6-(azétidin-1-yl)pyridin-3-yl]-5-triméthylsilyl-1-[(3-fluorophényl)méthyl]-1H-indole-2-carboxamide (composé N°3)

A une solution, agitée à 20°C sous atmosphère inerte, de 0,16 g (0,47 mmole) de l'acide 5-triméthylsilyl-1-[(3-fluorophényl)méthyl]-1*H*-indole-2-carboxylique, préparé selon le protocole décrit à l'étape 3.6, dans 10 mL de DMF, sont ajoutés, 98 mg (0,51 mmole) de chlorhydrate de *N*-(3-diméthylaminopropyl)-*N'*-éthylcarbodiimide (EDC) et 69 mg (0,51 mmole) de 1-hydroxy-benzotriazole (HOBt). Le mélange réactionnel est agité à température ambiante pendant 15 minutes. 84 mg (0,56 mmole) de 3-amino-6-(azétidin-1-yl)pyridine, préparé selon le protocole décrit à l'étape 1.6 sont ensuite ajoutés au mélange réactionnel. Après 16 h d'agitation à 20°C, le mélange réactionnel est versé dans un mélange de 30 mL d'une solution aqueuse saturée en NaHCO₃ et de 30 mL d'AcOEt. La phase aqueuse est extraite par 20 mL d'AcOEt. Les phases organiques rassemblées sont lavées avec 3 x 30 mL d'eau, séchées sur Na₂SO₄, puis concentrées sous pression réduite. Le solide obtenu après évaporation du solvant sous pression réduite, est lavé à l'ébullition de l'éther isopropylique, filtré à chaud puis séché sous pression réduite. 75 mg du produit attendu sont ainsi isolés.
PF = 212-213°C
R.M.N. ¹H (DMSO D₆), δ (ppm) : 10,1 (s, 1H) ; 8,2 (s, 1H) ; 7,75 (s, 1H) ; 7,7 (d, 1H) ; 7,4 (d, 1H); 7,3 (s, 1H) ; 7,35 (d, 1H); 7,2 (q,1H) ; 6,9 (t, 1H) ; 6,8 (d, 1H) ; 6,75 (d, 1H) ; 6,25 (d, 1H); 5,8 (s, 2H) ; 3,8 (t, 4H) ; 2,2 (m, 2H) ; 0.15 (s, 9H)
LC-MS: 473 ([M+H]+, Rt= 1,28 min.).

### Exemple 4 (Composé N°4)

### N-[6-(3-hydroxyazétidin-1-yl)pyridin-3-yl]-5-triméthylsilyl-1-[(3-fluorophényl)méthyl]-1H-indole-2-carboxamide

A une solution, agitée à 20°C sous atmosphère inerte, de 0,2 g (0,59 mmole) de l'acide 5-triméthylsilyl-1-[(3-fluorophényl)méthyl]-1*H*-indole-2-carboxylique, préparé selon le protocole décrit à l'étape 3.6, dans 6 mL de DMF, sont ajoutés, 123 mg (0,64 mmole) de chlorhydrate de *N*-(3-diméthylaminopropyl)-*N'*-éthylcarbodiimide (EDAC) et 87 mg (0,64 mmole) de 1-hydroxy-benzotriazole (HOBT). Le mélange réactionnel est agité à température ambiante pendant 15 minutes. On ajoute ensuite au mélange réactionnel, 145 mg (0,88 mmole) de 3-amino-6-(3-hydroxyazétidin-1-yl)pyridine, préparé à l'étape 2.2. Après 16 heures d'agitation à 20°C, On verse le mélange réactionnel dans un mélange de 30 mL d'une solution aqueuse saturée en NaHCO₃ et de 30 mL d'acétate d'éthyle. On décante et on extrait la phase aqueuse par 20 mL d'acétate d'éthyle. Les phases organiques rassemblées sont lavées trois fois avec 30 mL d'eau, séchées sur sulfate de sodium puis concentrées sous pression réduite. Le produit obtenu est purifié par chromatographie sur colonne de silice en éluant avec un mélange de dichlorométhane et de méthanol. Le solide obtenu après évaporation du solvant sous pression réduite est recristallisé dans l'éther isopropylique. On isole ainsi 200 mg du produit attendu.
PF = 207 - 208 °C
R.M.N. ¹H (DMSO D₆), δ (ppm) : 10,1 (s, 1H) ; 8,2 (s, 1H) ; 7,72 (s, 1H) ; 7,7 (d, 1H) ; 7,4 (d, 1H); 7,25 (m, 2H) ; 7,2 (m, 1H) ; 6,9 (m, 1H) ; 6,8 (m, 2H) ; 6,3 (d, 1H) ; 5,8 (s, 2H) ; 5,5 (d, 1H) ; 4,4 (m, 1H) ; 4,0 (m, 2H) ; 3,5 (m, 2H) ; 0,2 (s, 9H)
LC-MS: 489 ([M+H]+, Rt= 1,25 min.).

### Exemple 5 (Composé N°5)

### N-[6-(azétidin-1-yl)pyridin-3-yl]-7-triméthylsilyl-1-[(3-fluorophényl)méthyl]-1H-indole-2-carboxamide

### 5.1 7-Triméthylsilyl-1H-indole-2-carboxylate d'éthyle

Dans un tricol surmonté d'un réfrigérant et d'une ampoule d'addition et maintenue sous atmosphère inerte est porté à reflux 70 mL de xylène. On ajoute ensuite goutte à goutte une solution de 0,7 g (2,42 mmoles) du 2-azido-3-(3-triméthylsilylphényl)propénoate d'éthyle préparé selon la procédure décrite à l'étape 3.3, dans 1 mL de xylène. Le mélange réactionnel est maintenu à reflux sous agitation pendant 1 h supplémentaire. Après retour à température ambiante, le brut réactionnel obtenu après évaporation du solvant sous pression réduite est purifié par chromatographie flash sur colonne de gel de silice, en éluant avec un gradient de 0 à 20 % d'éther éthylique dans l'heptane. On isole ainsi deux produits régioisomères : 170 mg du 5-triméthylsilyl-1*H*-indole-2-carboxylate d'éthyle, sous la forme d'une poudre blanche et 150 mg du 7-triméthylsilyl-1*H-*indole-2-carboxylate d'éthyle attendu, sous la forme d'une huile.
R.M.N. ¹H (DMSO D₆), δ (ppm) : 10,8 (s, 1H); 7,7 (d, 1H) ; 7,4 (d, 1H) ; 7,2 (s, 1H) ; 7,1 (m, 1H) ; 4,3 (q, 2H) ; 1,3 (t, 3H) ; 0,4 (s, 9H).
LC-MS: 260 ([M-H]-, *R*t= 5,48 min.).

### 5.2 7-Triméthylsilyl-1-[(3-fluorophényl)méthyl]-1H-indole-2-carboxylate d'éthyle

A une suspension de 0,75 g (2,87 mmoles) de 7-triméthylsilyl-1*H* indole-2-carboxylate d'éthyle, obtenu selon le protocole décrit à l'étape 5.1 et de 1,18 g (8,61 mmoles) de carbonate de potassium dans 50 mL d'acétonitrile sec, maintenue sous atmosphère inerte, est ajoutée, lentement à température ambiante, 0,42 mL (3,44 mmoles) de bromure de 3-fluorobenzyle. Le mélange réactionnel est agité à reflux pendant 18 h. Après ce temps, la suspension refroidie est filtrée sur fritté puis rincée à l'acétonitrile. Le brut réactionnel obtenu après évaporation du filtrat sous pression réduite est purifié par chromatographie flash sur colonne de gel de silice, en éluant avec un gradient de 10 à 50 % de dichlorométhane dans l'heptane. On isole ainsi 0,67 g du 7-triméthylsilyl-1-[(3-fluorophényl)méthyl]-1*H*-indole-2-carboxylate d'éthyle attendu.
R.M.N. ¹H (DMSO D₆), δ (ppm): 7,8 (d, 1H) ; 7,5 (d, 1H) ; 7,2 (m, 2H) ; 6,95 (m, 2H) ; 6,35 (m, 2H) ; 5,9 (s, 2H) ; 4,1 (q, 2H) ; 1,1 (t, 3H) ; 0,2 (s, 9H).
LC-MS: 370 ([M+H]+, Rt= 6,15 min.).

### 5.3 Acide 7-triméthylsilyl-1-[(3-fluorophényl)méthyl]-1H-indole-2-carboxylique (Composé N° IV c)

A une solution de 0,5 g (1,35 mmoles) de 7-triméthylsilyl-1-[(3-fluorophényl)méthyl]-1*H-*indole-2-carboxylate d'éthyle, obtenu selon le protocole décrit à l'étape 5.2, dans 30 mL d'un mélange (v/v/v) de méthanol, d'eau et de dioxane est ajoutée 0,23 g (4,06 mmoles) d'hydroxyde de potassium. Après trois heures d'agitation à reflux, le mélange réactionnel est concentré au tiers sous pression réduite puis repris dans 10 mL d'eau. Le milieu est acidifié à pH 1 par ajouts successifs d'acide chlorhydrique 1 N. Le précipité est recueilli par filtration, lavé à l'eau puis séché sous pression réduite. On isole ainsi 0,37 g du produit attendu, qui est utilisé tel quel dans l'étape suivante.
R.M.N. ¹H (DMSO D₆), δ (ppm): 12,6 (pic élargi, 1H) ; 7,6 (d, 1H) ; 7,3 (d, 1H) ; 7,2 (s, 1H) ; 7,0 (m, 2H) ; 6,75 (m, 1H) ; 6,2 (m, 1H) ; 6,1 (d, 1H) ; 5,8 (s, 2H) ; 0,05 (s, 9H).
LC-MS: 342 ([M+H]+, *R*t= 1,48 min.).

### 5.4 N-[6-(azétidin-1-yl)pyridin-3-yl]-7-triméthylsilyl-1-[(3-fluorophényl)méthyl]-1H-indole-2-carboxamide (composé N°5)

A une solution, agitée à 20°C sous atmosphère inerte, de 0,5 g (1,46 mmole) de l'acide 7-triméthylsilyl-1-[(3-fluorophényl)méthyl]-1*H*-indole-2-carboxylique, préparé selon le protocole décrit à l'étape 5.3, dans 10 mL de DMF, sont ajoutés, 0,28 g (1,46 mmole) de chlorhydrate de *N*-(3-diméthylaminopropyl)-*N'-*éthylcarbodiimide (EDC) et 0,2 g (1,46 mmole) de 1-hydroxy-benzotriazole (HOBt). Le mélange réactionnel est agité à température ambiante pendant 20 minutes. 0,33 g (2,20 mmoles) de 3-amino-6-(azétidin-1-yl)pyridine, préparé selon le protocole décrit à l'étape 1.6 sont ensuite ajoutés au mélange réactionnel. Après 18 h d'agitation à 20°C, le mélange réactionnel est versé dans un mélange de 30 mL d'une solution aqueuse saturée en NaHCO₃ et de 30 mL d'AcOEt. La phase aqueuse est extraite par 20 mL d'AcOEt. Les phases organiques rassemblées sont lavées avec 3 x 30 mL d'eau, séchées sur Na₂SO₄, puis concentrées sous pression réduite. Le solide obtenu après évaporation du solvant sous pression réduite, est purifié par chromatographie flash sur colonne de gel de silice, en éluant avec un gradient de 2 à 10 % de MeOH dans le CH₂Cl₂. Le produit isolé est ensuite précipité puis lavé à l'ébullition d'un mélange d'hexane et d'éther isopropylique, filtré à chaud puis séché sous pression réduite. 147 mg du produit attendu sont ainsi isolés.
PF = 160-162°C
R.M.N. ¹H (DMSO D₆), δ (ppm) : 9,9 (s, 1H) ; 8,0 (s, 1H) ; 7,7 (d, 1H) ; 7,5 (d, 1H) ; 7,3 (d, 1H) ; 7,2 (s, 1H); 7,0 (m, 2H) ; 6,8 (m, 1H) ; 6,25 (d, 1H); 6,15 (m, 2H) ; 5,8 (s, 2H) ; 3,7 (t, 4H) ; 2,1 (m, 2H) ; 0.15 (s, 9H)
LC-MS: 473 ([M+H]+, Rt= 1,25 min.)

### Exemple 6 (Composé N°6)

### N-[6-(3-hydroxyazétidin-1-yl)pyridin-3-yl]-7-triméthylsilyl-1-[(3-fluorophényl)méthyl]-1H-indole-2-carboxamide (Composé n°6)

Le composé N° 6 a été préparé selon un procédé similaire à celui décrit à l'étape 5.4 en faisant réagir 0,15 g (0,44 mmole) de l'acide 7-triméthylsilyl-1-[(3-fluorophényl)méthyo-1*H*-indole-2-carboxylique préparé selon le protocole décrit à l'étape 5.3 avec 109 mg (0,66 mmole) de 3-amino-6-(3-hydroxyazétidin-1-yl)pyridine, préparé selon le protocole décrit à l'étape 2.2 en présence de 87 mg (0,44 mmole) du chlorhydrate de *N-*(3-diméthylaminopropyl)-*N'*-éthylcarbodiimide (EDAC) et de 61 mg (0,44 mmole) de 1-hydroxy-benzotriazole (HOBT) dans 5 mL de diméthylformamide. On obtient 27 mg du produit attendu.
R.M.N. ¹H (DMSO D₆), δ (ppm) : 9,9 (s, 1H) ; 8,0 (s, 1H) ; 7,7 (d, 1H) ; 7,5 (d, 1H) ; 7,3 (d, 1H) ; 7,2 (s, 1H); 7,0 (m, 2H) ; 6,8 (m, 1H) ; 6,25 (d, 1H); 6,15 (m, 2H) ; 5,8 (s, 2H) ; 5,5 (d, 1H) ; 4,4 (m, 1H) ; 4,0 (m, 2H) ; 3,5 (m, 2H) ; 0,15 (s, 9H)
LC-MS: 489 ([M+H]+, *R*t= 1,3 min.).
PF = 215-217°C

### Exemple 7 (Composé N°7)

### N-[6-(Diméthylamino)pyridin-3-yl]-5-triméthylsilyl-1-[(3-fluorophényl)méthyl]-1H-indole-2-carboxamide

Le composé N° 7 a été préparé selon un procédé similaire à celui décrit à l'étape 5.4 en faisant réagir 0,3 g (0,88 mmole) de l'acide 5-triméthylsilyl-1-[(3-fluorophényl)méthyl]-1*H*-indole-2-carboxylique préparé selon le protocole décrit à l'étape 3.6 avec 181 mg (1,32 mmole) de 3-amino-6-(diméthylamino)pyridine en présence de 185 mg (0,97 mmole) du chlorhydrate de *N*-(3-diméthylaminopropyl)-*N'-*éthylcarbodiimide (EDAC) et de 131 mg (0,97 mmole) de 1-hydroxy-benzotriazole (HOBT) dans 10 mL de diméthylformamide. Le produit obtenu est purifié par chromatographie sur colonne de silice en éluant avec un mélange de dichlorométhane et d'acétone. Le solide obtenu après évaporation du solvant sous pression réduite est recristallisé dans l'alcool isopropylique. On isole ainsi 200 mg du produit attendu.
PF = 204 - 205 °C
R.M.N. ¹H (DMSO D₆), δ (ppm) : 10,2 (s, 1H) ; 8,4 (s, 1H) ; 7,9 (m, 2H) ; 7,55 (d, 1H) ; 7,4 (m, 2H) ; 7,3 (m, 1H) ; 7,0 (m, 1H) ; 6,9 (m, 2H) ; 6,65 (d, 1H) ; 5,9 (s, 2H) ; 3,0 (s, 6H) ; 0,25 (s, 9H).
LC-MS: 461 ([M-H]-, Rt= 1,3 min.).

### Exemple 8 (Composé N°8)

### N-[6-(Azétidin-1-yl)pyridin-3-yl]-5-(éthyl)diméthylsilyl-1-[(3-fluorophényl)méthyl]-1H-indole-2-carboxamide

### 8.1 5-lodo -1-[(3-fluorophényl)méthyl]-1H-indole-2-carboxylate d'éthyle

A une suspension de 0,26 g (6,63 mmole) d'hydrure de sodium (60%) dans 10 mL de DMF sec, maintenue sous atmosphère inerte, est ajoutée, goutte à goutte à 0°C, une solution de 2,0 g (6,03 mmoles) de 5-iodo-1*H-*indole-2-carboxylate d'éthyle (obtenu selon le protocole décrit dans la littérature , Synthetic Communications (2003), 33(14), 2423-2427) dans 10 mL de DMF sec. Le mélange réactionnel est agité à température ambiante pendant pendant 1 heure. On ajoute ensuite, goutte à goutte, à 0°C, 1,37 g (7,24 mmoles) de bromure de 3-fluorobenzyle. Le mélange réactionnel est alors agité pendant 18 h à température ambiante puis dilué avec 200 mL d'un mélange d'eau et d'acétate d'éthyle (1/1). On décante et on extrait la phase aqueuse avec 2x30 mL d'acétate d'éthyle. Les phases organiques rassemblées sont lavées trois fois à l'eau, séchées sur du sulfate de sodium, filtrées et concentrées sous pression réduite. On purifie l'huile résultante par chromatographie sur colonne de gel de silice en éluant avec un mélange d'heptane et d'acétate d'éthyle. On isole 1,87 g du produit attendu.
R.M.N. ¹H (DMSO D₆), δ (ppm) : 8,15 (s, 1H) ; 7,6 (d, 1H) ; 7,5 (d, 1H) ; 7,35 (s, 1H) ; 7,3 (m, 1H) ; 7,1 (m, 1H) ; 6,85 (m, 2H) ; 5,9 (s, 2H) ; 4,3 (q, 2H) ; 1,3 (t, 3H).
LC-MS: 424 ([M+H]+, *R*t= 1,65).

### 8.2 Acide 5-iodo-1-[(3-fluorophényl)méthyl]-1H-indole-2-carboxylique

Une solution de 1,00 g (2,36 mmoles) de 5-iodo-1-[(3-fluorophényl)méthyl]-1*H*-indole-2-carboxylate d'éthyle, obtenu à l'étape 8.1, et de 0,4 g (7,09 mmoles) d'hydroxyde de potassium, dans 30 mL d'un mélange (v/v/v) de méthanol, de dioxane et d'eau, est agitée trois heures à reflux. Après ce temps, le mélange réactionnel est concentré sous pression réduite puis repris dans 10 mL d'eau. Le milieu est acidifié à pH 1 par ajouts successifs d'acide chlorhydrique 1 N. Le précipité est recueilli par filtration, lavé à l'eau puis séché sous pression réduite. On isole ainsi 0,69 g du produit attendu, qui est utilisé tel quel dans l'étape suivante.
R.M.N. ¹H (DMSO D₆), δ (ppm): 13,2 (pic élargi, 1H) ; 8,1 (s, 1H) ; 7,5 (d, 1H) ; 7,4 (d, 1H) ; 7,25 (m, 2H) ; 7,0 (m, 1H) ; 6,8 (m, 2H) ; 5,8 (s, 2H).
LC-MS: 394 ([M-H]-, Rt= 3,5).

### 8.3 N-[6-(Azétidin-1-yl)pyridin-3-yl]-5-iodo-1-[(3-fluorophényl)méthyl]-1H-indole-2-carboxamide

A une solution, agitée à 20°C sous atmosphère inerte, de 0,6 g (1,52 mmole) de l'acide 5-iodo-1-[(3-fluorophényl)méthyl]-1*H*-indole-2-carboxylique, préparé à l'étape 8.2, dans 15 mL de DMF, sont ajoutés, 0,29 g (1,52 mmole) de chlorhydrate de *N*-(3-diméthylaminopropyl)-*N'*-éthylcarbodiimide (EDAC) et 0,2 g (1,52 mmole) de 1-hydroxy-benzotriazole (HOBT). Le mélange réactionnel est agité à température ambiante pendant 20 minutes. On ajoute ensuite au mélange réactionnel, 0,25g (1,67 mmole) de 3-amino-6-(azétidin-1-yl)pyridine, préparé selon le protocole décrit à l'étape 1.6. Après 18 heures d'agitation à 20°C, On verse le mélange réactionnel dans un mélange de 50 mL d'une d'eau et de 50 mL d'acétate d'éthyle. On décante et on extrait la phase aqueuse par 20 mL d'acétate d'éthyle. Les phases organiques rassemblées sont lavées trois fois avec 30 mL d'eau, séchées sur sulfate de sodium puis concentrées sous pression réduite. Le produit isolé est ensuite précipité puis lavé à l'ébullition de l'éther isopropylique, filtré à chaud puis séché sous pression réduite. 0,47 g du produit attendu sont ainsi isolés.
R.M.N. ¹H (DMSO D₆), δ (ppm) : 10,3 (s, 1H) ; 8,35 (s, 1H) ; 8,2 (s, 1H) ; 7,85 (d, 1H) ; 7,55 (d, 1H) ; 7,45 (d, 1H) ; 7,3 (m, 2H) ; 7,05 (m, 1H) ; 6,9 (m, 2H) ; 6,4 (d, 1H) ; 5,9 (s, 2H) ; 3,9 (t, 4H) ; 2,3 (quint., 2H).
LC-MS: 527 ([M+H]+, Rt= 5,07).

### 8.4 N-[6-(Azétidin-1-yl)pyridin-3-yl]-5-(éthyl)diméthylsilyl-1-[(3-fluorophényl)méthyl]-1H-indole-2-carboxamide (composé N°8)

A une suspension de 0,15 g (0,28 mmole) de *N*-[6-(azétidin-1-yl)pyridin-3-yl]-5-iodo-1-[(3-fluorophényl)méthyl]-1*H*-indole-2-carboxamide préparé selon le protocole décrit à l'étape 8.3 et de 0,18 g (0,85 mmole) de triphosphate de potassium dans 2 mL de 1-méthyl-2-pyrrolidinone (NMP) sec, maintenue sous atmosphère inerte, sont ajoutées, 27 mg (0,31 mmole) de (éthyl)diméthylsilane et 15 mg (0,03 mmole) de bis(tri-*tert*butylphosphine)palladium. Le mélange réactionnel est alors agité à température ambiante sous atmosphère inerte pendant 6 h puis dilué avec 40 mL d'un mélange d'eau et d'acétate d'éthyle (1/1). On décante et on extrait la phase aqueuse avec 2x10 mL d'acétate d'éthyle. Les phases organiques rassemblées sont séchées sur du sulfate de sodium, filtrées et concentrées sous pression réduite. On purifie l'huile résultante par chromatographie sur colonne de gel de silice en éluant avec un mélange d'heptane et d'acétate d'éthyle. On isole 108 mg du produit attendu.
PF = 188-189 °C
R.M.N. ¹H (DMSO D₆), δ (ppm) : 10,25 (s, 1H) ; 8,4 (s, 1H) ; 7,85 (m, 2H) ; 7,6 (d, 1H) ; 7,35 (m, 3H) ; 7,1 (m, 1H) ; 6,9 (m, 2H) ; 6,4 (d, 1H) ; 5,9 (s, 2H) ; 3,9 (t, 4H) ; 2,3 (quint., 2H) ; 0,95 (t, 3H) ; 0,75 (q, 2H) ; 0,3 (s, 6H).
LC-MS: 487 ([M+H]+, Rt= 1,37).

### Exemple 9 (Composé N°9)

### N-[6-(azétidin-1-yl)pyridin-3-yl]-5-(cyclohexyl)diméthylsilyl-1-[(3-fluorophényl)méthyl]-1H-indole-2-carboxamide (composé N°9)

Le composé N° 9 a été préparé selon un procédé similaire à celui décrit à l'étape 8.4 en faisant réagir 0,15 g (0,28 mmole) de *N*-[6-(azétidin-1-yl)pyridin-3-yl]-5-iodo-1-[(3-fluorophényl)méthyl]-1*H*-indole-2-carboxamide préparé selon le protocole décrit à l'étape 8.3 avec 44 mg (0,31 mmole) de (cyclohexyl)diméthylsilane en présence de 0,18 g (0,85 mmole) de triphosphate de potassium et de 15 mg (0,03 mmole) de bis(tri-*tert*butylphosphine)palladium dans 2 mL de 1-méthyl-2-pyrrolidinone (NMP) sec. On obtient 93 mg du produit attendu sous la forme d'un solide blanc.
PF = 202 - 203 °C
R.M.N. ¹H (DMSO D₆), δ (ppm) : 10,25 (s, 1H) ; 8,4 (s, 1H) ; 7,8 (m, 2H) ; 7,5 (d, 1H) ; 7,35 (m, 3H) ; 7,0 (m, 1H) ; 6,9 (m, 2H) ; 6,4 (d, 1H) ; 5,9 (s, 2H) ; 3,9 (t, 4H) ; 2,3 (quint., 2H) ; 1,65 (m, 5H) ; 1,3-0,7 (m, 6H) ; 0,25 (s, 6H).
LC-MS: 541 ([M+H]+, *R*t= 1,56).

### Exemple 10 (Composé N°10)

### N-[6-(azétidin-1-yl)pyridin-3-yl]-6-triméthylsilyl-1-[[(3-trifluorométhyl)phényl]méthyl]-1H-indole-2-carboxamide

### 10.1 6-Triméthylsilyl-1-[[(3-trifluorométhyl)phényl]méthyl]-1H-indole-2-carboxylate d'éthyle

A une solution de 0,5 g (1,91 mmole) de 6-triméthylsilyl-1*H*-indole-2-carboxylate d'éthyle, obtenu selon le protocole décrit à l'étape 1.2 dans 8 mL de toluène sec, maintenue sous atmosphère inerte, est ajoutée, à température ambiante, 0,52 mL (3,83 mmoles) de 3-(trifluorométhyl)phénylméthanol et 0,92 g (3,83 mmoles) de (cyanométhylene)tributylphosphorane (CMBP). Le mélange réactionnel est agité à 110°C pendant 15 heures puis concentré à sec. Le brut réactionnel est ensuite purifié par chromatographies flash successives sur colonne de gel de silice pour donner 0,72 g de 6-triméthylsilyl-1-[[(3-trifluorométhyl)phényl]méthyl]-1*H*-indole-2-carboxylate d'éthyle attendu sous la forme d'une huile.
R.M.N. ¹H (DMSO D₆), δ (ppm) : 7,49-7,45 (m, 2H) ; 7,36-7,33 (m, 2H) ; 7,25 (t, 1H) ; 7,12 (s, 1H) ; 7,04-7,00 (m, 2H) ; 5,73 (s, 2H) ; 4,04 (q, 2H) ; 1,03 (t, 3H) ; 0,00 (s, 9H).
LC-MS: 420 ([M+H]+, *R*t= 3,57).

### 10.2 Acide 6-triméthylsilyl-1-[[(3-trifluorométhyl)phényl]méthyl]-1H-indole-2-carboxylique (Composé N° IVd)

A une solution de 0,31 g (0,73 mmole) de 6-triméthylsilyl-1-[[(3-trifluorométhyl)phényl]méthyl]-1*H*-indole-2-carboxylate d'éthyle, obtenu à l'étape 10.1, dans 10 mL d'éthanol est ajoutée une solution de 0,125 g (2,18 mmole) d'hydroxyde de potassium dans 1 mL d'eau. Après deux heures d'agitation à 85°C, le mélange réactionnel est concentré sous pression réduite puis repris dans 10 mL d'eau. Le milieu est acidifié à pH 5 par ajouts successifs d'acide chlorhydrique 1 N. Le précipité est recueilli par filtration, lavé à l'eau puis séché sous pression réduite. On isole ainsi 0,24 g du produit attendu, qui est utilisé tel quel dans l'étape suivante.
R.M.N. ¹H (DMSO D₆), δ (ppm) : 13,2 (pic élargi, 1H) ; 7,7 (m, 2H) ; 7,6 (m, 2H) ; 7,5 (m, 1H) ; 7,3 (m, 3H) ; 6,0 (s, 2H) ; 1,2 (s, 9H).

### 10.3 N-[6-(Azétidin-1-yl)pyridin-3-yl]-6-triméthylsilyl-1-[[(3-trifluorométhyl)phényl]méthyl]-1H-indole-2-carboxamide (composé N°10)

Le composé N° 10 a été préparé selon un procédé similaire à celui décrit à l'étape 8.3 en faisant réagir 0,11 g (0,28 mmole) de l'acide 6-triméthylsilyl-1-[[(3-trifluorométhyl)phényllméthyl]-1*H*-indole-2-carboxylique préparé selon le protocole décrit à l'étape 10.2 avec 46 mg (0,31 mmole) de 3-amino-6-(azétidin-1-yl)pyridine, préparé selon le protocole décrit à l'étape 1.6 en présence de 59 mg (0,31 mmole) du chlorhydrate de *N*-(3-diméthylaminopropyl)-*N'*-éthylcarbodiimide (EDAC) et 45 mg (0,31 mmole) de 1-hydroxy-benzotriazole (HOBT) dans 2 mL de diméthylformamide. On obtient 200 mg du produit attendu.
PF = 167 -168 °C
R.M.N. ¹H (DMSO D₆), δ (ppm) : 10,25 (s, 1H) ; 8,35 (d, 1H) ; 7,85 (dd, 1H) ; 7,7 (m, 2H) ; 7,65 (s, 1H) ; 7,6 (m, 1H) ; 7,5 (t, 1H) ; 7,45 (m, 1H) ; 7,35 (s, 1H) ; 7,25 (d, 1H) ; 6,4 (d, 1H) ; 6,0 (s, 2H) ; 3,95 (t, 4H) ; 2,3 (m, 2H) ; 0,2 (s, 9H).
LC-MS: 523 ([M+H]+, Rt= 1,42 min.).

### Exemple 11 (Composé N°11)

### N-[6-(3-hydroxyazétidin-1-yl)pyridin-3-yl]-6-triméthylsilyl-1-[[(3-trifluorométhyl)phényl]méthyl]-1H-indole-2-carboxamide (Composé n°11)

Le composé N° 11 a été préparé selon un procédé similaire à celui décrit à l'étape 8.3 en faisant réagir 0,11 g (0,28 mmole) de l'acide 6-triméthylsilyl-l-[[(3-trifluorométhyl)phényl]méthyl]-1*H*-indole-2-carboxylique préparé selon le protocole décrit à l'étape 10.2 avec 51 mg (0,31 mmole) de 3-amino-6-(3-hydroxyazétidin-1-yl)pyridine, préparé selon le protocole décrit à l'étape 2.2 en présence de 59 mg (0,31 mmole) du chlorhydrate de *N*-(3-diméthylaminopropyl)-*N'*-éthylcarbodiimide (EDAC) et 45 mg (0,31 mmole) de 1-hydroxy-benzotriazole (HOBT) dans 2 mL de diméthylformamide. On obtient 0,21 g du produit attendu.
PF = 183 - 184 °C
R.M.N. ¹H (DMSO D₆), δ (ppm) : 10,3 (s, 1H) ; 8,4 (d, 1H) ; 7,85 (m, 1H) ; 7,7 (m, 2H) ; 7,65 (s, 1H) ; 7,6 (m, 1H) ; 7,5 (t, 1H) ; 7,45 (m, 1H) ; 7,35 (s, 1H) ; 7,25 (d, 1H) ; 6,4 (d, 1H) ; 6,0 (s, 2H) ; 5,6 (d, 1H) ; 4,6 (m, 1H) ; 4,15 (m, 2H) ; 3,65 (m, 2H) ; 0,25 (s, 9H).
LC-MS: 539 ([M+H]+, Rt= 1,37min.).

### Exemple 12 (Composé N°12)

### N-[6-(Azétidin-1-yl)pyridin-3-yl]-5-(diphényl)méthylsilyl-1-[(3-fluorophényl)méthyl]-1H-indole-2-carboxamide

Le composé N° 12 a été préparé selon un procédé similaire à celui décrit à l'étape 8.4 en faisant réagir 0,15 g (0,28 mmole) de *N*-[6-(azétidin-1-yl)pyridin-3-yl]-5-iodo-1-[(3-fluorophényl)méthyl]-1*H*-indole-2-carboxamide préparé selon le protocole décrit à l'étape 8.3 avec 167 mg (0,84 mmole) de diphénylméthylsilane en présence de 0,18 g (0,84 mmole) de triphosphate de potassium et de 15 mg (0,03 mmole) de bis(tri-*tert*butylphosphine)palladium dans 2 mL de 1-méthyl-2-pyrrolidinone (NMP) sec. On obtient 19 mg du produit attendu.
PF = 98-100°C
R.M.N. ¹H (DMSO D₆), δ (ppm) : 10,25 (s, 1H) ; 8,4 (s, 1H) ; 7,85 (m, 2H) ; 7,65-7,25 (m, 14H) ; 7,05 (m, 1H) ; 6,95 (m, 2H) ; 6,4 (d, 1H) ; 5,9 (s, 2H) ; 3,95 (m, 4H) ; 2,3 (m, 2H) ; 0,9 (s, 3H).
LC-MS: 597 ([M+H]+, Rt= 1,51).

### Exemple 13 (Composé N°13)

### N-[6-(Azétidin-1-yl)pyridin-3-yl]-5-triméthylsilyl-1-[[(3-trifluorométhyl)phényl]méthyl]-1H-indole-2-carboxamide (composé N°13)

### 13.1 5-Triméthylsilyl-1-[[(3-trifiuorométhyl)phényl]méthyn-1H-indole-2-carboxylate d'éthyle

A une solution de 0,49 g (1,87 mmole) de 5-triméthylsilyl-1*H*-indole-2-carboxylate d'éthyle, obtenu selon le protocole décrit à l'étape 3.4 dans 8 mL de toluène sec, maintenue sous atmosphère inerte, est ajoutée, à température ambiante, 0,51 mL (3,749 mmoles) de 3-(trifluorométhyl)phénylméthanol et 0,9 g (3,749 mmoles) de (cyanométhylene)tributylphosphorane (CMBP). Le mélange réactionnel est agité à 110°C pendant 15 heures puis concentré à sec. Le brut réactionnel est ensuite purifié par chromatographie flash sur colonne de gel de silice, en éluant un mélange d'hexane et d'acétate d'éthyle pour donner 0,73 g du 5-triméthylsilyl-1-[[(3-trifluorométhyl)phényl]méthyl]-1*H*-indole-2-carboxylate d'éthyle attendu.
R.M.N. ¹H (DMSO D₆), δ (ppm) : 7,90 (s, 1H) ; 7,62-7,57 (m, 2H) ; 7,51-7,43 (m, 3H) ; 7,40 (s, 1H) ; 7,17 (d, 1H) ; 5,92 (s, 2H) ; 4,28 (q, 2H) ; 1,26 (t, 3H) ; 0,27 (s, 9H).
LC-MS: 420 ([M+H]+, *R*t= 3,62).

### 13.2 Acide 5-triméthylsilyl-1-[[(3-trifluorométhyl)phényl]méthyl]-1H-indole-2-carboxylique (Composé N° IVe)

A une solution de 0,31 g (0,73 mmole) de 5-triméthylsilyl-1-[[(3-trifluorométhyl)phényl]méthyl]-1*H*-indole-2-carboxylate d'éthyle, obtenu à l'étape 13.1, dans 10 mL d'éthanol est ajoutée une solution de 0,125 g (2,18 mmole) d'hydroxyde de potassium dans 1 mL d'eau. Après deux heures d'agitation à 85°C, le mélange réactionnel est concentré sous pression réduite puis repris dans 10 mL d'eau. Le milieu est acidifié à pH 5 par ajouts successifs d'acide chlorhydrique 1 N. Le précipité est recueilli par filtration, lavé à l'eau puis séché sous pression réduite. On isole ainsi 0,22 g du produit attendu, qui est utilisé tel quel dans l'étape suivante.
R.M.N. ¹H (DMSO D₆), δ (ppm) : 12,9 (pic élargi, 1H) ; 7,8 (s, 1H) ; 7,5 (m, 2H) ; 7,4 (m, 2H) ; 7,3 (d, 1H) ; 7,2 (s, 1H) ; 7,1 (d, 1H) ; 5,85 (s, 2H) ; 1,2 (s, 9H).

### 13.3 N-[6-(azétidin-1-yl)pyridin-3-yl]-5-triméthylsilyl-1-[[(3-trifluorométhyl)phényl]méthyl]-1H-indole-2-carboxamide (composé N°13)

Le composé N° 13 a été préparé selon un procédé similaire à celui décrit à l'étape 8.3 en faisant réagir 0,105 g (0,27 mmole) de l'acide 5-triméthylsilyl-1-[[(3-trifluorométhyl)phényl]méthyl]-1*H*-indole-2-carboxylique préparé selon le protocole décrit à l'étape 13.2 avec 44 mg (0,30 mmole) de 3-amino-6-(azétidin-1-yl)pyridine, préparé selon le protocole décrit à l'étape 1.6 en présence de 57 mg (0,30 mmole) du chlorhydrate de *N*-(3-diméthylaminopropyl)-*N'*-éthylcarbodiimide (EDAC) et 43 mg (0,30 mmole) de 1-hydroxy-benzotriazole (HOBT) dans 6 mL de diméthylformamide. On obtient 95 mg du produit attendu.
PF = 199-200°C
R.M.N. ¹H (DMSO D₆), δ (ppm) : 10,25 (s, 1H) ; 8,3 (s, 1H) ; 7,9 (s, 1H) ; 7,8 (d, 1H) ; 7,55 (m, 3H) ; 7,5 (m, 1H) ; 7,4 (m, 2H) ; 7,3 (d, 1H) ; 6,3 (d, 1H) ; 5,9 (s, 2H) ; 3,9 (t, 4H) ; 2,3 (quint., 2H) ; 0,25 (s, 9H).
LC-MS: 523 ([M+H]+, Rt= 1,50 min).

### Exemple 14 (Composé N°14)

### N-[6-(3-hydroxyazétidin-1-yl)pyridin-3-yl]-5-triméthylsilyl-1-[[(3-trifluorométhyl)phényl]méthyl]-1H-indole-2-carboxamide (Composé n°14)

Le composé N° 14 a été préparé selon un procédé similaire à celui décrit à l'étape 8.3 en faisant réagir 0,1 g (0,26 mmole) de l'acide 5-triméthylsilyl-1-[[(3-trifluorométhyl)phényl]méthyl]-1*H*-indole-2-carboxylique préparé selon le protocole décrit à l'étape 13.2 avec 46 mg (0,28 mmole) de 3-amino-6-(3-hydroxyazétidin-1-yl)pyridine, préparé selon le protocole décrit à l'étape 2.2 en présence de 54 mg (0,28 mmole) du chlorhydrate de *N*-(3-diméthylaminopropyl)-*N'*-éthylcarbodiimide (EDAC) et 41 mg (0,28 mmole) de 1-hydroxy-benzotriazole (HOBT) dans 6 mL de diméthylformamide. On obtient 100 mg du produit attendu.
PF = 217- 218 °C
R.M.N. ¹H (DMSO D₆), δ (ppm) : 10,25 (s, 1H) ; 8,2 (d, 1H) ; 7,75 (s, 1H) ; 7,7 (dd, 1H) ; 7,45-7,35 (m, 4H) ; 7,5 (m, 1H) ; 7,3 (m, 2H) ; 7,2 (m, 1H) ; 6,3 (d, 1H) ; 5,85 (s, 2H) ; 5,45 (d, 1H) ; 4,4 (m, 1H) ; 4,0 (m, 2H) ; 3,55 (m, 2H) ; 0,0 (s, 9H).
LC-MS: 539 ([M+H]+, Rt= 1,40 min.).

### Exemple 15 (Composé N°15)

### N-[6-(3-Hydroxyazétidin-1-yl)pyridin-3-yl]-6-triméthylsilyl-1-[(pyridin-4-yl)méthyl]-1H-indole-2-carboxamide (Composé n°15)

### 15.1. 6-Triméthylsilyl-1-[(4-pyridinyll)méthyl)]-1H-indole-2-carboxylate d'éthyle

A une solution de 0,5 g (1,91 mmole) de 6-triméthylsilyl-1*H*-indole-2-carboxylate d'éthyle, obtenu selon le protocole décrit à l'étape 1.2 dans 8 mL de toluène sec, maintenue sous atmosphère inerte, est ajoutée, à température ambiante, 0,42 g (3,82 mmoles) de 4-pyridinylméthanol et 0,92 g (3,826 mmoles) de (cyanométhylene)tributylphosphorane (CMBP). Le mélange réactionnel est agité à 110°C pendant 15 heures puis concentré à sec. Le brut réactionnel est ensuite purifié par chromatographie flash sur colonne de gel de silice, en éluant avec mélange d'heptane et d'acétate d'éthyle pour donner 0,644 g du 6-triméthylsilyl-1-[(pyridin-4-yl)méthyl]-1*H*-indole-2-carboxylate d'éthyle attendu sous la forme d'un solide jaune.
R.M.N. ¹H (DMSO D₆), δ (ppm) : 8,2 (d, 2H) ; 7,49 (d, 1H) ; 7,42 (s, 1H) ; 7,15 (s, 1H) ; 7,05 (d, 1H); 6,70 (d, 2H) ; 5,68 (s, 2H) ; 4,01 (q, 2H) ; 1,01 (t, 3H) ; 0,00 (s, 9H).
LC-MS: 353 ([M+H]+, *R*t= 2.63).

### 15.2 Acide 6-triméthylsilyl-1-[(4-pyridinyl)méthyl)]-1H indole-2-carboxylique (Composé N° IV f)

A une solution de 0,22 g (0,62 mmole) de 6-triméthylsilyl-1-[(4-pyridinyll)méthyl)]-1*H-*indole-2-carboxylate d'éthyle, obtenu à l'étape 15.1, dans 10 mL d'éthanol est ajoutée une solution de 0,11 g (1,87 mmole) d'hydroxyde de potassium dans 1 mL d'eau. Après deux heures d'agitation à 85°C, le mélange réactionnel est concentré sous pression réduite puis repris dans 10 mL d'eau. Le milieu est acidifié à pH 5 par ajouts successifs d'acide chlorhydrique 1 N. Le précipité est recueilli par filtration, lavé à l'eau puis séché sous pression réduite. On isole ainsi 0,19 g du produit attendu, qui est utilisé tel quel dans l'étape suivante.
R.M.N. ¹H (DMSO D₆), δ (ppm) : 8,2 (d, 2H) ; 7,4 (d, 1H) ; 7,3 (s, 1H); 7,0 (d, 1H) ; 6,9 (s, 1H) ; 6,8 (d, 2H) ; 5,8 (s, 2H) ; 0,95 (s, 9H).

### 15.3 N-[6-(3-hydroxyazétidin-1-yl)pyridin-3-yl]-6-triméthylsilyl-1-[(pyridin-4-yl)méthyl]-1H-indole-2-carboxamide (Composé n°15)

Le composé N° 15 a été préparé selon un procédé similaire à celui décrit à l'étape 8.3 en faisant réagir 94 mg (0,29 mmole) de l'acide 6-triméthylsilyl-1-[(4-pyridinyl)méthyl)]-1*H-*indole-2-carboxylique préparé selon le protocole décrit à l'étape 15.2 avec 53 mg (0,32 mmole) de 3-amino-6-(3-hydroxyazétidin-1-yl)pyridine, préparé selon le protocole décrit à l'étape 2.2 en présence de 61 mg (0,32 mmole) du chlorhydrate de *N*-(3-diméthylaminopropyl)-*N'*-éthylcarbodiimide (EDAC) et 46 mg (0,32 mmole) de 1-hydroxy-benzotriazole (HOBT) dans 2 mL de diméthylformamide. On obtient 80 mg du produit attendu.
R.M.N. ¹H (DMSO D₆), δ (ppm) : 10,2 (s, 1H) ; 8,45 (d, 2H) ; 8,35 (s, 1H) ; 7,85 (d, 1H) ; 7,75 (d, 1H) ; 7,6 (s, 1H) ; 7,45 (s, 1H) ; 7,3 (d, 1H) ; 7,0 (d, 2H) ; 6,4 (d, 1H) ; 5,95 (s, 2H) ; 5,6 (d, 1H) ; 4,6 (m, 1H) ; 4,15 (m, 2H) ; 3,65 (m, 2H) ; 0,25 (s, 9H).
LC-MS: 472 ([M+H]+, *R*t= 0.91 min.).

### Exemple 16 (Composé N°16)

### N-[6-(azétidin-1-yl)pyridin-3-yl]-6-triméthylsilyl-1-[(pyridin-4-yl)méthyl]-1H-indole-2-carboxamide

Le composé N° 16 a été préparé selon un procédé similaire à celui décrit à l'étape 8.3 en faisant réagir 95 mg (0,29 mmole) de l'acide 6-triméthylsilyl-1-[(4-pyridinyl)méthyl)]-1*H*-indole-2-carboxylique préparé selon le protocole décrit à l'étape 15.2 avec 48 mg (0,32 mmole) de 3-amino-6-(azétidin-1-yl)pyridine, préparé selon le protocole décrit à l'étape 1.6 en présence de 62 mg (0,32 mmole) du chlorhydrate de *N*-(3-diméthylaminopropyl)-*N'*-éthylcarbodiimide (EDAC) et 47 mg (0,32 mmole) de 1-hydroxy-benzotriazole (HOBT) dans 2 mL de diméthylformamide. On obtient 95 mg du produit attendu.
R.M.N. ¹H (DMSO D₆), δ (ppm) : 10,25 (s, 1H) ; 8,5 (d, 2H) ; 8,35 (s, 1H) ; 7,85 (d, 1H) ; 7,75 (d, 1H) ; 7,6 (s, 1H) ; 7,45 (s, 1H) ; 7,3 (d, 1H) ; 7,0 (d, 2H) ; 6,4 (d, 1H) ; 5,95 (s, 2H) ; 3,9 (t, 4H) ; 2,3 (quint., 2H) ; 0,25 (s, 9H).
LC-MS: 456 ([M+H]+, Rt= 0,95 min.).

### Exemple 17 (Composé N°17)

### N-[6-(Azétidin-1-yl)pyridin-3-yl]-5-triéthylsilyl-1-[(3-fluorophényl)méthyl]-1H-indole-2-carboxamide

### 17.1 5-Bromo-1-[(3-fluorophényl)méthyl]-1H-indole-2-carboxylate d'éthyle

A une suspension de 0,96 g (24,24 mmole) d'hydrure de sodium (60%) dans 15 mL de DMF sec, maintenue sous atmosphère inerte, est ajoutée, goutte à goutte à 0°C, une solution de 5,0 g (18,65 mmoles) de 5-bromo-1*H-*indole-2-carboxylate d'éthyle dans 25 mL de DMF sec. Le mélange réactionnel est agité à température ambiante pendant pendant 1 heure. On ajoute ensuite, goutte à goutte, à 0°C, 4,23 g (2,75 mmoles) de bromure de 3-fluorobenzyle. Le mélange réactionnel est alors agité pendant 18 h à température ambiante puis dilué avec 200 mL d'un mélange d'eau et d'acétate d'éthyle (1/1). On décante et on extrait la phase aqueuse avec 2x30 mL d'acétate d'éthyle. Les phases organiques rassemblées sont lavées trois fois à l'eau, séchées sur du sulfate de sodium, filtrées et concentrées sous pression réduite. On purifie l'huile résultante par chromatographie sur colonne de gel de silice en éluant avec un mélange d'heptane et d'acétate d'éthyle. On isole 5,76 g du produit attendu.
R.M.N. ¹H (DMSO D₆), δ (ppm) : 8,0 (s, 1H) ; 7,6 (d, 1H) ; 7,45 (d, 1H) ; 7,35 (s, 1H) ; 7,3 (m, 1H) ; 7,05 (m, 1H) ; 6,85 (m, 2H) ; 5,9 (s, 2H) ; 4,3 (q, 2H) ; 1,3 (t, 3H).
LC-MS: 376 ([M+H]+, *R*t= 11,8).

### 17.2 Acide 5-bromo-1-[(3-fluorophényl)méthyl]-1H-indole-2-carboxylique

Une solution de 1,5 g (3,99 mmoles) de 5-bromo-1-[(3-fluorophényl)méthyl]-1*H*-indole-2-carboxylate d'éthyle, obtenu à l'étape 17.1, et de 0,67 g (11,96 mmoles) d'hydroxyde de potassium, dans 45 mL d'un mélange (v/v/v) de méthanol, de dioxane et d'eau, est agitée trois heures à reflux. Après ce temps, le mélange réactionnel est concentré sous pression réduite puis repris dans 10 mL d'eau. Le milieu est acidifié à pH 1 par ajouts successifs d'acide chlorhydrique 1 N. Le précipité est recueilli par filtration, lavé à l'eau puis séché sous pression réduite. On isole ainsi 1,17 g du produit attendu, qui est utilisé tel quel dans l'étape suivante.
LC-MS: 346 ([M-H]-, Rt= 3,37 min.).

### 17.3 N-[6-(Azétidin-1-yl)pyridin-3-yl]-5-bromo-1-[(3-fluorophényl)méthyl]-1H-indole-2-carboxamide

A une solution, agitée à 20°C sous atmosphère inerte, de 1,4 g (4,02 mmole) de l'acide 5-bromo-1-[(3-fluorophényl)méthyl]-1*H*-indole-2-carboxylique, préparé à l'étape 17.2, dans 20 mL de DMF, sont ajoutés, 0,77 g (4,02 mmole) de chlorhydrate de N-(3-diméthylaminopropyl)-*N*'-éthylcarbodiimide (EDAC) et 0,54 g (4,02 mmole) de 1-hydroxy-benzotriazole (HOBT). Le mélange réactionnel est agité à température ambiante pendant 20 minutes. On ajoute ensuite au mélange réactionnel, 0,66 g (4,42 mmole) de 3-amino-6-(azétidin-1-yl)pyridine, préparé selon le protocole décrit à l'étape 1.6. Après 18 heures d'agitation à 20°C, On verse le mélange réactionnel dans un mélange de 50 mL d'une d'eau et de 50 mL d'acétate d'éthyle. On décante et on extrait la phase aqueuse par 20 mL d'acétate d'éthyle. Les phases organiques rassemblées sont lavées trois fois avec 30 mL d'eau, séchées sur sulfate de sodium puis concentrées sous pression réduite. Le produit isolé est ensuite précipité puis lavé à l'ébullition de l'éther isopropylique, filtré à chaud puis séché sous pression réduite. 11,46 g du produit attendu sont ainsi isolés.
R.M.N. ¹H (DMSO D₆), δ (ppm) : 10,4 (s, 1H) ; 8,38 (d, 1H) ; 8,0 (d, 1H) ; 7,85 (dd, 1H) ; 7,6 (d, 1H) ; 7,45-7,25 (m, 3H) ; 7,05 (dt, 1H) ; 6,9 (m, 2H) ; 6,4 (d, 1H) ; 5,9 (s, 2H) ; 3,9 (t, 4H) ; 2,3 (quint., 2H).
LC-MS: 479 ([M+H]+, Rt= 1,39 min.).

### 17.4 N-[6-(Azétidin-1-yl)pyridin-3-yl]-5-triéthylsilyl-1-[(3-fluorophényl)méthyl]-1H-indole-2-carboxamide (composé N°17)

Le composé N° 17 a été préparé selon un procédé similaire à celui décrit à l'étape 8.4 en faisant réagir 0,15 g (0,31 mmole) de *N*-[6-(azétidin-1-yl)pyridin-3-yl]-5-bromo-1-[(3-fluorophényl)méthyl]-1*H*-indole-2-carboxamide préparé selon le protocole décrit à l'étape 17.3 avec 110 mg (0,93 mmole) de triéthylsilane en présence de 0,2 g (0,94 mmole) de triphosphate de potassium et de 16 mg (0,03 mmole) de bis(tri-tertbutylphosphine)palladium dans 2 mL de 1-méthyl-2-pyrrolidinone (NMP) sec. On obtient 26 mg du produit attendu.
PF = 166 - 168 °C
R.M,N. ¹H (DMSO D₆), δ (ppm) : 10,2 (s, 1H) ; 8,4 (d, 1H) ; 7,85 (m, 2H) ; 7,55 (d, 1H) ; 7,35 (m, 3H) ; 7,05 (m, 1H) ; 6,95 (m, 2H) ; 6,4 (d, 1H) ; 5,9 (s, 2H) ; 3,9 (t, 4H) ; 2,3 (quint., 2H) ; 1,65 (m, 5H) ; 0,95 (m, 9H) ; 0,8(m, 6H).
LC-MS: 515 ([M+H]+, Rt= 1,55).

### Exemple 18 (Composé N°18)

### N-[6-(Azétidin-1-yl)pyridin-3-yl]-5-tert-butyl(diméthyl)silyl-1-[(3-fluorophényl)méthyl]-1H-indole-2-carboxamide

Le composé N° 18 a été préparé selon un procédé similaire à celui décrit à l'étape 8.4 en faisant réagir 0,15 g (0,31 mmole) de *N*-[6-(azétidin-1-yl)pyridin-3-yl]-5-bromo-1-[(3-fluorophényl)méthyl]-1*H-*indole-2-carboxamide préparé selon le protocole décrit à l'étape 17.3 avec 108 mg (0,93 mmole) de tert-butyldimethylsilane en présence de 0,2 g (0,94 mmole) de triphosphate de potassium et de 16 mg (0,03 mmole) de bis(tri-tertbutylphosphine)palladium dans 2 mL de 1-méthyl-2-pyrrolidinone (NMP) sec. On obtient 28 mg du produit attendu.
PF = 110 - 112 °C
R.M.N. ¹H (DMSO D₆), δ (ppm) : 10,2 (s, 1H) ; 8,4 (d, 1H) ; 7,85 (m, 2H) ; 7,55 (d, 1H) ; 7,4-7,3 (m, 3H) ; 7,05 (m, 1H) ; 6,95 (m, 2H) ; 6,4 (d, 1H) ; 5,9 (s, 2H) ; 3,9 (t, 4H) ; 2,3 (quint., 2H) ; 0,9 (s, 9H) ; +0,3(s, 6H).
LC-MS: 515 ([M+H]+, Rt= 1,53).

### Exemple 19 (Composé N°19)

### N-[6-(Pyrrolidin-1-yl)pyridin-3-yl]-5-(cyclohexyl)diméthylsilyl-1-[(3-fluorophényl)méthyl]-1H-indole-2-carboxamide

### 19.1 N-[6-(Pyrrolidin-1-yl)pyridin-3-yl]-5-iodo-1-[(3-fluorophényl)méthyl]-1H-indole-2-carboxamide

Le composé a été préparé selon un procédé similaire à celui décrit à l'étape 5.4 en faisant réagir 0,3 g (0,76 mmole) de l'acide 5-iodo-1-[(3-fluorophényl)méthyl]-1*H* indole-2-carboxylique préparé selon le protocole décrit à l'étape 8.2 avec 148 mg (0,91 mmole) de 3-amino-6-(pyrrolidin-1-yl)pyridine en présence de 145 mg (0,76 mmole) du chlorhydrate de *N*-(3-diméthylaminopropyl)-*N'*éthylcarbodiimide (EDAC) et de 105 mg (0,76 mmole) de 1-hydroxy-benzotriazole (HOBT) dans 5 mL de diméthylformamide. On obtient 290 mg du produit attendu.
R.M.N. ¹H (DMSO D₆), δ (ppm) : 10,3 (s, 1H) ; 8,4 (d, 1H) ; 8,15 (s, 1H) ; 7,85 (dd, 1H) ; 7,55 (d, 1H) ; 7,45 (d, 1H) ; 7,3 (m, 2H) ; 7,05 (m, 1H) ; 6,95 (m, 2H) ; 6,45 (d, 1H) ; 5,9 (s, 2H) ; 3,4 (m, 4H) ; 1,9 (m, 4H).
LC-MS: 541 ([M+H]+, Rt= 1,25).

### 19.2 N-[6-(Pyrrolidin-1-yl)pyridin-3-yl]-5-(cyclohexyl)diméthylsilyl-1-[(3-fluorophényl)méthyl]-1H-indole-2-carboxamide (composé 19)

Le composé N° 19 a été préparé selon un procédé similaire à celui décrit à l'étape 8.4 en faisant réagir 0,15 g (0,28 mmole) de *N*-[6-(pyrrolidin-1-yl)pyridin-3-yl]-5-iodo-1-[(3-fluorophényl)méthyl]-1*H-*indole-2-carboxamide préparé selon le protocole décrit à l'étape 19.1 avec 120 mg (0,83 mmole) de cyclohexyldimethylsilane en présence de 0,18 g (0,83 mmole) de triphosphate de potassium et de 14 mg (0,027mmole) de bis(tri-tertbutylphosphine)palladium dans 2 mL de 1-méthyl-2-pyrrolidinone (NMP) sec. On obtient 41 mg du produit attendu.
PF=192-194°C
R.M.N. ¹H (DMSO D₆), δ (ppm) : 10,2 (s, 1H) ; 8,4 (d, 1H) ; 7,85 (m, 2H) ; 7,55 (d, 1H) ; 7,4-7,3 (m, 3H) ; 7,05 (m, 1H) ; 6,95 (m, 2H) ; 6,45 (d, 1H) ; 5,9 (s, 2H) ; 3,4 (m, 4H) ; 2,0 (m, 4H) ; 1,7 (m, 4H) ; 1,15 (m, 6H) ; 0,8 (m, 1H) ; 0,3 (s, 6H).
LC-MS: 555 ([M+H]+, Rt= 1,64).

### Exemple 20 (Composé N°20)

### N-[6-(Azétidin-1-yl)pyridin-3-yl]-5-(phényl)diméthylsilyl-1-[(3-fluorophényl)méthyl]-1H-indole-2-carboxamide (composé N°20)

Le composé N° 20 a été préparé selon un procédé similaire à celui décrit à l'étape 8.4 en faisant réagir 0,25 g (0,28 mmole) de *N-*[6-(azétidin-1-yl)pyridin-3-yl]-5-iodo-1-[(3-fluorophényl)méthyl]-1*H*-indole-2-carboxamide préparé selon le protocole décrit à l'étape 8.3 avec 71 mg (0,52 mmole) de phényldiméthylsilane en présence de 0,30 g (1,42 mmole) de triphosphate de potassium et de 24 mg (0,05 mmole) de bis(tri-tertbutylphosphine)palladium dans 3 mL de 1-méthyl-2-pyrrolidinone (NMP) sec. On obtient 140 mg du produit attendu sous la forme d'un solide blanc.
PF = 200 - 202 °C
R.M.N. ¹H (DMSO D₆), δ (ppm) : 10,2 (s, 1H) ; 8,4 (s, 1H) ; 7,85 (m, 2H) ; 7,55 (m, 3H) ; 7,45-7,25 (m, 6H) ; 7,05 (m, 1H) ; 6,95 (m, 2H) ; 6,4 (d, 1H) ; 5,9 (s, 2H) ; 3,95 (t, 4H) ; 2,3 (quint., 2H) ; 0,6 (s, 6H).
LC-MS: 535 ([M+H]+, Rt= 1,44).

### Exemple 21 (Composé N°21 )

### N-[6-(Azétidin-1-yl)pyridin-3-yl]-5-(isopropyl)diméthylsilyl-1-[(3-fluorophényl)méthyl]-1H-indole-2-carboxamide

Le composé N° 21 a été préparé selon un procédé similaire à celui décrit à l'étape 8.4 en faisant réagir 0,15 g (0,31 mmole) de *N*-[6-(azétidin-1-yl)pyridin-3-yl]-5-bromo-1-[(3-fluorophényl)méthyl]-1*H*-indole-2-carboxamide préparé selon le protocole décrit à l'étape 17.3 avec 95 mg (0,93 mmole) de dimethyl(isopropyl)silane en présence de 0,2 g (0,94 mmole) de triphosphate de potassium et de 16 mg (0,03 mmole) de bis(tri-*tert*butylphosphine)palladium dans 2 mL de 1-méthyl-2-pyrrolidinone (NMP) sec. On obtient 23 mg du produit attendu.
PF = 179-180°C
R.M.N. ¹H (DMSO D₆), δ (ppm) : 10,25 (s, 1H) ; 8,35 (s, 1H) ; 7,85 (m, 2H) ; 7,55 (m, 1H) ; 7,3 (m, 3H) ; 7,05 (m, 1H) ; 6,95 (m, 2H) ; 6,4 (d, 1H) ; 5,9 (s, 2H) ; 3,9 (m, 4H) ; 2,3 (m, 2H) ; 0,9 (m, 7H) ; 0,3(s, 6H).
LC-MS: 501 ([M+H]+, *R*t= 1,49).

### Exemple 22 (Composé N°22)

### N-[6-(Azétidin-1-yl)pyridin-3-yl]-5-(méthyl)diéthylsilyl-1-[(3-fluorophényl)méthyl]-1H-indole-2-carboxamide

Le composé N° 22 a été préparé selon un procédé similaire à celui décrit à l'étape 8.4 en faisant réagir 0,15 g (0,31 mmole) de *N*-[6-(azétidin-1-yl)pyridin-3-yl]-5-bromo-1-[(3-fluorophényl)méthyl]-1*H*-indole-2-carboxamide préparé selon le protocole décrit à l'étape 17.3 avec 95 mg (0,93 mmole) de diéthyl(méthyl)silane en présence de 0,2 g (0,94 mmole) de triphosphate de potassium et de 16 mg (0,03 mmole) de bis(tri-tertbutylphosphine)palladium dans 2 mL de 1-méthyl-2-pyrrolidinone (NMP) sec. On obtient 13 mg du produit attendu.
PF = 171-173°C
R.M.N. ¹H (DMSO D₆), δ (ppm) : 10,2 (s, 1H) ; 8,35 (s, 1H) ; 7,85 (m, 2H) ; 7,55 (m, 1H) ; 7,35 (m, 3H) ; 7,05 (m, 1H) ; 6,95 (m, 2H) ; 6,4 (d, 1H) ; 5,9 (s, 2H) ; 3,9 (m, 4H) ; 2,3 (m, 2H) ; 0,95 (m, 6H) ; 0,8(m, 4H) ; 0,25 (s, 3H).
LC-MS: 501 ([M+H]+, Rt= 1,50).

Le tableau 1 qui suit illustre les structures chimiques et les propriétés physiques de quelques composés de formule générale (l) selon l'invention.

Dans ce tableau :
- les composés de formule générale (l) sont définis avec n égal à 1 ;
- la colonne « PF (°C) ou [MH]⁺ » renseigne respectivement, soit les points de fusion des produits en degrés Celsius (°C), soit leur pic de masse en LC-MS ; la méthode de chromatographie liquide (LC) employée pour la détermination des pics de masse des composés n° 15 et 16 du tableau 1 est la suivante :
   Appareil : UPLC / TOF
   Phases mobiles : A = H2O + 0.05% TFA et B = ACN + 0.035% TFA
   Gradient 3 min : T0: 98%A T1,6 à T2,1min : 100%B T2,5 à T3min : 98%A
   Débit : 1.0mL/min - T° colonne = 40°C - Injection 2µL
   Colonne Acquity BEH C18 (50*2,1mm ; 1,7µm)
- les composés sont sous forme de base libre ;
- « CH₃ » correspond à un groupe méthyle, « Et » correspond à un groupe éthyle, « cHex » correspond à un groupe cyclohexyle, «te*rt*Bu » correspond à un groupe *tert*butyle, « Ph » correspond à un groupe phényle, « iPr » correspond à un groupe isopropyle

**Tableau 1**

| | | | | | |
|---|---|---|---|---|---|
| 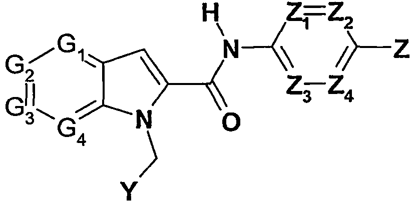 | | | | | |

| **N°** | **G₁, G₂, G₃, G₄** | **Y** | **Z₁, Z₂, Z₃, Z₄** | **Z** | **PF (°C) (ou [MH]⁺)** |
|---|---|---|---|---|---|
| 1 | CH, CH, -Si(CH₃)₃, CH | 3-fluorophényl | CH,CH,CH,N | Azétidinyle | 208-210 |
| 2 | CH, CH, -Si(CH₃)₃, CH | 3-fluorophényl | CH,CH,CH,N | 3-Hydroxy azétidinyle | 188-189 |
| 3 | CH, -Si(CH₃)₃, CH, CH | 3-fluorophényl | CH,CH,CH,N | Azétidinyle | 212-213 |
| 4 | CH, -Si(CH₃)₃, CH, CH | 3-fluorophényl | CH,CH,CH,N | 3-Hydroxy azétidinyle | 207-208 |
| 5 | CH, CH, CH, -Si(CH₃)₃ | 3-fluorophényl | CH,CH,CH,N | Azétidinyle | 160-162 |
| 6 | CH, CH, CH, -Si(CH₃)₃ | 3-fluorophényl | CH,CH,CH,N | 3-Hydroxy azétidinyle | 215-217 |
| 7 | CH, -Si(CH₃)₃, CH, CH | 3-fluorophényl | CH,CH,CH,N | diméthyami no | 204-205 |
| 8 | CH, -Si(Me₂)Et, CH, CH | 3-fluorophényl | CH,CH,CH,N | Azétidinyle | 188-189 |
| 9 | CH, -Si(cHex)(Me₂), CH, CH | 3-fluorophényl | CH,CH,CH,N | Azétidinyle | 202-203 |
| 10 | CH, CH, -Si(CH₃)₃, CH | 3-(trifluorométhyl) phényl | CH,CH,CH,N | Azétidinyle | 167-168 |
| 11 | CH, CH, -Si(CH₃)₃, CH | 3-(trifluorométhyl) phényl | CH,CH,CH,N | 3-Hydroxy azétidinyle | 183-184 |
| 12 | CH, -SiCH₃(Ph)₂, CH, CH | 3-fluorophényl | CH,CH,CH,N | Azétidinyle | 98-100 |
| 13 | CH, -Si(CH₃)₃, CH, CH | 3-(trifluorométhyl) phényl | CH,CH,CH,N | Azétidinyle | 199-200 |
| 14 | CH, -Si(CH₃)₃, CH, CH | 3-(trifluorométhyl) phényl | CH,CH,CH,N | 3-Hydroxy azétidinyle | 217-218 |
| 15 | CH, CH, -Si(CH₃)₃,CH | pyridin-4-yl | CH,CH,CH,N | 3-Hydroxy azétidinyle | (472) |
| 16 | CH, CH, -Si(CH₃)₃, CH | pyridin-4-yl | CH,CH,CH,N | Azétidinyle | (456) |
| 17 | CH, -Si(Et)₃, CH, CH | 3-fluorophényl | CH,CH,CH,N | Azétidinyle | 166-168 |
| 18 | CH, -Si(*tert*Bu)(Me₂),CH, CH | 3-fluorophényl | CH,CH,CH,N | Azétidinyle | 110-112 |
| 19 | CH, -Si(cHex)(Me₂), CH, CH | 3-fluorophényl | CH,CH,CH,N | Pyrrolidinyle | 192-194 |
| 20 | CH, -Si(Me_{z})Ph, CH, CH | 3-fluorophényl | CH,CH,CH,N | Azétidinyle | 200-202 |
| 21 | CH, -Si(iPr)(Me₂), CH, CH | 3-fluorophényl | CH,CH,CH,N | Azétidinyle | 179-180 |
| 22 | CH, -Si(Et₂)(Me), CH, CH | 3-fluorophényl | CH,CH,CH,N | Azétidinyle | 171-173 |

Les composés de l'invention ont été soumis à des essais pharmacologiques *in vitro* et *in vivo* qui ont mis en évidence leur intérêt comme substances à activités thérapeutiques.

Les composés de l'invention présentent également des caractéristiques de solubilité dans l'eau qui favorise une bonne activité *in vivo.*

### Test d'inhibition du courant induit par la capsaïcine sur les DRG de rat

### - Culture primaire de cellules de ganglions de racine dorsale (DRG) de rat :

Les neurones du DRG expriment naturellement le récepteur TRPV1. Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 µg/ml gentamicine et 50 ng/ml de NGF, puis déposées sur des lamelles de verre recouvertes de laminine (0.25 x 106 cellules par lamelle) qui sont ensuite placées dans des boîtes 12 puits Coming. Les cellules sont incubées à 37°C en atmosphère humidifiée contenant 5% de CO₂ et 95% d'air. De la cytosine β-D-arabinoside (1 µM) est ajoutée 48 h après la mise en culture, pour prévenir le développement des cellules non neuronales. Les lamelles sont transférées dans les chambres expérimentales pour les études de patch-clamp après 7-10 jours de culture.

### - Electrophysiologie :

Les chambres de mesure (volume 800 µl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont la sortie unique, constituée par un tube de polyéthylène (ouverture 500µm) est placée à moins de 3 mm de la cellule étudiée. La configuration "cellule entière" de la technique de patch-clamp à été utilisée. Les pipettes en verre borosilicaté (résistance 5-10 MOhms) sont approchées de la cellule grâce à un micromanipulateur piézoélectrique 3D ( Burleigh, PC1000) . Les courants globaux (potentiel de membrane fixé à -60 mV) sont enregistrés avec un amplificateur Axopatch 1 D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution micromolaire de capsaïcine , provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 0,1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.
Les pourcentages d'inhibition de la réponse capsaïcine (1 microM) sont compris entre 20% et 100% pour les composés les plus actifs de l'invention testés à la concentration de 10 nM à 0,1 nM (voir exemple dans le tableau 2).
Les composés de l'invention sont donc des antagonistes efficaces in vitro des récepteurs de type TRPV1.

**Tableau 2**

| **N°composé** | **% inhibition en patch DRG** |
|---|---|
| 2 | 84% (1 nM) |
| 3 | 95% (10nM) |

Les résultats de ces essais montrent que les composés les plus actifs de l'invention bloquent les effets induits par la stimulation des récepteurs TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formule (l), ou un sel pharmaceutiquement acceptable, ou encore un hydrate ou un solvat dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.
Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.
On peuvent également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.
Les composés de l'invention peuvent également être utilisés pour la préparation d'un médicament destiné à prévenir et/ou traiter les désordres métaboliques tels que le diabète.
De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.
Les composés de l'invention peuvent également être utilisés pour la préparation d'un médicament destiné à traiter la dépression.
Les composés de l'invention pourraient aussi être utilisés pour traiter les maladies du système nerveux central telles que la sclérose en plaques.
Les composés de l'invention pourraient aussi être utilisés pour traiter les cancers.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (l) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique. Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Décrite est également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (l) 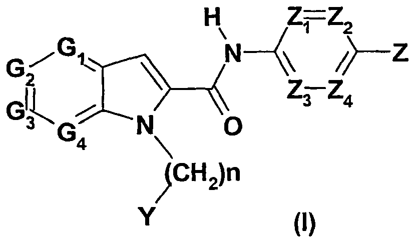 dans laquelle
G₁ G₂, G₃ et G₄ représentent, indépendamment l'un de l'autre, un groupe C-X ou un atome d'azote ;
X est choisi parmi un atome d'hydrogène, d'halogène ou un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxyle, C₃-C₇-cycloalcoxyle, C₃-C₇-cycloalkyl-C₁-C₆,-alkylène-O-, C₁-C₆-fluoroalcoxyle, cyano, C₁-C₆-thioalkyle, NR₁R₂, aryl-C₁-C₆-alkylène-O, aryle, hétéroaryle ou -Si(X₁)(X₂)(X₃) ; les groupes C₁-C₆-alkyle, C₃-C₇₋cycloalkyle, C₃-C₇₋cycloalkyl-C₁-C₃-alkylène, C₁-C₆-fluoroalkyle, C₁-C₆-alcoxyle, C₃-C₇-cycloalcoxyle, C₃-C₇-cycloalkyl-C₁-C₆-alkylène-O- étant éventuellement substitués par un groupe hydroxy, C₁-C₆-alcoxyle ou NR₄R₅ ; l'aryle et l'hétéroaryle étant éventuellement substitué par un ou plusieurs substituants choisis parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
l'un des G₁, G₂, G₃ et G₄ et au maximum l'un des G₁, G₂, G₃ et G₄ représente un groupe C-X où X est un groupe-Si(X₁)(X₂)(X₃) ;
X₁, X₂, X₃ représentent, indépendamment l'un de l'autre, un groupe C₁-C₆-alkyle, C₃-C₇₋cycloalkyle, C₃-C₇-cycloalkyl-C₁-C₆-alkyle, hydroxyle, aryle ou hétéroaryle ;
les groupes aryle ou hétéroaryle de X₁, X₂, X₃ étant éventuellement substitués par un ou plusieurs substituants choisi parmi un halogène, un groupe C₁-C₆-alkyle, C₃-C₇ cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
les groupes C₁-C₆-alkyle, C₃-C₇-cycloalkyle, C₃-C₇-cycloalkyl-C₁-C₆-alkyle de X₁, X₂, X₃ étant éventuellement substitués par un ou plusieurs substituants choisi parmi un groupe hydroxyle, C₁-C₆-alcoxyle, aryl-O-, NR₁R₂, aryle ou hétéroaryle, l'aryle et l'hétéroaryle étant éventuellement substitué par un ou plusieurs substituants choisis parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
X₁ et X₂ pouvant également former ensemble avec l'atome de silicium qui les porte un cycle de 4 à 7 chaînons contenant éventuellement un hétéroatome choisi parmi O, S et N;
n est égal à 0, 1, 2 ou 3 ;
Y représente un aryle ou un hétéroaryle ; l'aryle ou l'hétéroaryle étant éventuellement substitué par un ou plusieurs groupes choisis parmi un atome d'halogène ou un groupe C₁-C₆-alkyle, C₃-C₇-cycloalkyle, C₃-C₇-cycloalkyle-C₁-C₆-alkylène, C₁-C₆-fluoroalkyle, hydroxyle, C₁-C₆-alcoxyle, C₃-C₇-cycloalcoxyle, C₃-C₇-cycloalkyle-C₁-C₆-alkylène-O-, C₁-C₆-fluoroalcoxyle, cyano, C₁-C₆-thioalkyle, thiol, -S(O)-C₁-C₆-alkyle, -S(O)₂-C₁-C₆-alkyle, C(O)NR₁R₂, SO₂NR₁R₂, SF₅, nitro, OCONR₁R₂, NR₃COR₄, NR₃CONR₁R₂, NR₁R₂, NR₃SO₂NR₁R₂, NR₃COR₄, NR₃SO₂R₅, aryl-C₁-C₆-alkylène ou aryle ; l'aryle et l'aryl-C₁-C₆-alkylène étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
Z₁, Z₂, Z₃, Z₄ représentent, indépendamment l'un de l'autre, un atome d'azote ou un groupe C(R₆), l'un au moins correspondant à un atome d'azote et l'un au moins correspondant à un groupe C(R₆) ; l'atome d'azote ou un des atomes d'azote présent dans le cycle, défini comme azote de position 1, étant éventuellement substitué par R₇ lorsque l'atome de carbone en position 2 ou 4 par rapport à cet azote de référence est substitué par un groupe oxo ou thio ;
Z représente
soit une amine cyclique reliée par l'atome d'azote, de formule : 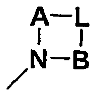 dans laquelle
A représente un groupe C₁-C₇-alkylène éventuellement substitué par un ou deux groupes R₈ ;
B représente un groupe C₁-C₇-alkylène éventuellement substitué par un ou deux groupes R₉ ;
L représente une liaison, un atome de soufre, d'oxygène ou d'azote ; l'atome d'azote étant éventuellement substitué par un groupe R₁₀ ou R₁₁ ;
les atomes de carbone de l'amine cyclique Z étant éventuellement substitués par un ou plusieurs groupes R₁₂ identiques ou différents l'un de l'autre ;
soit une amine acyclique, reliée par l'atome d'azote, de formule NRaRb dans laquelle Ra et Rb représentent, indépendamment l'un de l'autre, un atome d'hydrogène ou un groupe C₁-C₆-alkyle, C₃-C₇-cycloalkyle, C₃-C₇-cycloalkyle-C₁-C₆-alkylène, C₁-C₆-fluoroalkyle, hydroxyle, C₁-C₆-alcoxyle, C₃-C₇-cycloalkyle-C₁-C₆-alkylène-O-, C₁-C₆-fluoroalcoxyle, C₁-C₆-alkyle-C(O)-, HO-C(O)-C₁-C₆-alkylène, C₁-C₆-alkyle-OC(O)-C₁-C₆-alkylène, aryle ou hétéroaryle, Ra et Rb pouvant être éventuellement substitués par un ou plusieurs groupes Rc identiques ou différents l'un de l'autre ;
Rc représente un atome d'halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C7-cycloalkyle-C₁-C₆-alkylène-O-, C₁-C₆-fluoroalcoxyle, C₁-C₆-thioalkyle, -S(O)-C₁-C₆-alkyle, -S(O)₂-C₁-C₆-alkyle, cyano, C(O)NRₗR₂, NR₁R₂, SO₂NR₁R₂, NR₃COR₄, NR₃SO₂R₅, OC(O)NR₁R₂. NR₃COOR₅, NR₃CONR₁R₂, NR₃SO₂NR₁R₂, hydroxyle, thiol, oxo, thio, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₁ et R₂ représentent, indépendamment l'un de l'autre, un atome d'hydrogène ou un groupe C₁-C₆-alkyle, C₃-C₇-cycloalkyle, C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇ cycloalkyle, C₃-C₇-cycloalkyle-C₁-C₆,-alkylène, C₁-C₆-fluoroalkyle, C₁-C₆-alcoxyle, C₁-C₆-fluoroalcoxyle, nitro ou cyano ; ou R₁ et R₂ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe C₁-C₆-alkyle, C₃-C₇-cycloalkyle, C₃-C₇₋cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇₋cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₃ et R₄ représentent, indépendamment l'un de l'autre,
un atome d'hydrogène ou un groupe C₁-C₆-alkyle, C₃-C₇-cycloalkyle, C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
ou R₃ et R₄ forment ensemble un groupe (C₂-C₅)alkylène ;
ou R₁ et R₃ forment ensemble un groupe (C₂-C₅)alkylène ;
R₅ représente un groupe C₁-C₆-alkyle, C₃-C₇-cycloalkyle, C₃-C₇-cycloalkyle-C₁-C₆-alkylène, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
ou R₃ et R₅ forment ensemble un groupe (C₂-C₅)alkylène ;
R₆ représente un atome d'hydrogène ou d'halogène ou un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, C₁-C₆-thioalkyle, -S(O)-C₁-C₆-alkyle, -S(O)₂-C₁-C₆-alkyle, hydroxyle, thiol, oxo, thio, aryle, aryl-C₁-C₆-alkylène, hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₇ représente un atome d'hydrogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₃-C₇₋cycloalkyle-C₁-C₆-alkylène-O-, C₁-C₆-fluoroalcoxyle, aryle, aryl-C₁-C₆-alkylène ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₁-C₆-fluoroalcoxyle, nitro ou cyano ;
R₈, R₉ et R₁₀ sont définis tels que :
deux groupes R₈ peuvent former ensemble une liaison ou un groupe C₁-C₆-alkylène ; deux groupes R₉ peuvent former ensemble une liaison ou un groupe C₁-C₆-alkylène ; R₈ et R₉ peuvent former ensemble une liaison ou un groupe C₁-C₆-alkylène ;
R₈ et R₁₀ peuvent former ensemble une liaison ou un groupe C₁-C₆-alkylène ;
R₉ et R₁₀ peuvent former ensemble une liaison ou un groupe C₁-C₆-alkylène ;
R₁₁ représente un atome d'hydrogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, hydroxyle, C₁-C₆-alkyl-CO-, COOR₅, C(O)NR₁R₂, aryl-C₁-C₆-alkylène, aryle ou hétéroaryle, l'aryle et l'hétéroaryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₁-C₆-fluoroalcoxyle, nitro ou cyano ;
R₁₂ représente un atome de fluor, un groupe C₁-C₆-alkyle éventuellement substitué par un groupe R₁₃ C₃-C₇-cycloalkyle, C₃-C₇-cycloalkyle-C₁-C₆-alkylène, C₁-C₆-fluoroalkyle, C₁-C₆-cycloalk-1,1-diyle, C₃-C₇-hétérocycloalk-1,1-diyle éventuellement substitué sur un atome d'azote par un groupe R₁₁ ; C₁-C₆-alcoxyle, C₃-C₇-cycloalcoxyle, C₃-C₇-cycloalkyle-C₁-C₆-alkylène-O-, C₁-C₆-fluoroalcoxyle, CO₂H C(O)O-C₁-C₆-alkyle, C(O)NR₁R₂, NR₁R₂, NR₃COR₄, OC(O)NR₁R₂. NR₃COOR₅, NR₃CONR₁R₂, hydroxyle, thiol, oxo, thio, aryl-C₁-C₆-alkylène, aryle ; l'aryle étant éventuellement substitué par un ou plusieurs substituants choisi parmi un halogène, un groupe C₁-C₆-alkyle, C₃-C₇-cycloalkyle, C₃-C₇-cycloalkyle-C₁-C₆-alkylène, C₁-C₆-fluoroalkyle, C₁-C₆-alcoxyle, C₃-C₇-cycloalcoxyle, C₁-C₆-fluoroalcoxyle, nitro ou cyano ;
R₁₃ représente un groupe C₁-C₆-alcoxyle, C₃-C₇-cycloalcoxyle, C₃-C₇-cycloalkyle-C₁-C₆-alkylène-O-, C(O)NR₁R₂, NR₁R_{2,} NR₃COR₄, OC(O)NR₁R₂ NR₃COOR₅, hydroxyle ;
le ou les atomes d'azote des composés de formule générale (l) pouvant être sous forme oxydée ;
à l'état de base ou de sel d'addition à un acide, ainsi qu'à l'état d'hydrate ou de solvate.

2. Composé de formule (l) selon la revendication 1, **caractérisé en ce que**
l'un de G₁, G₂, G₃ et G₄ représente un groupe C-X où X est un groupe -Si(X₁)(X₂)(X₃) ; les autres de G₁, G₂, G₃ et G₄ représentent un groupe CH ;
X₁, X₂, X₃ représentent, indépendamment l'un de l'autre, un groupe C₁-C₆-alkyle, C₃-C₇-cycloalkyle ou aryle ; à l'état de base ou de sel d'addition à un acide, ainsi qu'à l'état d'hydrate ou de solvate.

3. Composé de formule (l) selon la revendication 1 ou 2, **caractérisé en ce que** n est égal à 1 ; à l'état de base ou de sel d'addition à un acide, ainsi qu'à l'état d'hydrate ou de solvate.

4. Composé de formule (l) selon l'une quelconque des revendications 1 à 3, **caractérisé en ce que** Y représente un aryle ou un hétéroaryle ; l'aryle ou l'hétéroaryle étant éventuellement substitué par un ou plusieurs groupes choisis parmi un atome d'halogène ou un groupe C₁-C₆-fluoroalkyle ; à l'état de base ou de sel d'addition à un acide, ainsi qu'à l'état d'hydrate ou de solvate.

5. Composé de formule (l) selon l'une quelconque des revendications 1 à 4, **caractérisé en ce que** Z₁, Z₂, Z₃, Z₄ représentent, indépendamment l'un de l'autre, un atome d'azote ou un groupe C(R₆), l'un correspondant à un atome d'azote et les autres correspondant à un groupe C(R₆) ; R₆ représente un atome d'hydrogène ;
à l'état de base ou de sel d'addition à un acide, ainsi qu'à l'état d'hydrate ou de solvate.

6. Composé de formule (l) selon l'une quelconque des revendications 1 à 5, **caractérisé en ce que**
Z représente une amine cyclique reliée par l'atome d'azote, de formule : 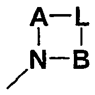 dans laquelle
A représente un groupe C₁-C₇-alkylène éventuellement substitué par un ou deux groupes R₈;
B représente un groupe C₁-C₇-alkylène éventuellement substitué par un ou deux groupes R₉;
L représente une liaison, un atome de soufre, d'oxygène ou d'azote ; l'atome d'azote étant éventuellement substitué par un groupe R₁₀ ou R₁₁ ;
les atomes de carbone de l'amine cyclique Z étant éventuellement substitués par un ou plusieurs groupes R₁₂ identiques ou différents l'un de l'autre ;
R₈, R₉, R₁₀, R₁₁ et R₁₂ étant tels que définis dans la formule générale (l) selon la revendication 1 ;
à l'état de base ou de sel d'addition à un acide, ainsi qu'à l'état d'hydrate ou de solvate.

7. Composé de formule (l) selon l'une quelconque des revendications 1 à 6, **caractérisé en ce que**
Z représente une amine cyclique reliée par l'atome d'azote, de formule : 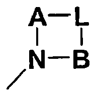 dans laquelle
A représente un groupe C₁-C₃-alkylène ;
B représente un groupe C₁-C₃-alkylène ;
L représente une liaison ;
les atomes de carbone de l'amine cyclique Z étant éventuellement substitués par un ou plusieurs groupes R₁₂ identiques ou différents l'un de l'autre ;
R₁₂ représente un groupe hydroxyle ;
à l'état de base ou de sel d'addition à un acide, ainsi qu'à l'état d'hydrate ou de solvate.

8. Composé de formule (l) selon l'une quelconque des revendications 1 à 5, **caractérisé en ce que** Z représente une amine acyclique, reliée par l'atome d'azote, de formule NRaRb dans laquelle Ra et Rb représentent, indépendamment l'un de l'autre, un atome d'hydrogène ou un groupe C₁-C₆-alkyle ; à l'état de base ou de sel d'addition à un acide, ainsi qu'à l'état d'hydrate ou de solvate.

9. Composé de formule (l) selon l'une quelconque des revendications 1 à 8, **caractérisé en ce que**
l'un de G₁, G₂, G₃ et G₄ représente un groupe C-X où X est un groupe -Si(X₁)(X₂)(X₃) ; les autres de G₁, G_{2.} G₃ et G₄ représentent un groupe CH ;
X₁, X₂, X₃ représentent, indépendamment l'un de l'autre, un groupe C₁-C₆-alkyle, C₃-C₇-cycloalkyle ou aryle ;
n est égal à 1 ;
Y représente un aryle ou un hétéroaryle ; l'aryle ou l'hétéroaryle étant éventuellement substitué par un ou plusieurs groupes choisis parmi un atome d'halogène ou un groupe C₁-C₆-fluoroalkyle ;
Z₁, Z₂, Z₃, Z₄ représentent, indépendamment l'un de l'autre, un atome d'azote ou un groupe C(R₆), l'un correspondant à un atome d'azote et les autres correspondant à un groupe C(R₆) ; R₆ représente un atome d'hydrogène ;
Z représente
soit une amine cyclique reliée par l'atome d'azote, de formule : 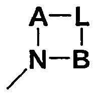 dans laquelle
A représente un groupe C₁-C₃-alkylène ;
B représente un groupe C₁-C₃-alkylène ;
L représente une liaison ;
les atomes de carbone de l'amine cyclique Z étant éventuellement substitués par un ou plusieurs groupes R₁₂ identiques ou différents l'un de l'autre ;
R₁₂ représente un groupe hydroxyle ;
soit une amine acyclique, reliée par l'atome d'azote, de formule NRaRb dans laquelle Ra et Rb représentent, indépendamment l'un de l'autre, un atome d'hydrogène ou un groupe C₁-C₆-alkyle ;
à l'état de base ou de sel d'addition à un acide, ainsi qu'à l'état d'hydrate ou de solvate.

10. Composé de formule (l) selon la revendication 1, choisi parmi :
*N*-[6-(azétidin-1-yl)pyridin-3-yl]-6-triméthylsilyl-1-[(3-fluorophényl)méthyl]-1H-indole-2-carboxamide ;
*N*-[6-(3-hydroxyazétidin-1-yl)pyridin-3-yl]-6-triméthylsilyl-1-[(3-fluorophényl)méthyl]-1*H-*indole-2-carboxamide ;
*N*-[6-(azétidin-1-yl)pyridin-3-yl]-5-triméthylsilyl-1-[(3-fluorophényl)méthyl]-1*H*-indole-2-carboxamide ;
*N*-[6-(3-hydroxyazétidin-1-yl)pyridin-3-yl]-5-triméthylsilyl-1-[(3-fluorophényl)méthyl]-1*H-*indole-2-carboxamide ;
*N*-[6-(azétidin-1-yl)pyridin-3-yl]-7-triméthylsilyl-1-[(3-fluorophényl)méthyl]-1*H*-indole-2-carboxamide ;
*N*-[6-(3-hydroxyazétidin-1-yl)pyridin-3-yl]-7-triméthylsilyl-1-[(3-fluorophényl)méthyl]-1*H-*indole-2-carboxamide ;
*N*-[6-(Diméthylamino)pyridin-3-yl]-5-triméthylsilyl-1-[(3-fluorophényl)méthyl]-1*H*-indole-2-carboxamide ;
*N*-[6-(azétidin-1-yl)pyridin-3-yl]-5-(éthyl)diméthylsilyl-1-[(3-fluorophényl)méthyl]-1*H-*indole-2-carboxamide ;
*N*-[6-(azétidin-1-yl)pyridin-3-yl]-5-(cyclohexyl)diméthylsilyl-1-[(3-fluorophényl)méthyl]-1*H*-indole-2-carboxamide ;
1*N* [6-(azétidin-1-yl)pyridin-3-yl]-6-triméthylsilyl-1-[[(3-trifluorométhyl)phényl]méthyl]-1*H-*indole-2-carboxamide ;
*N*-[6-(3-hydroxyazétidin-1-yl)pyridin-3-yl]-6-triméthylsilyl-1-[[(3-trifluorométhyl)phényl]méthyl]-1*H-*indole-2-carboxamide ;
*N*-[6-(azétidin-1-yl)pyridin-3-yl]-5-(diphényl)méthylsilyl-1-[(3-fluorophényl)méthyl]-1*H-*indole-2-carboxamide ;
*N-*[6-(azétidin-1-yl)pyridin-3-yl]-5-triméthylsilyl-1-[[(3-trifluorométhyl)phényl]méthyl]-1*H-*indole-2-carboxamide ;
*N*-[6-(3-hydroxyazétidin-1-yl)pyridin-3-yl]-5-triméthylsilyl-1-[[(3-trifluorométhyl)phényljméthyl]-1*H-*indole-2-carboxamide ;
*N*-[6-(3-hydroxyazétidin-1-yl)pyridin-3-yl]-6-triméthylsilyl-1-[(pyridin-4-yl)méthyl]-1*H-*indole-2-carboxamide ;
*N*-[6-(azétidin-1-yl)pyridin-3-yl)pyridin-3-yl]-6-triméthylsilyl-1-[(pyridin-4-yl)méthyl]-1*H-*indole-2-carboxamide ;
*N*-[6-(azétidin-1-yl)pyridin-3-yl]-5-triéthylsilyl-1-[(3-fluorophényl)méthyl]-1*H*-indole-2-carboxamide ;
*N*-[6-(azétidin-1-yl)pyridin-3-yl]-5-tert-butyl(diméthyl)silyl-1-[(3-fluorophényl)méthyl]-1*H-*indole-2-carboxamide ;
*N*-[6-(Pyrrolidin-1-yl)pyridin-3-yl]-5-(cyclohexyl)diméthylsilyl-1-[(3-fluorophényl)méthyl]-1 H-indole-2-carboxamide ;
*N-*[6-(azétidin-1-yl)pyridin-3-yl]-5-phényldiméthylsilyl-1-[(3-fluorophényl)méthyl]-1*H-*indole-2-carboxamide ;
*N*-[6-(azétidin-1-yl)pyridin-3-yl]-5-(isopropyl)diméthylsilyl-1-[(3-fluorophényl)méthyl]-1*H-*indole-2-carboxamide ;
*N*-[6-(Azétidin-1-yl)pyridin-3-yl]-5-(méthyl)diéthylsilyl-1-[(3-fluorophényl)méthyl]-1*H-*indole-2-carboxamide.

11. Procédé de préparation d'un composé de formule (l) selon l'une quelconque des revendications 1 à 10, **caractérisé en ce que** l'on fait réagir un composé de formule générale (IV) 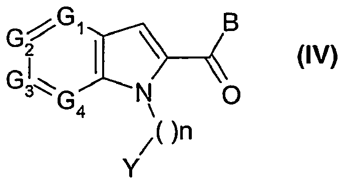 dans laquelle G₁, G₂, G₃ et G₄, Y et n sont tels que définis dans la formule générale (l) selon la revendication 1 et B représente un atome de chlore,
avec une amine de formule générale (V) 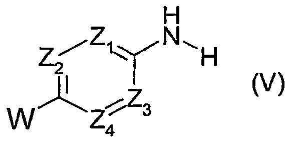 dans laquelle W = Z et Z, Z₁, Z₂, Z₃ et Z₄ sont tels que définis dans la formule générale (l) selon la revendication 1, dans un solvant.

12. Procédé de préparation d'un composé de formule (l) selon l'une quelconque des revendications 1 à 10,
**caractérisé en ce que** l'on fait réagir un composé de formule générale (IV) 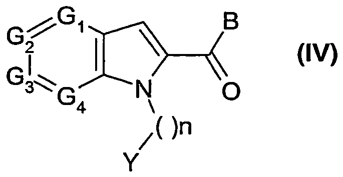 dans laquelle G₁, G₂, G₃ et G₄, Y et n sont tels que définis dans la formule générale (l) selon la revendication 1 et B représente un groupe hydroxyle,
avec une amine de formule générale (V) 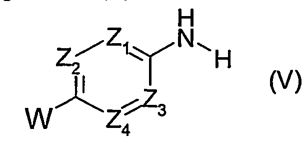 dans laquelle W = Z et Z, Z₁, Z₂, Z₃ et Z₄ sont tels que définis dans la formule générale (l) selon la revendication 1, en présence d'un agent de couplage, et éventuellement d'une base, dans un solvant.

13. Procédé de préparation d'un composé de formule (l) selon l'une quelconque des revendications 1 à 10, **caractérisé en ce que** l'on fait réagir un composé de formule
générale (VI) 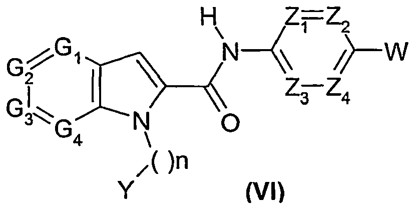 dans laquelle G₁, G₂, G₃ et G₄, Y, n, Z₁, Z₂, Z₃ et Z₄ sont tels que définis dans la formule générale (I) selon la revendication 1 et W représente un atome d'halogène, avec amine de formule Z-H, dans laquelle Z est tel que défini dans la formule générale (l) selon la revendication 1.

14. Procédé de préparation d'un composé de formule (l) selon l'une quelconque des revendications 1 à 10, **caractérisé en ce que** l'on fait réagir un composé de formule générale (X) 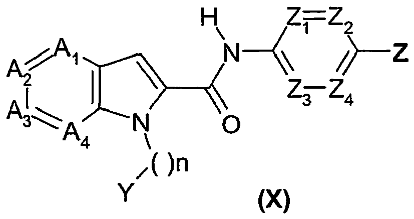 dans laquelle Y, n, Z, Z₁, Z₂, Z₃ et Z₄ sont tels que définis dans la formule générale (l) selon la revendication 1 et A₁, A₂, A₃, et A₄ représentent, indépendamment l'un de l'autre, un groupe C-X ou un atome d'azote ; X étant tel que défini dans la formule générale (l); l'un des A₁, A₂, A₃, A₄ et au maximum l'un des A₁, A₂, A₃, A₄ représente un groupe C-q où q est un atome d'halogène dans la position où le groupe silanyle doit être introduit,
avec un silane de formule SiH(X₁)(X₂)(X₃) dans laquelle X₁, X₂ et X₃ sont tels que définis dans la formule générale (l) selon la revendication 1,
en présence d'une quantité catalytique d'un complexe métallique et d'une base dans un solvant.

15. Composé (IVa), (IVb), (IVc), (lVd), (IVe) ou (IVf):

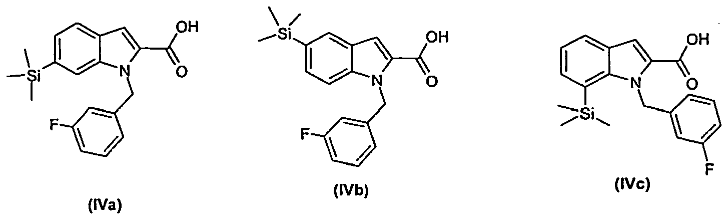

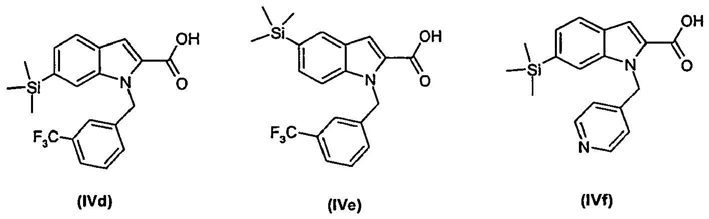

16. Médicament, **caractérisé en ce qu'**il comprend un composé de formule (I) selon l'une quelconque des revendications 1 à 10, ou un sel d'addition de ce composé à un acide pharmaceutiquement acceptable, ou encore un hydrate ou un solvate du composé de formule (I).

17. Composition pharmaceutique, **caractérisée en ce qu'**elle comprend un composé de formule (I) selon l'une quelconque des revendications 1 à 10, ou un sel pharmaceutiquement acceptable, un hydrate ou un solvate de ce composé, ainsi qu'au moins un excipient pharmaceutiquement acceptable.

18. Utilisation d'un composé de formule **(I)** selon l'une quelconque des revendications 1 à 10 pour la préparation d'un médicament destiné à la prévention et au traitement des pathologies dans lesquelles les récepteurs de type TRPV1 sont impliqués.

19. Utilisation d'un composé de formule (I) selon l'une quelconque des revendications 1 à 10 pour la préparation d'un médicament destiné à prévenir ou à traiter la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques, la dépression, les cancers.

## Claims

1. Compound corresponding to formula (I) 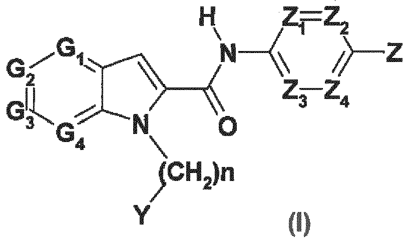 in which:
G₁, G₂, G₃ and G₄ are, independently of on another, a C-X group or a nitrogen atom;
X is chosen from a hydrogen atom, a halogen atom or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, cyano, C₁-C₆-thioalkyl, NR₁R₂, aryl-C₁-C₆-alkylene-O, aryl, heteroaryl or [EDIT 1]-Si(X₁)(X₂)(X₃) group; the C₁-C₆-alkyl,. C₃-C₇-cycloalkyl,-C₃-C₇-cycloalkyl-C₁-C₃-alkylene, C₁-C₆-fluoroalkyl, C₁-C₆-alkoxyl, C₃-C₇-cycloalkoxyl and C₃-C₇-cycloalkyl-C₁-C₆-alkylene-O-groups being optionally substituted with a hydroxyl, C₁-C₆-alkoxyl or NR₄R₅ group; the aryl the heteroaryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl nitro or cyano group;
one of G₁, G₂, G₃ and G₄ and at most one of G₁, G₂, G₃ and G₄ is a C-X group where X is an -Si (X₁) (X₂) (X₃) group; X₁, X₂ and X₃ are, independently of one another, a C₁-C₆-alkyl, C₃-C₇-cycloalkyl, C₃-C₇-cycloalkyl-C₁-C₆-alkyl, hydroxyl, aryl or heteroaryl group;
the aryl or heteroaryl groups of X₁, X₂, X₃ being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆₋fluoroalkyl, C₁-C₆-alkoxyl, C₁-C₆-fluoroalkoxyl, nitro or cyano group;
the C₁-C₆-alkyl, C₃-C₇-cycloalkyl an C₃-C₇-cycloalkyl-C₁-C₆-alkyl groups of X₁, X₂, X₃ being optionally substituted with one or more substituents chosen from a hydroxyl, C₁-C₆-alkoxyl, aryl-O-, NR₁R₂, aryl or heteroaryl group, the aryl and the Heteroaryl being optionally substituted with one or more substituents chosen from a halogen, and a C₁-C₆-alkyl, C₃-C₇-cycloalkyl, C₃-C₇-cycloalkyl-C₂-C₆-alkylene, C₁-C₆-fluoroalkyl, C₁-C₆-alkoxyl, C₁-C₆-fluoroalkoxyl, nitro or cyano group;
it also being possible for X₁ and X₂ to form, together with the silicon atom which bears them, a 4- to 7-membered ring optionally containing a heteroatom chosen from O, S and N;
n is equal to 0, 1, 2 or 3;
Y is an aryl or a heteroaryl; the aryl or the heteroaryl being optionally substituted with one or more substituents chosen from a halogen atom or a C₁-C₆-alkyl, C₃-C₇-cycloalkyl, C₃-C₇-cycloalkyl-C₁-C₆-alkylene, C₁-C₆-fluoroalkyl, hydroxyl, C₁-C₆-alkoxyl, C₃-C₇-cycloalkoxyl, C₃-C₇-cycloalkyl-C₁-C₆-alkylene-O-, C₁-C₆-fluoroalkoxyl, cyano, C₁-C₆-thioalkyl, thiol, -S(O)-C₁-C₆-alkyl, -S(O)₂-C₁-C₆-alkyl, C(O)NR₁R₂, SO₂NR₁R₂, SF₅, nitro, OCONR₁R₂, NR₃COR₄, NR₃CONR₃R₂, NR₁R₂, NR₃SO₂NR₁R₁₂, NR₃COR₄, NR₃SO₂R₅, aryl-C₁-C₆-alkylene or aryl group; the aryl and the aryl-C₁-C₆-alkylene being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano group;
Z₁, Z₂, Z₃, Z₄ are, independently of one another, a nitrogen atom or a C(R₆) group, at least one corresponding to a nitrogen atom and at least one corresponding to a C(R₆) group; the nitrogen atom or one of the nitrogen atoms present in the ring, defined as nitrogen of position 1, being optionally substituted with R₇ when the carbon atom at position 2 or 4 relative to this reference nitrogen is substituted with an oxo or thio group;
Z is
either a cyclic amine linked via the nitrogen atom, of formula: 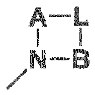 in which:
A is a C₁-C₇-alkylene group optionally substituted with one or two R₈ groups;
B is a C₁-C₇-alkylene group optionally substituted with one or two R₉ groups;
L is a bound, or a sulphur, oxygen or nitrogen atom; the nitrogen atom being optionally substituted with an R₁₀ or R₁₁;
the carbon atoms of the cyclic amine Z being optionally substituted with one or more R₁₂ groups which may be identical to or different from another;
or an acyclic amine, linked via the nitrogen atom, of formula NRaRb in which Ra and Rb are, independently of one another, a hydrogen atom or a C₁-C₆-alkyl, C₃-C₇-cycloalkyl, C₃-C₇-Cycloalkyl-C₁-C₆-alkylene, C₁-C₆-fluoroalkyl, hydroxyl, C₁-C₆-alkoxyl, C₃-C₇-cycloalkyl-C₁-C₆-alkylene-O-, C₁-C₆-fluoroalkoxyl, C₁-C₆-alkyl-C(O) -, HO-C(O)-C₁-C₆-alkylene, C₁-C₆-alkyl-O-C(O)-C₁-C₆-alkylene, aryl or heteroaryl group, it being possible for Ra and Rb to be optionally substituted with one or more Rc groups which may be identical to or different from one another;
Rc is a halogen atom, or a C₁-C₆-alkyl, C₃-C₇-cycloalkyl, C₃-C₇-cycloalkyl-C₁-C₉-alkylene, C₁-C₆-fluoroalkyl, C₁-C₆-alkoxyl, C₃-C₇-cycloalkyl-C₁-C₆-alkylene-O-, C₁-C₆-fluoroalkoxyl, C₁-C₆-thioalkyl, -S(O)-C₁-C₆-alkyl S(O)₂=C₁-C₆-alkyl, cyano, C(O) NR₁R₂, NR₁R₂, SO₂NR₁R₂, NR₃COR₄, NR₃SO₂R₅, OC(O)NR₁R₂, NR₃COOR₅, NR₃CONR₁R₂, NR₃SO₂NR₁R₂, hydroxyl, thiol, oxo, thio, aryl-C₁-C₆-alkylene, aryl or heteroaryl group, the aryl and the heteroaryl being optionally substituted with one or more substituents chosen from a halogen, and a C₁-C₆-alkyl., C₃-C₇-cycloalkyl, C₃-C₇-cycloalkyl-C₁-C₆-alkylene, C₁-C₆-fluoroal, C₁-C₆-alkoxyl, C₁-C₆-fluoroalkoxyl, nitro or cyano group;
an R₂ are, independently of one another, a hydrogen atom or a C₁-C₆-alkyl, C₃-C₇-cycloalkyl, C₃-C₇-cycloalkyl-C₁-C₆-alkylene, aryl-C₁-C₆-alkylene, aryl or Heteroaryl group, the aryl and the heteroaryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano group; or R₁ and R₂ form, together with the nitrogen atom which bears them, an azetidinyl, pyrrolidinyl, piperidinyl, azepinyl, morpholinyl, thiomorpholinyl, piperazinyl or homopiperazinyl group, ,this group being optionally substituted with a C₁-C₆-alkyl, C₃-C₇-cycloalkyl, C₃-C₇-cycloalkyl-C₁-C₆-alkylene, aryl-C₁-C₆-alkylene, aryl or heteroaryl group, the aryl and the heteroaryl being optionally substituted with one or more substituents chosen a halogen, an a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano group;
R₃ and R₄ are, independently of one another,
a hydrogen atom or a C₁-C₆-alkyl, C₃-C₇-cycloalkyl, C₃-C₇-cycloalkyl-C₁-C₆-alkylene, aryl-C₁-C₆-alkylene, aryl or heteroaryl group, the aryl and the heteroaryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano grou;
or R₃ and R₄ together form a (C₂-C₅) alkylene group;
or R₁ and R₃ together form a (C₂-C₅)alkylene group;
R₅ is a C₁-C₆-alkyl, C₃-C₇-cycloalkyl, C₃-C₇-cycloalkyl-C₁-C₆-alkylene, aryl-C₁-C₆-alkylene, aryl or heteroaryl group, the aryl and the heteroaryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano group; or R₃ and R₅ together form a (C₂-C₅) alkylene group;
R₆ is a hydrogen or halogen atom, or a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, C₁-C₆-thioalkyl, -S(O) -C₁-C₆-alkyl, -S (O)₂-C₁-C₆-alkyl, hydroxyl, thiol, oxo, thio, aryl, aryl-C₁-C₆-alkylene or heteroaryl group, the aryl and the heteroaryl being optionally substituted with one or more substituents chosen from a halogen, and a C₁-C₆₀-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano group;
R₇ is a hydrogen atom, or a C₁-C₆-alkyl, C₃-C₇-cycloalkyl, C₃-C₇-cycloalkyl-C₁₋C₆-alkylene,
C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, aryl, aryl-C₁-C₆-alkylene or heteroaryl group, the aryl and the heteroaryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₁-C₆-fluoroalkoxyl, nitro or cyano group;
R₈, R₉ and R₁₀ are defined such that:
two R₈ groups may together form a bond or a C₁-C₆-alkylene group;
two R₉ groups may together form a bond or a C₁-C₆-alkylene group;
R₈ and R₉ may together form a bond or a C₁-C₆-alkylene group;
R₈ and R₁₀ may together form a bond or a C₁-C₆-alkylene group;
R₉ and R₁₀ may together form a bond or a C₁-C₆-alkylene group;
R₁₁ is a hydrogen atom, or a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, hydroxyl, C₁-C₆-alkyl-CO-, COOR₅, C(O)NR₁R₂, aryl-C₁-C₆-alkylene, aryl or heteroaryl group, the aryl and the heteroaryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₃-C₇-cycloalkoxyl, C₁-C₆-fluoroalkoxyl, nitro or cyano group;
R₁₂ is a fluorine atom, a C₁-C₆-alkyl group optionally substituted with an R₁₃ group; C₃-C₇-cycloalkyl, C₃-C₇-cycloalkyl-C₁-C₆-alkylene, C₁C₆-fluoroalkyl, C₁-C₆ cycloalk-1,1-diyl, C₃-C₇-heterocycloalk-1,1-diyl optionally substituted on a nitrogen atom with an R₁₁ group; C₁-C₆-alkoxyl, C₃-C₇-cycloalkoxyl, C₃-C₇-cycloalkyl-C₁-C₆-alkylene-O-, C₁-C₆-fluoroalkoxyl, CO₂H, C(O)O-C₁-C₆-alkyl, C(O)NR₁R₂, NR₁R₂, NR₃COR₄, OC(O)NR₁R₂, NR₃COOR₅, NR₃CONR₃R₂, hydroxyl, thiol, oxo, thio, aryl-C₁-C₆-alkylene or aryl; the aryl being optionally substituted with one or more substituents chosen from a halogen, and a C₁-C₆-alkyl, C₃-C₇-cycloalkyl, C₃-C₇-cycloalkyl-C₁-C₆-alkylene, C₁-C₆-fluoroalkyl, C₁-C₆-alkoxyl, C₃-C₇-cycloalkoxyl, C₁-C₆-fluoroalkoxyl, nitro, or cyano group;
R₁₃ is a C₁-C₆-alkoxyl, C₃-C₇-cycloalkoxyl, C₃-C₇-cycloalkyl-C₁-C₆-alkylene-O-, C(O)NR₁R₂, NR₁R₂, NR₃COR₄, OC(O)NR₁R₂, NR₃COOR₅ or hydroxyl group;
it being possible for the nitrogen atom(s) of the compounds of general formula (I) to be in oxidized form;
in the form of a base or of an addiction salt with an acid, and also in the form of a hydrate or of a solvate.

2. Compound of formula (I) according to Claim 1, **characterized in that**
one of G₁. G₂, G₃ and G₄ is a C-X group where X is an - Si (X₁) (X₂) (X₃) group; the others of G₁, G₂, G₃ and G₄ being a CH group;
X₁, X₂ and X₃ are, independently of one another, a C₁-C₆-alkyl, C₃-C₇-cycloalkyl or aryl group; in the form of a base or of an addition salt with an acid, and also in the form of a hydrate or of a solvate.

3. Compound of formula (I) according to Claim 1 or 2, **characterized in that** n is equal to 1; in the form of a base or of an audition salt with an acid, and also in the form of a hydrate or of a solvate.

4. Compound of formula (I) according to any one of Claims 1 to 3, **characterized in that** Y is an aryl or a heteroaryl; the aryl or the heteroaryl being optionally substituted with one or more groups chosen from a halogen atom or a C₁-C₆-fluoroalkyl group; in the form of a base or of an audition salt with an acid, and also in the form of a hydrate or of a solvate.

5. Compound of formula (I) according to any one of Claims 1 to 4, **characterized in that** Z₁, Z₂, Z₃ and Z₄ are, independently of on another, a nitrogen atom or a C(R₆) group, one corresponding to a nitrogen atom and the others corresponding to a C(R₆) group; R₆ is a hydrogen atom;
in the form of a base or of an addition salt with an acid, and also in the form of a hydrate or of a solvate.

6. Compound of formula (I) according to any one of Claims 1 to 5, **characterized in that**
Z is a cyclic amine linked via the nitrogen atom, of formula: 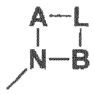 in which:
A is a C₁-C₇-alkylene group optionally substituted with one or two R₈ groups;
B is a C₁-C₇-alkylene group optionally substituted with one or two R₉ groups;
L is a bond, or a sulphur, oxygen or nitrogen atom; the nitrogen atom being optionally substituted with an R₁₀ or R₁₁ group;
the carbon atoms of the cyclic amine Z being optionally substituted with or more R₁₂ groups which may be identical to or different from one another;
R₈, R₉, R₁₀ R₁₁ and R₁₂ being as defined in general formula (I) according to Claim 1;
in the form of a base or of an audition salt with an acid, and also in the form of a hydrate or of a solvate.

7. Compound of formula (I) according to any one of
Claims 1 to 6, **characterized in that** Z is a cyclic amine linked via the nitrogen atom, of formula: 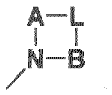 in which:
A is a C₁-C₃-alkylene group;
B is a C₁-C₃-alkylene group;
L is a bond;
the carbon atoms of the cyclic amine Z being optionally substituted with one or more R₁₂ groups which may be identical to or different from one another;
R₁₂ is a hydroxyl group;
in the form of a base or of an addition salt with an acid, and also in the form of a hydrate or of a solvate.

8. Compound of formula (I) according to any one of Claims 1 to 5, **characterized in that** Z is an acyclic amine, linked via the nitrogen atom, of formula NRaRb in which Ra and Rb are, independently of one another, a hydrogen atom or a C₁-C₆-alkyl group; in the form of a base or of an addition salt with an acid, and also in the form of a hydrate or of a solvate.

9. Compound of formula (I) according to any one of Claims 1 to 8, **characterized in that**
one of G₁, G₂, G₃ and G₄ is a C-X group where X is an Si(X₁)(X₂) (X₃) group; the others of G₁, G₂, G₃ and G₄ being a CH group;
X₁, X₂ and X₃ are, independently of one another, a C₁-C₆-alkyl, C₃-C₇-cycloalkyl or aryl group;
n is equal to 1;
Y is an aryl or a heteroaryl; the aryl or the Heteroaryl being optionally substituted with one or more groups chosen from a halogen atom or a C₁-C₆-fluoroalkyl group;
Z₁, Z₂, Z₃ and Z₄ are, independently of one another, a nitrogen atom or a C(R₆) group, one corresponding to a nitrogen atom and the others corresponding to a C(R₆) group; R₆ is a hydrogen atom;
Z is:
either a cyclic amine linked via the nitrogen atom, of formula: 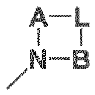
in which:
A is a C₁-C₃-alkylene group;
B is a C₁-C₃-alkylene group;
L is a bond;
the carbon atoms of the cyclic amine Z being optionally substituted with one or more R₁₂ groups which may be identical to or different from one another;
R₁₂ is a hydroxyle group;
or an acyclic amine, linked via the nitrogen atom, of formula NRaRb in which Ra and Rb are, independently of one another, a hydrogen atom or a C₁-C₆-alkyl group;
in the form of a base or of an addition salt with an acid, and also in the form of a hydrate or of a solvate.

10. Compound of formula (I) according to Claim 1, chosen from:
*N*-[6-(azetidin-1-yl)pyridin-3-yl]-6-trimethylsilyl-1-[(3-fluorophenyl)methyl]-1*H*-indole-2-carboxamide
*N*-[6-(3-hydroxyazetidin-1-yl)pyridin-3-yl]-6-trimethylsilyl-1-[(3-fluorophenyl)methyl]-1*H*-indole-2-carboxamide
*N*-[6-(azetidin-1-yl)pyridin-3-yl]-5-trimethylsilyl-1-[(3-fluorophenyl)methyl]-1*H*-indole-2-carboxamide *N*-[6-(3-hydroxyazetidin-1-yl)pyridin-3-yl]-5-trimethylsilyl-1-[(3-fluorophenyl)methyl]-1*H*-indole-2-carboxamide
*N*-[6-(azetidin-1-yl)pyridin-3-yl]-7-trimethylsilyl-1-[(3-fluorophenyl)methyl]-1H-indole-2-carboxamide
*N*-[6-(3-hydroxyazetidin-1-yl)pyridin-3-yl]-7-trimethylsilyl-1-[(3-fluorophenyl)methyl]-1*H*-indole-2-carboxamide
N-[6-(Dimethylamino)pyridin-3-yl]-5-trimethylsilyl-1-[(3-fluorophenyl)methyl]-1*H*-indole-2-carboxamide
*N*-[6-(azetidin-1-yl)pyridin-3-yl]-5-(ethyl)dimethylsilyl-1-[(3-fluorophenyl)methyl]1*H-*indole-2-carboxamide
*N*-[6-(azetidin-1-yl)pyridin-3-yl]-5-(cyclohexyl)dimethylsilyl-1-[(3-fluorophenyl)methyl]-1*H*-indole-2-carboxamide
1*N*-[6-(azetidin-1-yl)pyridin-3-yl]-6-trimethylsilyl-1-[[(3-trifluoromethyl)-phenyl]methyl]-1*H*-indole-2-carboxamide
*N*-[6-(3-hydroxyazetidin-1-yl)pyridin-3-yl]-6-trimethylsilyl-1-[[(3-trifluoromethyl)phenyl]methyl]-1*H*-indole-2-carboxamide
*N*-[6-(azetidin-1-yl)pyridin-3-yl]-5-(diphenyl)methylsilyl-1-[(3-fluorophenyl)methyl]-1*H-*indole-2-carboxamide
*N*-[6-(azetidin-1-yl)pyridin-3-yl]-5-trimethylsilyl-1-[[(3-trifluoromethyl)phenyl]methyl]-1*H*-indole-2-carboxamide
*N*-[6-(3-hydroxyazetidin-1-yl)pyridin-3-yl]-5-trimethylsilyl-1-[[(3-trifluoromethyl)phenyl]methyl]-1*H*-indole-2-carboxamide
*N*-[6-(3-hydroxyazetidin-1-yl)pyridin-3-yl]-6-trimethylsilyl-1-[(pyridin-4-yl)methyl]-1*H*-indole-2-carboxamide
*N*-[6-(azetidin-1-yl)pyridin-3-yl)pyridin-3-yl]-6-trimethylsilyl-1-[(pyridin-4-yl)methyl]-1*H*-indole-2-carboxamide
*N*-[6-(azetidin-1-yl)pyridin-3-yl]-5-triethylsilyl-1-[(3-fluorophenyl)methyl]-1*H*-indole-2-carboxamide
*N*-[6-(azetidin-1-yl)pyridin-3-yl]-5-tert-butyl(dimethyl)silyl-1-[(3-fluorophenyl)methyl]-1*H-*indole-2-carboxamide
*N*-[6-(Pyrrolidin-1-yl)pyridin-3-yl]-5-(cyclohexyl)dimethylsilyl-1-[(3-fluorophenyl)methyl]-1*H*-indole-2-carboxamide
*N*-[6-(azetidin-1-yl)pyridin-3-yl]-5-phenyldimethylsilyl-1-[(3-fluorophenyl)methyl]-1*H-*indole-2-carboxamide
*N*-[6-(azetidin-1-yl)pyridin-3-yl]-5-(isopropyl)dimethylsilyl-1-[(3-fluorophenyl)methyl]-1*H-*indole-2-carboxamide
*N*-[6-(Azetidin-1-yl)pyridin-3-yl]-5-(methyl)diethylsilyl-1-[(3-fluorophenyl)methyl]-1*H-*indole-2-carboxamide.

11. Process for preparing a compound of formula (I) according to any one of Claims 1 to 10, **characterized in that** a compound of general formula (IV) 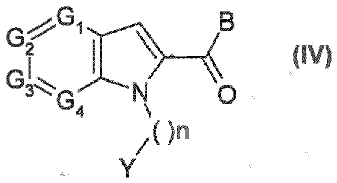 in which G₁, G₂, G₃ and G₄, Y and n are as defined in general formula (I) according to Claim 1 and B is a chlorine atom,
is reacted with an amine of general formula (V) 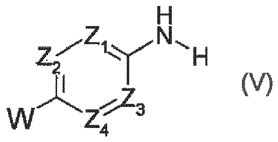 in which W = Z and Z, Z₁, Z₂, Z₃ and Z₄ are as defined in general formula (I) according to Claim 1, in a solvent.

12. Process for preparing a compound of formula (I) according to any one of Claims 1 to 10,
**characterized in that** a compound of general formula (IV) 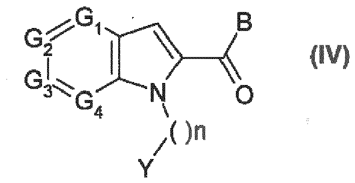 in which G₁, G₂, G₃ and G₄, Y and n are as defined in general formula (I) according to Claim 1 and B is a hydroxyl group,
is reacted with an amine of general formula (V) 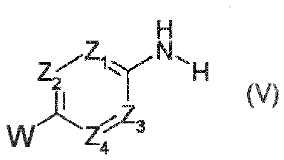 in which W = Z and Z, Z₁, Z₂, Z₃ and Z₄ are as defined in general formula (I) according to Claim 1, in the presence of a coupling agent, and optionally of a base, in a solvent.

13. Process for preparing a compound of formula (I) according to any one of Claims 1 to 10, **characterized in that** a compound of general formula (VI) 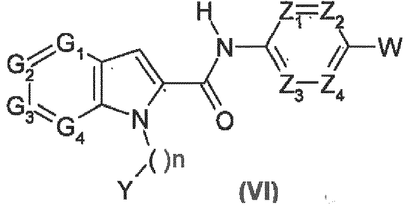 in which G₁, G₂, G₃ and G₄, Y, n, Z₁, Z₂, Z₃ and Z₄ are as defined in general formula (I) according to Claim 1 and W is a halogen atom,
is reacted with an amine of formula Z-H, in which Z is as defined in general formula (I) according to Claim 1.

14. Process for preparing a compound of formula (I) according to any one of Claims 1 to 10, **characterized in that** a compound of general formula (X) 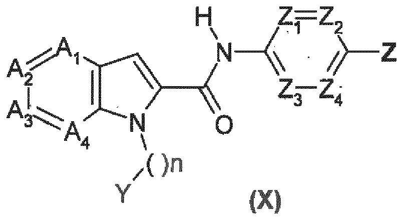 in which Y, n, Z, Z₁, Z₂, Z₃ and Z₄ are as defined in general formula (I) according to Claim 1 and A₁, A₂, A₃ and A₄ are, independently of one another, a C-X group or a nitrogen atom; X being as defined in general formula (I); one of A₁, A₂, A₃ and A₄ and at most one of A₁, A₂, A₃ and A₄ is a C-q group where q is a halogen atom in the position where the silanyl group should be introduced,
is reacted with a silane of formula SiH(X₁) (X₂) (X₃) in which X₁, X₂ and X₃ are as defined in general formula (I) according to Claim 1,
in the presence of a catalytic amount of a metal complex and of a base in a solvent.

15. Compound (IVa), (IVb), (IVc), (IVd), (IVe) or (IVf):

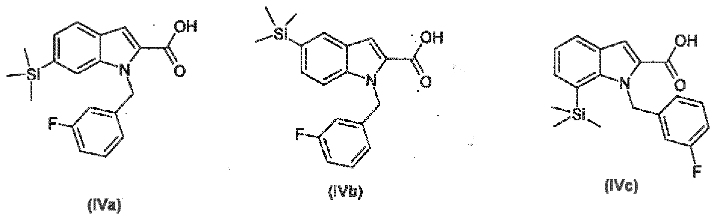

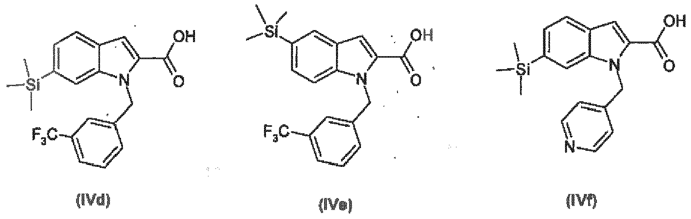

16. Medicament, **characterized in that** it comprises a compound of formula (I) according to any one of Claims 1 to 10, or an addition salt of this compound with a pharmaceutically acceptable acid, or else a hydrate or a solvate of the compound of formula (I).

17. Pharmaceutical composition, **characterized in that** it comprises a compound of formula (I) according to any one of Claims 1 to 10, or a pharmaceutically acceptable salt, a hydrate or a solvate of this compound, and also at least one pharmaceutically acceptable excipient.

18. Use of a compound of formula (I) according to any one of Claims 1 to 10, for the preparation of a medicament for preventing and treating pathologies in which TRPV1-type receptors are involved.

19. Use of a compound of formula (I) according to any one of Claims 1 to 10, for the preparation of a medicament for preventing or treating pain, inflammation, metabolic disorders, urological disorders, gynaecological disorders, gastrointestinal disorders, respiratory disorders, psoriasis, pruritus, dermal, ocular or mucosal irritations, herpes, shingles, multiple sclerosis, depression or cancers.

## Patentansprüche

1. Verbindung der Formel (I) 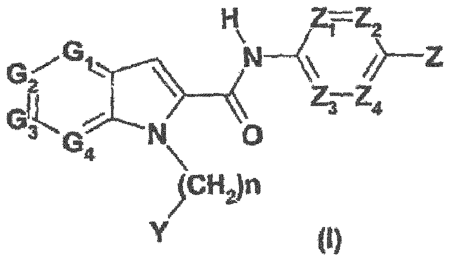 worin
G₁, G₂, G₃ und G₄ unabhängig voneinander für eine Gruppe C-X oder ein Stickstoffatom stehen;
X aus einem Wasserstoffatom, einem Halogenatom oder einer C₁-C₆-Alkyl- C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Cyano-, C₁-C₆-Thioalkyl-, NR₁R₂-, Aryl-C₁-C₆-alkylen-O-, Aryl-, Heteroaryl- oder -Si(X₁)(X₂) (X₃) -Gruppe ausgewählt ist; wobei die C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy- und C₃-C₇-Cycloalkyl-C₁-C₆-alkylen-O-Gruppen gegebenenfalls durch eine Hydroxy-, C₁-C₆-Alkoxy- oder NR₄R₅-Gruppe substituiert sind und das Aryl und das Heteroaryl gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert ist;
eine der Gruppen G₂, G₂, G₃ und G₄ und höchstens eine der Gruppen G₁, G₂, G₃ und G₄ für eine Gruppe C-X, wobei X eine -Si (X₁) (X₂) (X₃) -Gruppe bedeutet, steht;
X₁, X₂ und X₃ unabhängig voneinander für eine C₁-C₆-Alkyl-, C₃-C₇-Cycloalkyl-, C₃-C₇-Cycloalkyl-C₁-C₆-alkyl-, Hydroxy-, Aryl- oder Heteroarylgruppe stehen;
wobei die Aryl- oder Heteroarylgruppen von X₁, X₂ und X₃ gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₂-C₆-Alkoxy-, C₁-C₆-Fluoralkoxy-, Nitro- oder Cyanogruppe ausgewählte Substituenten substituiert sind;
wobei die C₁-C₆-Alkyl-, C₃-C₇-Cycloalkyl- und C₃-C₇-Cycloalkyl-C₁-C₆-alkylgruppen von X₁, X₂ und X₃ gegebenenfalls durch einen oder mehrere unter einer Hydroxy-, C₁-C₆-Alkoxy-, Aryl-O-, NR₁R₂-, Aryl- oder Heteroarylgruppe ausgewählte Substituenten substituiert sind, wobei das Aryl und das Heteroaryl gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
wobei X₁ und X₂ auch zusammen mit dem Siliciumatom, das sie trägt, einen 4- bis 7-gliedrigen Ring, der gegebenenfalls ein aus O, S und N ausgewähltes Heteroatom enthält, bilden, können;
n gleich 0, 1, 2 oder 3 ist;
Y für ein Aryl oder ein Heteroaryl steht; wobei das Aryl und das Heteroaryl gegebenenfalls durch einen oder mehrere unter einem Halogenatom oder einer C₁-C₆-Alkyl-, C₃-C-₇-Cycloalkyl-, C₃-C₇-Cycloalkyl-C₁-C₆-alkylen-, C₁-C₆-Fluoralkyl-, Hydroxy-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Cyano-, C₁-C₆-Thioalkyl-, Thiol-, -S (O) -C₁-C₆-Alkyl-, -S(O)₂-C₁-C₆-Alkyl-, C (O) NR₁R₂-, SO₂NR₁R₂-, SF₅ -, Nitro-, OCONR₁R₂ -, NR₃COR₄-, NR₃CONR₁R₂-, NR₁R₂-, NR₃SO₂NR₁R₂-, NR₃COR₄-, NR₃SO₂R₅-, Aryl-C₁-C₆-alkylen- oder Arylgruppe ausgewählte Substituenten substituiert sind; wobei das Aryl und das Aryl-C₁-C₆-alkylen gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluor-alkoxy-, Nitro- oder Cyanogruppe ausgewählte Substituenten substituiert sind;
Z₁, Z₂, Z₃ und Z₄ unabhängig voneinander für ein Stickstoffatom oder eine Gruppe C(R₆) stehen, wobei mindestens eine dieser Gruppen einem Stickstoffatom entspricht und mindestens eine dieser Gruppen einer Gruppe C(R₆) entspricht; wobei das Stickstoffatom oder eines der Stickstoffatome in dem Ring, der als Stickstoff in 1-Position definiert ist, gegebenenfalls durch R₇ substituiert ist, wenn das Kohlenstoffatome in 2- oder 4-Position zu diesem Referenz-Stickstoff durch eine Oxo- oder Thiogruppe substituiert ist;
Z für
ein über das Stickstoffatom gebundenes cyclisches Amin der Formel: 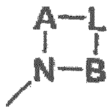 worin
A für eine C₁-C₇-Alkylengruppe, die gegebenenfalls durch eine oder zwei Gruppen R₈ substituiert ist, steht;
B für eine C₁-C₇-Alkylengruppe, die gegebenenfalls durch eine oder zwei Gruppen R₉ substituiert ist, steht;
L für eine Bindung oder ein Schwefel-, Sauerstoff- oder Stickstoffatom steht; wobei das Stickstoffatom gegebenenfalls durch eine Gruppe R₁₀ oder R₁₁ substituiert ist;
wobei die Kohlenstoffatome des cyclischen Amins Z gegebenenfalls durch eine oder mehrere gleiche oder voneinander verschiedene Gruppen R₁₂ substituiert sind;
oder ein über das Stickstoffatom gebundenes acyclisches Amin der Formel NRaRb, worin Ra und Rb unabhängig voneinander für ein Wasserstoffatom oder eine C₁-C₆-Alkyl-, C₃-C₇-Cycloalkyl-, C₃-C₇-Cycloalkyl-C₁-C₆-alkylen-, C₁-C₆-Fluoralkyl-Hydroxy-, C₁-C₆-Alkoxy-, C₃-C₇-Cycloalkyl-C₁-C₆-alkylen-O-, C₁-C₆-Fluoralkoxy-, C₁-C₆-Alkyl-C(O)-, HO-C(O) -C₁-C₆-Alkylen-, C₁-C₆-Alkyl-O-C (O) -C₁-C₆-alkylen-, Aryl- oder Heteroarylgruppe stehen, wobei Ra und Rb gegebenenfalls durch eine oder mehrere gleiche oder voneinander verschiedene Gruppen Rc substituiert sein können;
Rc für ein Halogenatom oder eine C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₃-C₇-Cycloalkyl-C₁-C₆-alkylen-O-, C₁-C₆-Fluoralkoxy-, C₁-C₆-Thio-alkyl-, -S (O) -C₁-C₆-Alkyl- -S (O)₂C₁-C₆-Alkyl-Cyano-, C(O)NR₁R₂-, NR₁R₂-, SO₂NR₁R₂-, NR₃COR₄-, NR₃SO₂R₅-, OC(O)NR₁R₂-, NR₃COOR₅-, NR₃CONR₁R₂-, NR₃SO₂NR₁R₂- Hydroxy-, Thiol-, Oxo-, Thio-, Aryl-C₁-C₆-alkylen-, Aryl- oder Heteroarylgruppe steht, wobei das Aryl und das Heteroaryl gegebenenfalls durch einen oder mehrere unter einem Halogen oder einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
steht;
R₁ und R₂ unabhängig voneinander für ein Wasserstoffatom oder eine C₁-C₆-Alkyl-, C₃-C₇-Cycloalkyl-, C₃-C₇-Cycloalkyl-C₁-C₆-alkylen-, Aryl-C₁-C₆-alkylen-, Aryl- oder Heteroarylgruppe stehen, wobei das Aryl und das Heteroaryl gegebenenfalls durch einen oder mehrere unter einem Halogen oder einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind; oder R₁ und R₂ zusammen mit dem Stickstoffatom, das sie trägt, eine Azetidinyl-, Pyrrolidinyl-, Piperidinyl-, Azepinyl-, Morpholinyl-, Thiomorpholinyl-, Piperazinyl- oder Homopiperazinylgruppe bilden, wobei diese Gruppe gegebenenfalls durch eine C₁-C₆-Alkyl-, C₃-C₇-Cycloalkyl-, C₃-C₇-Cycloalkyl-C₁-C₆-alkylen-, Aryl-C₁-C₆-alkylen-, Aryl- oder Heteroarylgruppe substituiert ist, wobei das Aryl und das Heteroaryl gegebenenfalls durch einen oder mehrere unter einem Halogen oder einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
R₃ und R₄ unabhängig voneinander für ein Wasserstoffatom oder eine C₁-C₆-Alkyl , C₃-C-₇-Cycloalkyl-, C₃-C₇-Cycloalkyl-C₁-C₆-alkylen-, Aryl-C₁-C₆-alkylen-, Aryl- oder Heteroarylgruppe stehen, wobei das Aryl und das Heteroaryl gegebenenfalls durch einen oder mehrere unter einem Halogen oder einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
oder R₃ und R₄ zusammen eine (C₂-C₅)-Alkylengruppe bilden;
oder R₁ und R₃ zusammen eine (C₂-C₅)-Alkylengruppe bilden;
R₅ für eine C₁-C₆-Alkyl-, C₃-C₇-Cycloalkyl-, C₃-C₇-Cycloalkyl-C₁-C₆-alkylen-, Aryl-C₁-C₆-alkylen-, Aryl- oder Heteroarylgruppe steht, wobei das Aryl und das Heteroaryl gegebenenfalls durch einen oder mehrere unter einem Halogen oder einer C₁-C₆-Alkyl-, C₃-C₇-Cycloalkyl-, C₃-C₇-Cycloalkyl-C₁-C₆-akylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
oder R₃ und R₅ zusammen eine (C₂-C₅)-Alkylengruppe bilden;
R₆ für ein Wasserstoff- oder Halogenatom oder eine C₁-C₆-Alkyl-, C₃-C₇-Cycloalkyl-, C₃-C-₇-Cycloalkyl-C₁-C₆-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, C₁-C₆-Thioalkyl-, -S (O) -C₁-C₆-Alkyl-, -S(O)₂-C₁-C₆-Alkyl-, Hydroxy-, Thiol-, Oxo-, Thio-, Aryl-, Aryl-C₁-C₆-alkylen- oder Heteroarylgruppe steht, wobei das Aryl und das Heteroaryl gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
R₇ für ein Wasserstoffatom oder eine C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₃-C₇-Cyclo-alkoxy-, C₃-C₇-Cycloalkyl-C₁-C₆-alkylen-O-, C₁-C₆-Fluoralkoxy-, Aryl-, Aryl-C₁-C₆-alkylen- oder Heteroarylgruppe steht, wobei das Aryl und das Heteroaryl gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₁-C₆-Fluoralkoxy-, Nitro- oder Cyanogruppe ausgewählte Substituenten substituiert sind;
R₈, R₉ und R₁₀ so definiert sind, dass:
zwei Gruppen R₈ zusammen eine Bindung oder eine C₁-C₆-Alkylengruppe bilden können;
zwei Gruppen R₉ zusammen eine Bindung oder eine C₁-C₆-Alkylengruppe bilden können;
R₈ und R₉ zusammen eine Bindung oder eine C₁-C₆-Alkylengruppe bilden können;
R₈ und R₁₀ zusammen eine Bindung oder eine C₁-C₆-Alkylengruppe bilden können;
R₉ und R₁₀ zusammen eine Bindung oder eine C₁-C₆-Alkylengruppe bilden können;
R₁₁ für ein Wasserstoffatom oder eine C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₃-C₇-Cyclo-alkoxy-, C₃-C₇-Cycloalkyl-C₁-C₆-alkylen-O-, C₁-C₆-Fluoralkoxy-, Hydroxy-, C₁-C₆-Alkyl-CO-, COOR₅-, C(O)NR₁R₂-, Aryl-C₁-C₆-alkylen-, Aryl- oder Heteroarylgruppe steht, wobei das Aryl und das Heteroaryl gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₃-C₇-Cycloalkoxy, C₁-C₆-Fluoralkoxy-, Nitro- oder Cyanogruppe ausgewählte Substituenten substituiert sind;
R₁₂ für ein Fluoratom, eine C₁-C₆-Alkylgruppe, die gegebenenfalls durch eine Gruppe R₁₃ substituiert ist; C₃-C₇-Cycloalkyl, C₃-C₇-Cycloalkyl-C₁-C₆-alkylen, C₁-C₆-Fluoralkyl, C₁-C₆-Cycloalk-1,1-diyl, C₃-C₇-Heterocycloalk-1,1-diyl, das gegebenenfalls an einem Stickstoffatom durch eine Gruppe R₁₁ substituiert ist; C₁-C₆-Alkoxy, C₃-C₇-Cycloalkoxy, C₃-C₇-Cycloalkyl-C₁-C₆-alkylen-O-, C₁-C₆-Fluor-alkoxy, CO₂H, C(O)O-C₁-C₆-Alkyl, C(O)NR₁R₂, NR₁R₂, NR₃COR₄, OC(O)NR₁R_{2,} NR₃COOR₅, NR₃CONR₁R₂, Hydroxyl, Thiol, Oxo, Thio, Aryl-C₁-C₆-alkylen oder Aryl steht; wobei das Aryl gegebenenfalls durch einen oder mehrere unter einem Halogen und einer C₁-C₆-Alkyl-, C₃-C₇-Cycloalkyl-, C₃-C₇-Cycloalkyl-C₁-C₆-alkylen-, C₁-C₆-Fluoralkyl-, C₁-C₆-Alkoxy-, C₃-C₇-Cycloalkoxy, C₁-C₆-Fluoralkoxy-, Nitro- oder Cyanogruppe ausgewählte Substituenten substituiert ist; R₁₃ für eine C₁-C₆-Alkoxy-, C₃-C₇-Cycloalkoxy-, C₃-C₇-Cycloalkyl-C₁-C₆-alkylen-O-, C(O)NR₁R₂-, NR₁R₂-, NR₃COR₄- , OC (O) NR₁R₂- , NR₃COOR₅- oder Hydroxygruppe steht;
wobei das Stickstoffatom bzw. die Stickstoffatome der Verbindungen der allgemeinen Formel (I) in oxidierter Form vorliegen kann bzw. können;
in Basen- oder Säureadditionssalzform sowie in Hydrat- oder Solvatform.

2. Verbindung der Formel (I) nach Anspruch 1, **dadurch gekennzeichnet, dass**
eine der Gruppen G₁, G₂, G₃ und G₄ für eine Gruppe C-X, wobei X eine -Si (X₁) (X₂) (X₃) -Gruppe bedeutet, steht; die anderen Gruppen G₁, G₂, G₃ und G₄ für eine CH-Gruppe stehen;
X₁, X₂ und X₃ unabhängig voneinander für eine C₁-C₆-Alkyl-, C₃-C₇-Cycloalkyl- oder Arylgruppe stehen; in Basen- oder Säureadditionssalzform sowie in Hydrat- oder Solvatform.

3. Verbindung der Formel (I) nach Anspruch 1 oder 2, **dadurch gekennzeichnet, dass** n gleich 1 ist; in Basen- oder Säureadditionssalzform sowie in Hydrat- oder Solvatform.

4. Verbindung der Formel (I) nach einem der Ansprüche 1 bis 3, **dadurch gekennzeichnet, dass** Y für ein Aryl oder ein Heteroaryl steht; wobei das Aryl und das Heteroaryl gegebenenfalls durch eine oder mehrere unter einem Halogenatom oder einer C₁-C₆-Fluoralkylgruppe ausgewählte Gruppen substituiert sind; in Basen- oder Säureadditionssalzform sowie in Hydrat- oder Solvatform.

5. Verbindung der Formel (I) nach einem der Ansprüche 1 bis 4, **dadurch gekennzeichnet, dass** Z₁, Z₂, Z₃ und Z₄ unabhängig voneinander für ein Stickstoffatom oder eine Gruppe C(R₆) stehen, wobei eine dieser Gruppen einem Stickstoffatom entspricht und die anderen einer Gruppe C(R₆) entsprechen; R₆ für ein Wasserstoffatom steht;
in Basen- oder Säureadditionssalzform sowie in Hydrat- oder Solvatform.

6. Verbindung der Formel (I) nach einem der Ansprüche 1 bis 5, **dadurch gekennzeichnet, dass** Z für ein über das Stickstoffatom gebundenes cyclisches Amin der Formel: 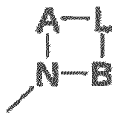 worin
A für eine C₁-C₇-Alkylengruppe, die gegebenenfalls durch eine oder zwei Gruppen R₈ substituiert ist, steht;
B für eine C₁-C₇-Alkylengruppe, die gegebenenfalls durch eine oder zwei Gruppen R₉ substituiert ist, steht;
L für eine Bindung oder ein Schwefel-, Sauerstoff- oder Stickstoffatom steht; wobei das Stickstoffatom gegebenenfalls durch eine Gruppe R₁₀ oder R₁₁ substituiert ist;
wobei die Kohlenstoffatome des cyclischen Amins Z gegebenenfalls durch eine oder mehrere gleiche oder voneinander verschiedene Gruppen R₁₂ substituiert sind;
steht;
wobei R₈, R₉, R₁₀, R₁₁ und R₁₂ wie in der allgemeinen Formel (I) gemäß Anspruch 1 definiert sind;
in Basen- oder Säureadditionssalzform sowie in Hydrat- oder Solvatform.

7. Verbindung der Formel (I) nach einem der Ansprüche 1 bis 6, **dadurch gekennzeichnet, dass** Z für ein über das Stickstoffatom gebundenes cyclisches Amin der Formel: 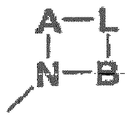 worin
A für eine C₁-C₃-Alkylengruppe steht;
B für eine C₁-C₃-Alkylengruppe steht;
L für eine Bindung steht;
wobei die Kohlenstoffatome des cyclischen Amins Z gegebenenfalls durch eine oder mehrere gleiche oder voneinander verschiedene Gruppen R₁₂ substituiert sind;
R₁₂ für eine Hydroxygruppe steht;
steht;
in Basen- oder Säureadditionssalzform sowie in Hydrat- oder Solvatform.

8. Verbindung der Formel (I) nach einem der Ansprüche 1 bis 5, **dadurch gekennzeichnet, dass** Z für ein über das Stickstoffatom gebundenes acyclisches Amin der Formel NRaRb, worin Ra und Rb unabhängig voneinander für ein Wasserstoffatom oder eine C₁-C₆-Alkylgruppe stehen, steht; in Basen- oder Säureadditionssalzform sowie in Hydrat- oder Solvatform.

9. Verbindung der Formel (I) nach einem der Ansprüche 1 bis 8, **dadurch gekennzeichnet, dass**
eine der Gruppen G₁, G₂, G₃ und G₄ für eine Gruppe C-X, wobei X eine -Si (X₁) (X₂) (X₃) -Gruppe bedeutet, steht; die anderen Gruppen G₁, G₂, G₃ und G₄ für eine CH-Gruppe stehen;
X₁, X₂ und X₃ unabhängig voneinander für eine C₁-C₆-Alkyl-, C₃-C₇-Cycloalkyl- oder Arylgruppe stehen;
n gleich 1 ist;
Y für ein Aryl oder ein Heteroaryl steht; wobei das Aryl und das Heteroaryl gegebenenfalls durch eine oder mehrere unter einem Halogenatom oder einer C₁-C₆-Fluoralkylgruppe ausgewählte Gruppen substituiert sind;
Z₁, Z₂, Z₃ und Z₄ unabhängig voneinander für ein Stickstoffatom oder eine Gruppe C(R₆) stehen, wobei eine dieser Gruppen einem Stickstoffatom entspricht und die anderen einer Gruppe C(R₆) entsprechen; R₆ für ein Wasserstoffatom steht;
Z für ein über das Stickstoffatom gebundenes cyclisches Amin der Formel: 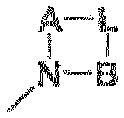 worin
A für eine C₁-C₃-Alkylengruppe steht;
B für eine C₁-C₃-Alkylengruppe steht;
L für eine Bindung steht;
wobei die Kohlenstoffatome des cyclischen Amins Z gegebenenfalls durch eine oder mehrere gleiche oder voneinander verschiedene Gruppen R₁₂ substituiert sind;
R₁₂ für eine Hydroxygruppe steht;
oder ein über das Stickstoffatom gebundenes acyclisches Amin der Formel NRaRb, worin Ra und Rb unabhängig voneinander für ein Wasserstoffatom oder eine C₁-C₆-Alkylgruppe stehen,
steht;
in Basen- oder Säureadditionssalzform sowie in Hydrat- oder Solvatform.

10. Verbindung der Formel (I) nach Anspruch 1, ausgewählt aus:
*N*-[6-(Azetidin-1-yl)pyridin-3-yl]-6-trimethylsilyl-1-[(3-fluorphenyl)methyl]-1*H*-indol-2-carboxamid;
*N*-[6-(3-Hydroxyazetidin-1-yl)pyridin-3-yl]-6-trimethylsilyl-1-[(3-fluorphenyl)methyl]-1*H*-indol-2-carboxamid;
*N*-[6-(Azetidin-1-yl)pyridin-3-yl]-5-trimethylsilyl-1-[(3-fluorphenyl)methyl]-1*H*-indol-2-carboxamid;
*N*-[6-(3-Hydroxyazetidin-1-yl)pyridin-3-yl]-5-trimethylsilyl-1-[(3-fluorphenyl)methyl]-1*H*-indol-2-carboxamid;
*N*-[6-(Azetidin-1-yl)pyridin-3-yl]-7-trimethylsilyl-1-[(3-fluorphenyl)methyl]-1*H*-indol-2-carboxamid;
*N*-[6-(3-Hydroxyazetidin-1-yl)pyridin-3-yl]-7-trimethylsilyl-1-[(3-fluorphenyl)methyl]-1*H*-indol-2-carboxamid;
*N*-[6-(Dimethylamino)pyridin-3-yl]-5-trimethylsilyl-1-[(3-fluorphenyl)methyl]-1*H*-indol-2-carboxamid;
*N*-[6-(Azetidin-1-yl)pyridin-3-yl]-5-(ethyl)-dimethylsilyl-1-[(3-fluorphenyl)methyl]-1*H*-indol-2-carboxamid;
*N*-[6-(Azetidin-1-yl)pyridin-3-yl]-5-(cyclohexyl)-dimethylsilyl-1-[(3-fluorphenyl)methyl]-1*H*-indol-2-carboxamid;
1*N*-[6-(Azetidin-1-yl)pyridin-3-yl]-6-trimethylsilyl-1-[[(3-trifluormethyl)phenyl]methyl]-1*H-*indol-2-carboxamid;
*N*-[6-(3-Hydroxyazetidin-1-yl)pyridin-3-yl]-6-trimethylsilyl-1-[[(3-trifluormethyl)phenyl]-methyl]-1*H*-indol-2-carboxamid;
*N*-[6-(Azetidin-1-yl)pyridin-3-yl]-5-(diphenyl)-methylsilyl-1-[(3-fluorphenyl)methyl]-1*H*-indol-2-carboxamid;
*N*-[6-(Azetidin-1-yl)pyridin-3-yl]-5-trimethylsilyl-1-[[(3-trifluormethyl)phenyl]methyl]-1*H-*indol-2-carboxamid;
*N*-[6-(3-Hydroxyazetidin-1-yl)pyridin-3-yl]-5-trimethylsilyl-1-[[(3-trifluormethyl)phenyl]-methyl]-1H-indol-2-carboxamid;
*N*-[6-(3-Hydroxyazetidin-1-yl)pyridin-3-yl]-6-trimethylsilyl-1-[(pyridin-4-yl)methyl]-1*H*-indol-2-carboxamid;
*N*-[6-(Azetidin-1-yl)pyridin-3-yl)pyridin-3-yl]-6-trimethylsilyl-1-[(pyridin-4-yl)methyl]-1*H*-indol-2-carboxamid;
*N*-[6-(Azetidin-1-yl)pyridin-3-yl]-5-triethylsilyl-1-[(3-fluorphenyl)methyl]-1H-indol-2-carboxamid;
*N*-[6-(Azetidin-1-yl)pyridin-3-yl]-5-tert.-butyl-(dimethyl)silyl-1-[(3-fluorphenyl)methyl]-1*H-*indol-2-carboxamid;
*N*-[6-(Pyrrolidin-1-yl)pyridin-3-yl]-5-(cyclohexyl)dimethylsilyl-1-[(3-fluorphenyl)methyl]-1*H-*indol-2-carboxamid;
*N*-[6-(Azetidin-1-yl)pyridin-3-yl]-5-phenyl-dimethylsilyl-1-[(3-fluorphenyl)methyl]-1*H*-indol-2-carboxamid;
*N*-[6-(Azetidin-1-yl)pyridin-3-yl]-5-(isopropyl)-dimethylsilyl-1-[(3-fluorphenyl)methyl]-1*H*-indol-2-carboxamid;
*N*-[6-(Azetidin-1-yl)pyridin-3-yl]-5-(methyl)-diethylsilyl-1-[(3-fluorphenyl)methyl]-1*H*-indol-2-carboxamid.

11. Verfahren zur Herstellung einer Verbindung der Formel (I) gemäß einem der Ansprüche 1 bis 10, **dadurch gekennzeichnet, dass** man eine Verbindung der allgemeinen Formel (IV) 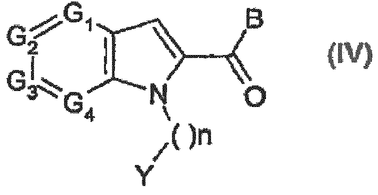 worin G₁, G₂, G₃ und G₄, Y und n wie in der allgemeinen Formel (I) gemäß Anspruch 1 definiert sind und B für ein Chloratom steht,
in einem Lösungsmittel mit einem Amin der allgemeinen Formel (V) 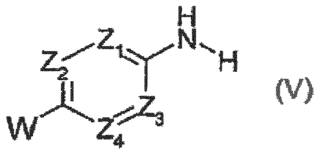 worin W = Z und Z, Z₁, Z₂, Z₃ und Z₄ wie in der allgemeinen Formel (I) gemäß Anspruch 1 definiert sind, umsetzt.

12. Verfahren zur Herstellung einer Verbindung der Formel (I) gemäß einem der Ansprüche 1 bis 10, **dadurch gekennzeichnet, dass** man eine Verbindung der allgemeinen Formel (IV) 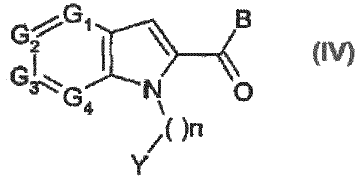 worin G₁, G_{2;} G₃ und G₄, Y und n wie in der allgemeinen Formel (I) gemäß Anspruch 1 definiert sind und B für eine Hydroxygruppe steht,
in Gegenwart eines Kupplungsmittels und gegebenenfalls einer Base in einem Lösungsmittel mit einem Amin der allgemeinen Formel (V) 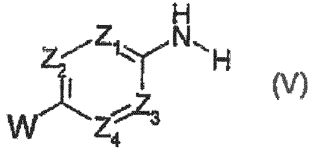 worin W = Z und Z, Z₁, Z₂, Z₃ und Z₄ wie in der allgemeinen Formel (I) gemäß Anspruch 1 definiert sind, umsetzt.

13. Verfahren zur Herstellung einer Verbindung der Formel (I) gemäß einem der Ansprüche 1 bis 10, **dadurch gekennzeichnet, dass** man eine Verbindung der allgemeinen Formel (VI) 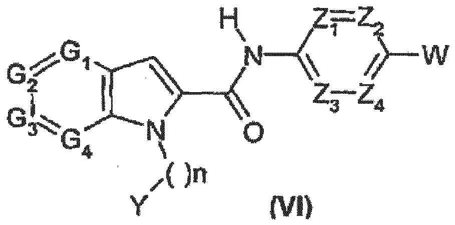 worin G₁, G₂, G₃ und G₄, Y, n, Z₁, Z₂, Z₃ und Z₄ wie in der allgemeinen Formel (I) gemäß Anspruch 1 definiert sind und W für ein Halogenatom steht, mit einem Amin der allgemeinen Formel Z-H, worin Z wie in der allgemeinen Formel (I) gemäß Anspruch 1 definiert ist, umsetzt.

14. Verfahren zur Herstellung einer Verbindung der Formel (I) gemäß einem der Ansprüche 1 bis 10, **dadurch gekennzeichnet, dass** man eine Verbindung der allgemeinen Formel (X) 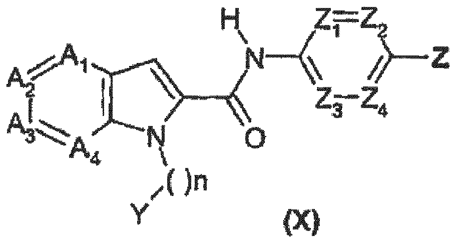 worin Y, n, Z, Z₁, Z₂, Z₃ und Z₄ wie in der allgemeinen Formel (I) gemäß Anspruch 1 definiert sind und A₁, A₂, A₃ und A₄ unabhängig voneinander für eine Gruppe C-X oder ein Stickstoffatom stehen; wobei X wie in der allgemeinen Formel (I) gemäß Anspruch 1 definiert ist, eine der Gruppen A₁, A₂, A₃ und A₄ und höchstens eine der Gruppen A₁, A₂, A₃ und A₄ für eine Gruppe C-q steht, wobei q für ein Halogenatom in der Position, in der die Silanylgruppe eingeführt werden soll, steht,
in Gegenwart einer katalytisch wirksamen Menge eines Metallkomplexes und einer Base in einem Lösungsmittel mit einem Silan der Formel SiH (X₁) (X₂) (X₃) , worin X₁, X₂ und X₃ wie in der allgemeinem Formel (I) gemäß Anspruch 1 definiert sind, umsetzt.

15. Verbindung (IVa), (IVb), (Ivc), (Ivd), (Ive) oder (IVf):

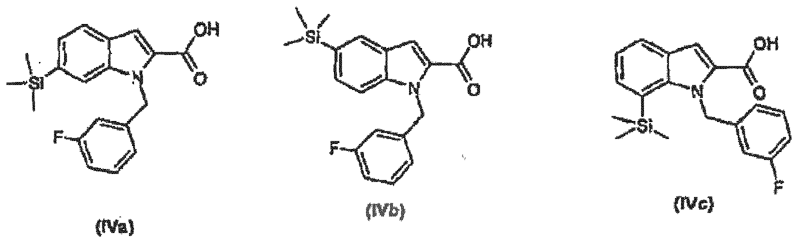

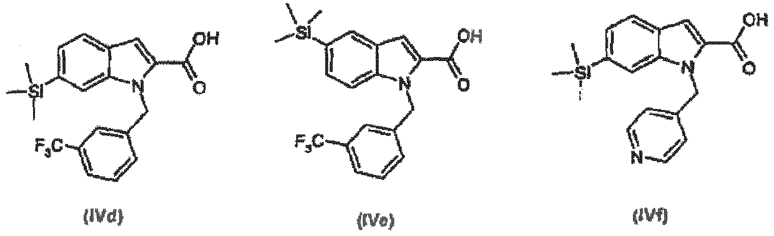

16. Arzneimittel, dadurch gekenntzeichnet, dass es eine Verbindung der Formel (I) gemäß einem der Ansprüche 1 bis 10 oder ein Säureadditionssalz dieser Verbindung mit einer pharmazeutisch unbedenklichen Säure oder auch ein Hydrat oder ein Solvat der Verbindung der Formel (I) umfasst.

17. Pharmazeutische Zusammensetzung, **dadurch gekennzeichnet, dass** sie eine Verbindung der Formel (I) gemäß einem der Ansprüche 1 bis 10 oder ein pharmazeutisch unbedenkliches Salz oder auch ein Hydrat oder ein Solvat dieser Verbindung sowie mindestens einen pharmazeutisch unbedenklichen Hilfsstoff umfasst.

18. Verwerdung einer Verbindung der Formel (I) gemäß einem der Ansprüche 1 bis 10 zur Herstellung eines Arzneimittels zur Prävention oder Behandlung von Pathologien, an denen Rezeptoren vom TRPV1-Typ beteiligt sind.

19. Verwendung einer Zusammensetzung der Formel (I) gemäß einem der Ansprüche 1 bis 10 zur Herstellung eines Arzneimittels zur Prävention oder Behandlung von Schmerzen, Entzündungen, Stoffwechselstörungen, urologischen Störungen, gynäkologischen Störungen, gastroiritestinalen Störungen, respiratorischen Störungen, Psoriasis, Pruritis, Haut-, Augen- oder Schleimhautreizungen, Herpes, Zona, multipler Sklerose, Depression oder Krebserkrankungen.
